# EUROPEAN PATENT APPLICATION

(11) **EP 2 610 245 A1**
(43) Date of publication of application: **03.07.2013**
(21) Application number: 11195970.6
(22) Date of filing: 28.12.2011
(51) Int. Cl.: C07D 211/60, A61K 31/445, A61P 25/24

(54) **Pipecolate-sulfonamides for treatment of psychiatric disorders**

(71) Applicant: Max-Planck-Gesellschaft zur Förderung der Wissenschaften e.V., 80539 München (DE)
(72) Inventor: Gopalakrishnan, Ranganath, 380052 Ahmedabad (IN); Hausch, Felix, 80796 Munich (DE)
(74) Representative: Arth, Hans-Lothar

(57) **Abstract**

The present invention relates to compounds having a pipecolate sulfonamide scaffold, pharmaceutically acceptable salts of these compounds and pharmaceutical compositions containing at least one of these compounds together with pharmaceutically acceptable carrier, excipient and/or diluents. Said pipecolate sulfonamide compounds can be used for prophylaxis and/or treatment of psychiatric disorders and neurodegenerative diseases, disorders and conditions.

## Description

The present invention relates to pipecolate sulfonamide derivatives and stereoisomeric forms, prodrugs, solvates, hydrates and/or pharmaceutically acceptable salts of these compounds as well as pharmaceutical compositions containing at least one of these pipecolate sulfonamide derivatives together with pharmaceutically acceptable carrier, excipient and/or diluents. Said pipecolate sulfonamide derivatives have been identified as specific inhibitors of the FK506 binding proteins (FKBP's), especially the FKBP-51 and FKBP-52, and are useful for the treatment of psychiatric disorders and neurodegenerative diseases, disorders and conditions, for treating vision disorders and/or improving vision; for treating memory impairment and/or enhancing memory performance and for treating alopecia and promoting hair growth.

### Background of the invention

The FK506-binding protein (FKBP) family of immunophilins consists of proteins with a variety of protein-protein interaction domains and versatile cellular functions. This highly conserved protein family binds with immunosuppressive drugs, such as FK506 and rapamycin. This protein family displays peptidyl propyl isomerase (PPlase) activity as seen with cyclophilins and parvulins. FKBP12, a 12 kD protein is the most widely studied member of this family.

The immunosuppressant drugs FK506, rapamycin, and cyclosporin are well known as potent T-cell specific immunosuppressants, and are effective against autoimmunity, transplant or graft rejection, inflammation, allergic responses, other autoimmune or immune-mediated diseases, and infectious diseases. FK506 and rapamycin apart from binding to FKBP12 also interact and inhibit calcineurin (CaN) and mTOR respectively thereby mediating their immunosuppressive action.

The high molecular weight multidomain homologs of FKBP12 (FKBP51/52) act as co chaperons for the heat shock protein (Hsp90) and modulate the signal transduction of the glucocorticoid receptor by participating in the Heat shock protein 90 (Hsp90) - steroid receptor complex.

In this complex, FKBP 51 and 52 modulate the binding competence and signalling of steroid hormone receptors and thereby regulate the cellular responsiveness to circulating hormone levels. This is supported by a natural animal model (squirrel monkey) and by knockout mice, where the essential role of FKPB 51 and 52 on the Glucocorticoid Receptor (GR) activity have been clearly demonstrated. Moreover, polymorphisms in the FKBP51-encoding gene of psychiatric patients have been associated with a faster response to antidepressants, with a higher incidence in depressive episodes and with a higher susceptibility for peritraumatic dissociation.

The immunosuppressive compounds disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to α-ketoamide analogs of FKBP 12 inhibitors devoid of immunosuppressive activity. The electrophilicity of the α-ketoamide moiety in the non-immunosuppressive analogs is an undesired reactive liability that could result in metabolic instability or potential toxicity. A solution could be found in sulfonamide analogs that lack a reactive liability and show similar affinities.

It is the object of the present invention to provide compounds and/or pharmaceutically acceptable salts thereof which inhibit FKBP 51 and FKBP 52 but which show no immunosuppressive activity, are metabolic stable and non toxic.

A further aspect of the invention is to provide compounds and/or pharmaceutically acceptable salts thereof which can be used as pharmaceutically active agents, especially for the treatment of psychiatric disorders and neurodegenerative diseases, disorders and conditions, for treating vision disorders and/or improving vision, for treating memory impairment and/or enhancing memory performance and for treating alopecia, as well as compositions comprising at least one of those compounds and/or pharmaceutically acceptable salts thereof as pharmaceutically active ingredients.

The object of the present invention is solved by the teaching of the independent claims. Further advantageous features, aspects and details of the invention are evident from the dependent claims, the description, and the examples of the present application.

### Description of the invention

The novel pipecolate sulfonamide derivatives according to the present invention are represented by the following general formula (I): 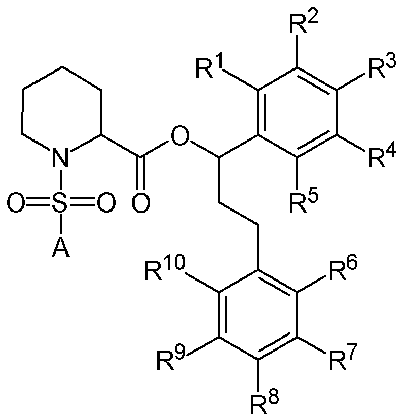 wherein
**R¹-R¹⁷** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] , -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH_{2,} -CH(CH₃)-C≡C-CH₃, -CH=CH-CH(CH₃)-CH=CH₂, -CH=C(CH₃)-CH₂-CH=CH₂, -C₂H₄-CH(CH₃)-C=CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 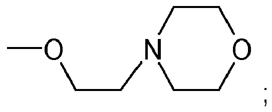 R^{N} represents the following -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -c₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COCN, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SOCH₃, -SOC₂H₅, -SOC₃H₇, -SO₂-cyclo-C₃H₅, -SO₂CH(CH₃)₂, -SO₂C(CH₃)₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C=CH, -CH(CH₃)-CH=CH-CH=CH₂, -CH=CH-CH₂-C(CH₃)=CH₂, -CH(CH₃)-C=C-CH₃, -CH=CH-CH(CH₃)-CH=CH₂, -CH=C(CH₃)-CH₂-CH=CH₂, -C₂H₄-CH(CH₃)-C=CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
and **A** represents the following 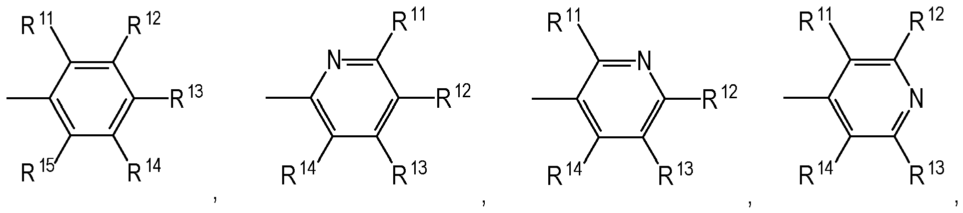 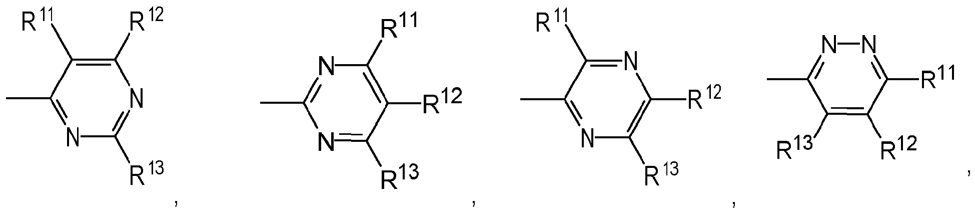 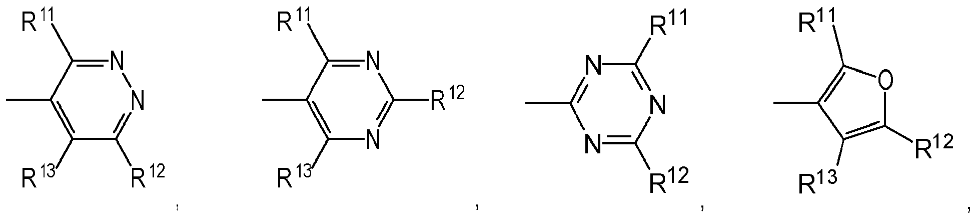 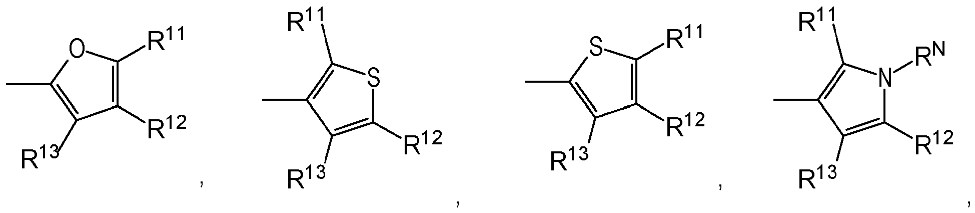 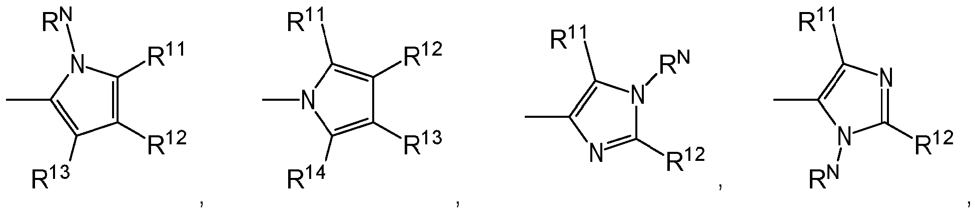 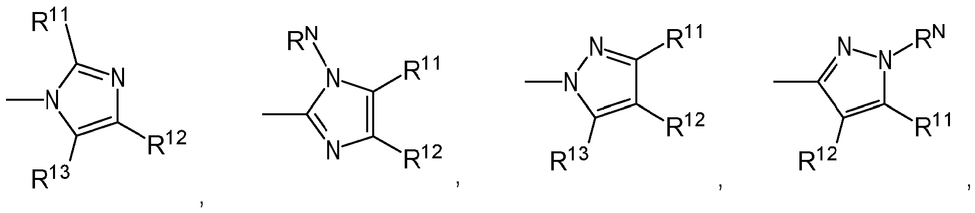 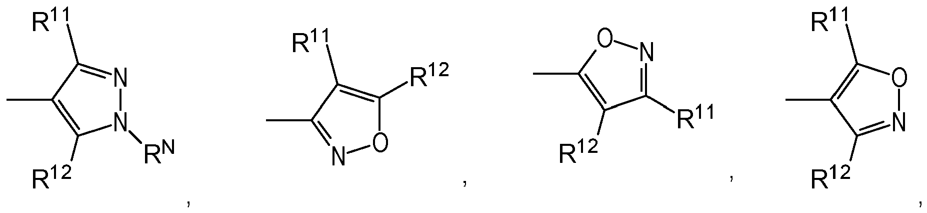 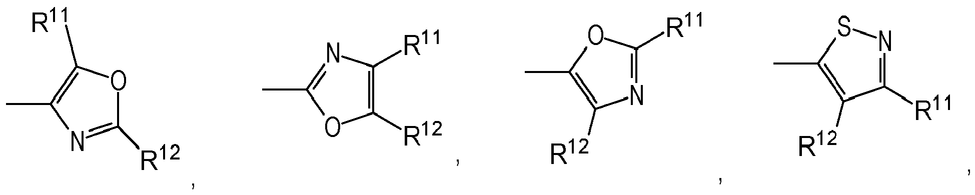 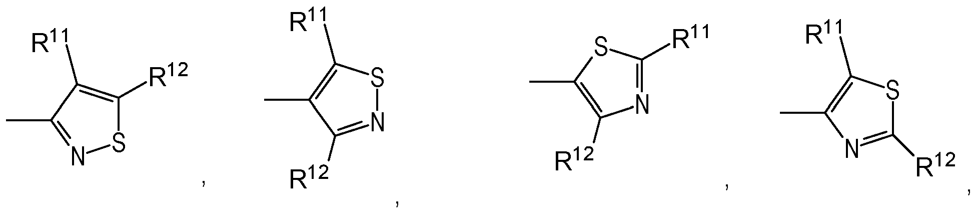 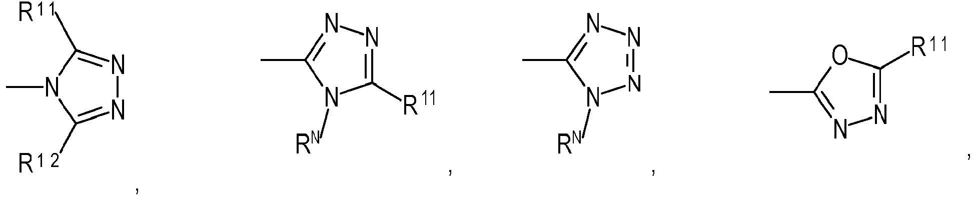 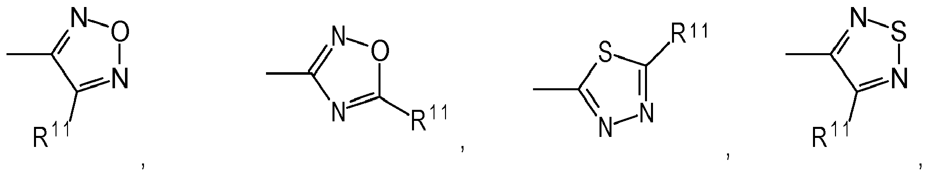 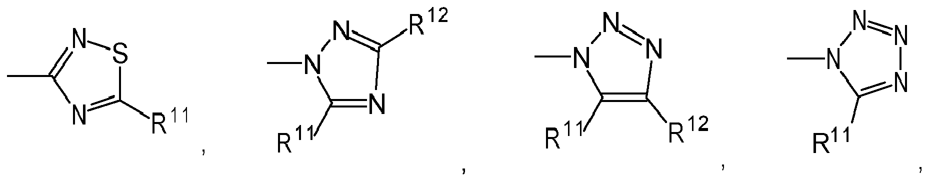 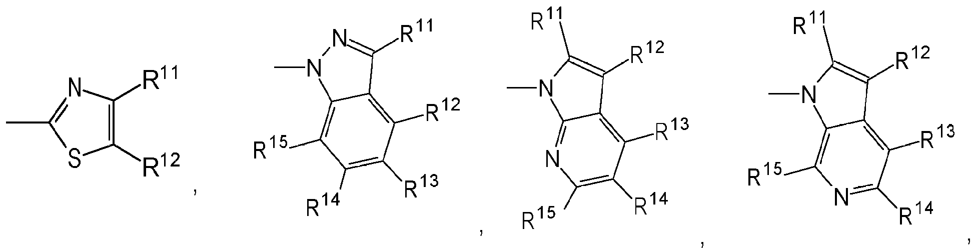 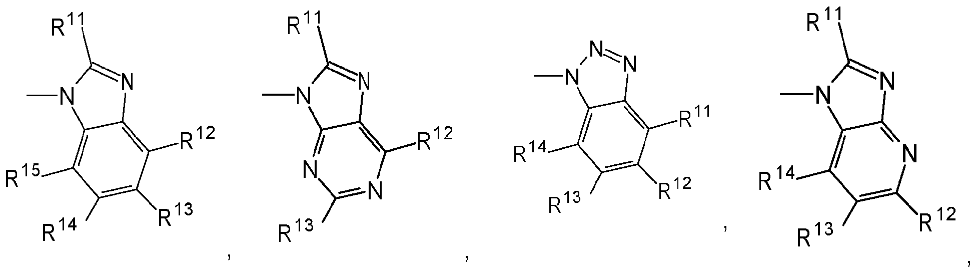 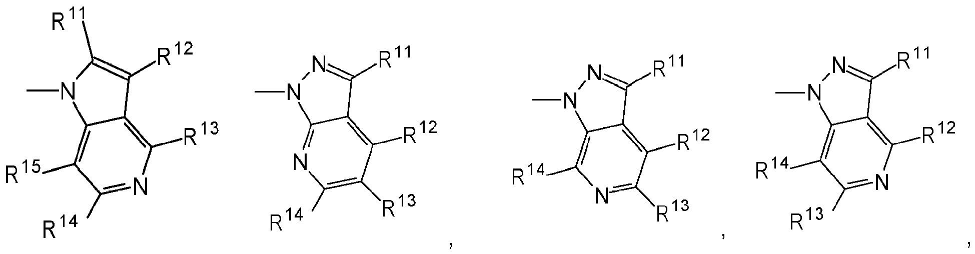 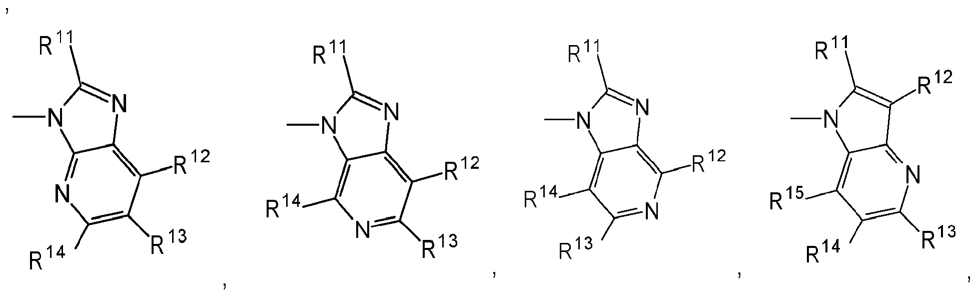 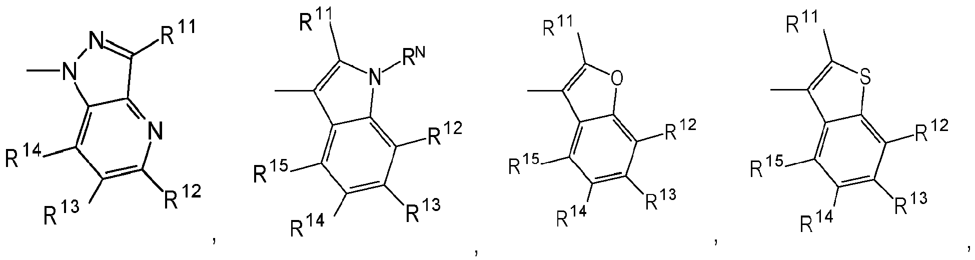 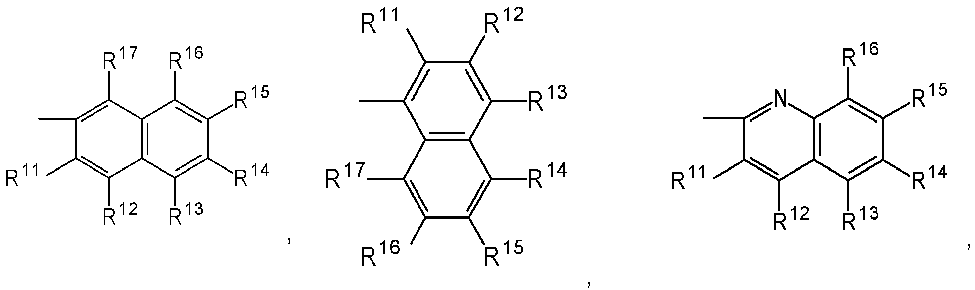 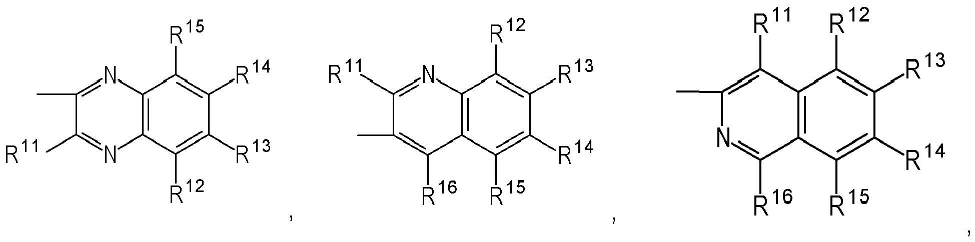 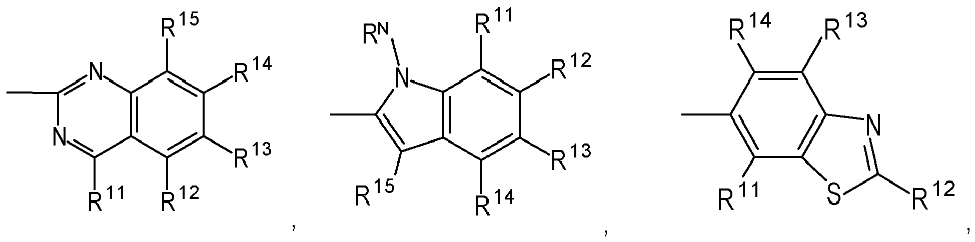 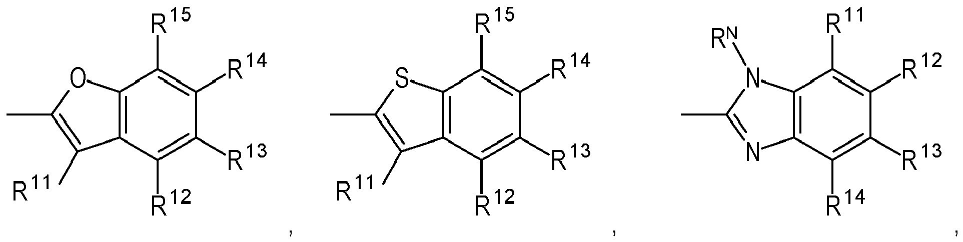 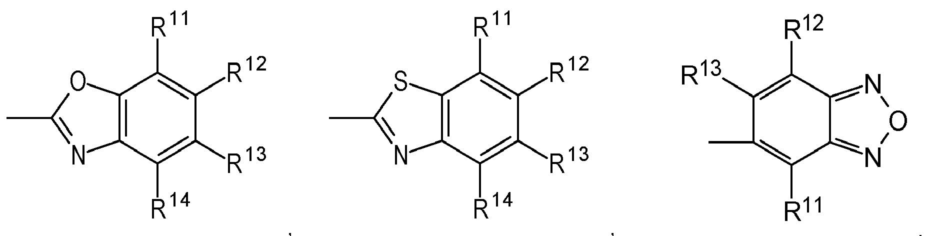 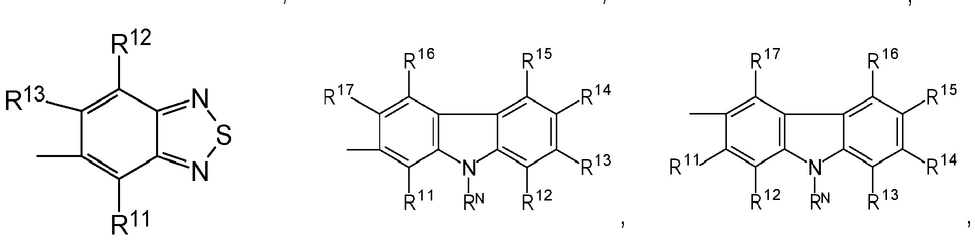 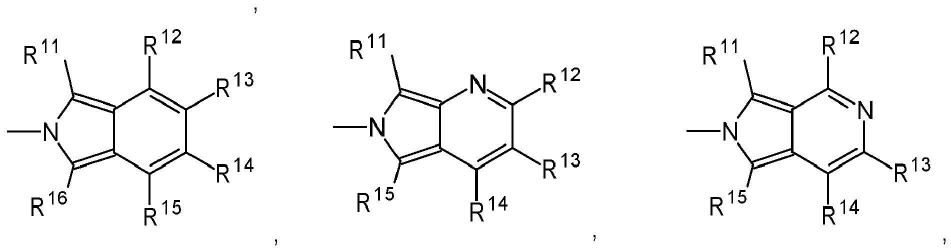 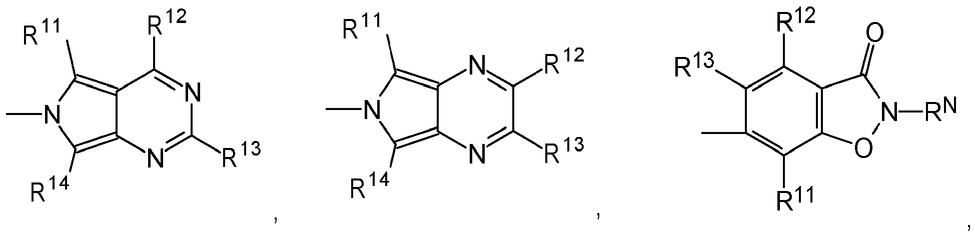 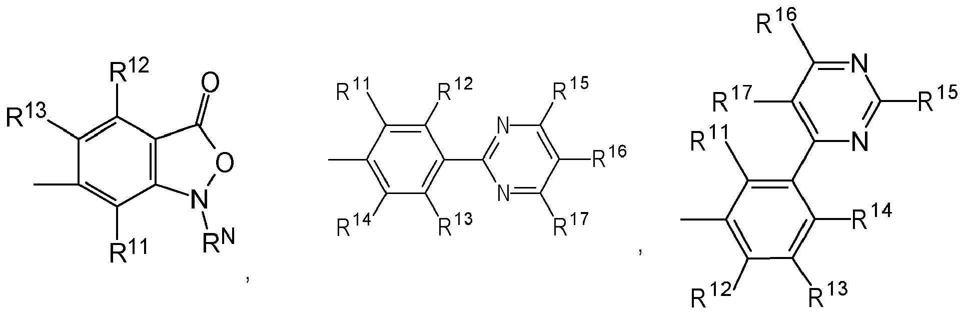 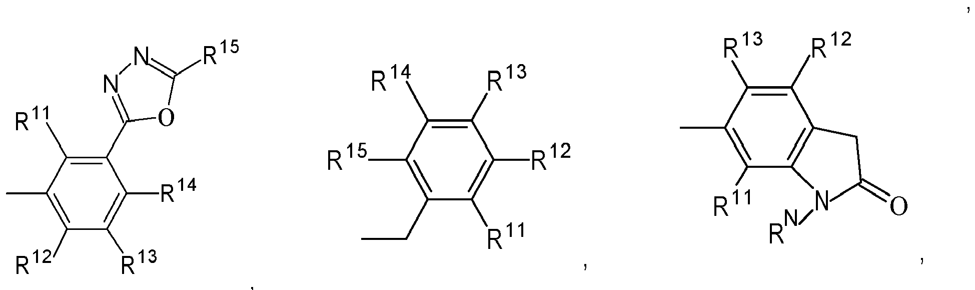 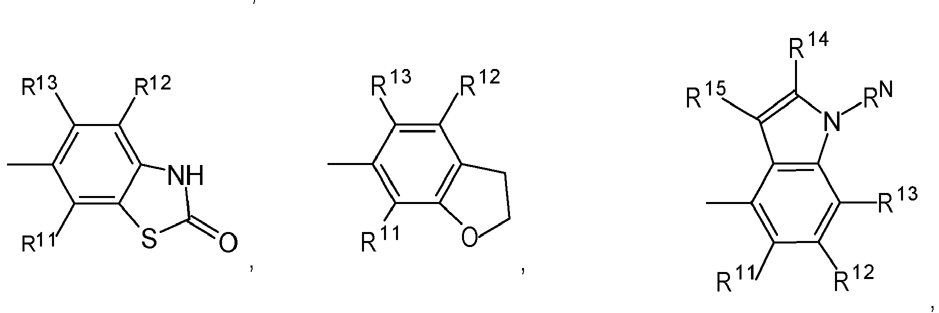 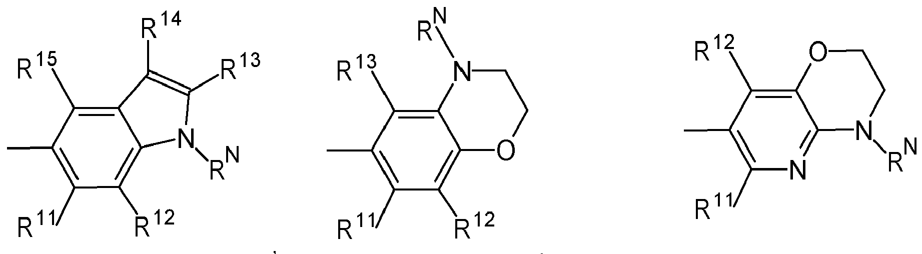 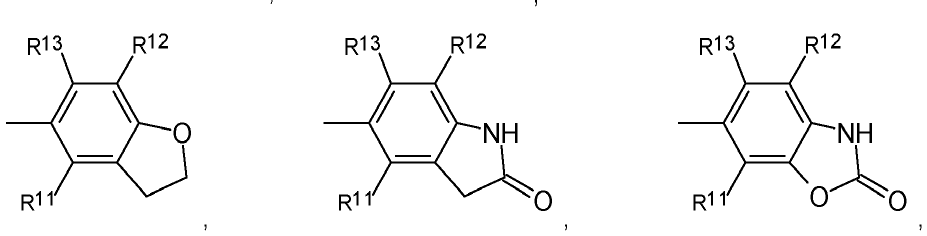 and enantiomers, stereoisomeric forms, mixtures of enantiomers, anomers, deoxyforms, diastereomers, mixtures of diastereomers, prodrugs, tautomers, hydrates, solvates and racemates of the above mentioned compounds and pharmaceutically acceptable salts thereof.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

The following subformulas (II) - (IV) of formula (I) are preferred: 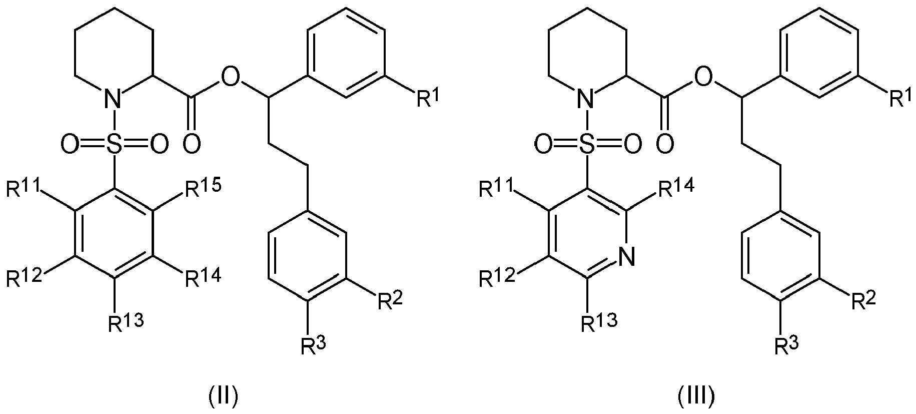 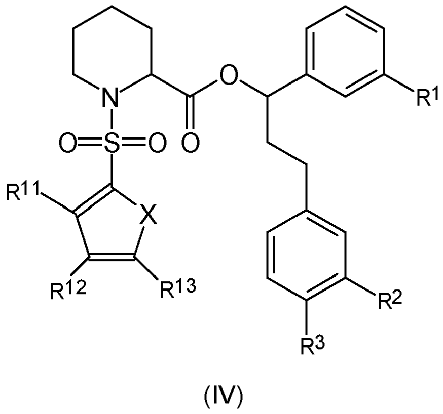 wherein
**X** represents O, S; and the substituents R¹ - R³ and R¹¹ - R¹⁵ have the meanings as defined herein.

According to the general formula (I) the following compounds are especially preferred:
2-(3-((R)-3-(3,4-dimethoxyphenyl)-1-((S)-1-(4-(pyrimidin-2-yl)phenylsulfonyl)pipridine -2-carbonyloxy) propyl)phenoxy)acetic acid,
2-(3-((R)-1-((S)-1-(3-chlorophenylsulfonyl)piperidine-2-carbonyloxy)-3-(3,4-dimethoxy phenyl) propyl- phenoxy)acetic acid,
2-(3-((R)-1-((S)-1-(benzo[d]thiazol-5-ylsulfonyl)piperidine-2-carbonyloxy)-3-(3,4-dimethoxy phenyl)propyl) phenoxy)acetic acid,
2-(3-((R)-3-(3,4-dimethoxyphenyl)-1-((S)-1-(furan-3-ylsulfonyl)piperidine-2-carbonyloxy) propyl)phenoxy)acetic acid,
2-(3-((R)-1-((S)-1-(benzo[b]thiophen-2-ylsulfonyl)piperidine-2-carbonyloxy)-3-(3,4-dimeth xy phenyl propyl)phenoxy)acetic acid,
2-(3-((R)-1-((S)-1-(3-cyanophenylsulfonyl)piperidine-2-carbonyloxy)-3-(3,4-dimethoxyphen -yl)propyl)phenoxy)acetic acid,
2-(3-((R)-3-(3,4-dimethoxyphenyl)-1-((S)-1-(3-nitrophenylsulfonyl)piperidine-2-carbonylo -xy propyl)phenoxy)acetic acid,
2-(3-((R)-3-(3,4-dimethoxyphenyl)-1-((S)-1-(3-(2-methylpyrimidin-4-yl)phenylsulfonyl) piperidine-2-carbonyloxy)propyl)phenoxy)acetic acid,
2-(3-((R)-3-(3,4-dimethoxyphenyl)-1-((S)-1-(3-(pyrimidin-4-yl)phenyl sulfonyl)piperidine-2-carbonyloxy)propyl)phenoxy)acetic acid,
2-(3-((R)-1-((S)-1-(3,5-dichlorophenylsulfonyl)piperidine-2-carbonyloxy)-3-(3,4-dimethoxy -phenyl)propyl)phenoxy)acetic acid,
2-(3-((R)-1-((S)-1-(2,3-dichlorophenylsulfonyl)piperidine-2-carbonyloxy)-3-(3,4-dimethoxy phenyl)propyl)phenoxy)acetic acid,
2-(3-((R)-3-(3,4-dimethoxyphenyl)-1-((S)-1-(3,4-dimethoxyphenylsulfonyl) piperidine-2-carbonyloxy)propyl)phenoxy)acetic acid,
2-(3-((R)-1-((S)-1-(3,5-bis(trifluoromethyl)phenylsulfonyl)piperidine-2-carbonyloxy)-3-(3,4-dimethoxyphenyl)propyl)phenoxy)acetic acid,
2-(3-((R)-1-((S)-1-(3-bromo-5-(trifluoromethyl)phenylsulfonyl)piperidine-2-carbonyloxy)-3-(3,4-dimethoxyphenyl)propyl)phenoxy)acetic acid,
2-(3-((R)-1-((S)-1-(3,5-dichloro-4-hydroxyphenylsulfonyl)piperidine-2-carbonyloxy)-3-(3,4-dimethoxyphenyl)propyl)phenoxy)acetic acid,
2-(3-((R)-1-((S)-1-(3,5-dichloro-4-methoxyphenylsulfonyl)piperidine-2-carbonyloxy)-3-(3,4-dimethoxyphenyl)propyl)phenoxy)acetic acid,
(S)-((R)-3-(3,4-dimethoxyphenyl)-1-(3-(2-morpholinoethoxy)phenyl)propyl)1-(3,5-dichloro-phenylsulfonyl)piperidine-2-carboxylate,
(S)-((R)-3-(3,4-dimethoxyphenyl)-1-(3-(2-morpholinoethoxy)phenyl)prop-yl) 1-(benzo[d]thi -azol -6-ylsulfonyl)piperidine-2-carboxylate,
(S)-((R)-3-(3,4-dimethoxyphenyl)-1-(3-(2-morpholinoethoxy)phenyl)propyl)1-(3,5-dichloro-4-hydroxyphenylsulfonyl)piperidine-2-carboxylate,
(S)-((R)-3-(3,4-dimethoxyphenyl)-1-(3-(2-morpholinoethoxy)phenyl)propyl)1-(3,5-dichloro-4-methoxyphenylsulfonyl)piperidine-2-carboxylate,
(S)-((R)-3-(3,4-dimethoxyphenyl)-1-(3-(2-morpholinoethoxy)phenyl)propyl)1-(2-oxo-2,3-dihydrobenzo[d]thiazol-6-ylsulfonyl)piperidine-2-carboxylate,
2-(3-((R)-3-(3,4-dimethoxyphenyl)-1-((S)-1-(2-oxoindolin-5-ylsulfonyl) piperidine-2-carbonyloxy)propyl)phenoxy)acetic acid,
2-(3-((R)-3-(3,4-dimethoxyphenyl)-1-((S)-1-(2-oxo-2,3-dihydrobenzo[d]oxazol-6-ylsulfonyl)piperidine-2-carbonyloxy)propyl)phenoxy)aceticacid,
2-(3-((R)-1-((S)-1-(4-acetamido-3,5-dichorophenylsulfonyl)piperidine-2-carbo nyloxy)-3-(3,4-dimethoxyphenyl)propyl)phenoxy)acetic acid,
5-((S)-2-(((R)-1-(3-(carboxymethoxy)phenyl)-3-(3,4-dimethoxyphenyl)propoxy) carbonyl)piperidin-1-ylsulfonyl)-2,3-dimethoxybenzoic acid,
2-(3-((R)-3-(3,4-dimethoxyphenyl)-1-((S)-1-(7-nitro-2,3-dihydrobenzofuran-5-ylsulfonyl)piperidine-2-carbonyloxy)propyl)phenoxy)acetic acid,
2-(3-((R)-1-((S)-1-(7-amino-2,3-dihydrobenzofuran-5-ylsulfonyl)piperidine-2-carbonyloxy)-3-(3,4-dimethoxyphenyl)propyl)phenoxy)aceticacid,
2-(3-((R)-3-(3,4-dimethoxyphenyl)-1-((S)-1-(2-methylbenzo[d]thiazol-6-ylsulf onyl)piperidine-2-carbonyloxy)propyl)phenoxy)acetic acid,
2-(3-((R)-3-(3,4-dimethoxyphenyl)-1-((S)-1-(2-oxo-2,3-dihydrobenzo[d]thiazol-6-ylsulfonyl)piperidine-2-carbonyloxy)propyl)phenoxy)aceticacid,
2-(3-((R)-1-((S)-1-(3,5-bis(methoxycarbonyl)phenylsulfonyl)piperidine-2-carbonyl oxy)-3-(3,4-dimethoxyphenyl)propyl)phenoxy)acetic acid,
2-(3-((R)-1-((S)-1-(3,5-difluorophenylsulfonyl)piperidine-2-carbonyloxy)-3-(3,4-dimethoxyphenyl)propyl)phenoxy)acetic acid,
2-(3-((R)-1-((S)-1-(3-chloro-4-methoxyphenylsulfonyl)piperidine-2-carbonyloxy)-3-(3,4-dimethoxyphenyl)propyl)phenoxy)aceticacid,
2-(3-((R)-3-(3,4-dimethoxyphenyl)-1-((S)-1-(3-fluorophenylsulfonyl)piperidine-2-carbonyloxy)propyl)phenoxy)acetic acid,
2-(3-((R)-1-((S)-1-(3-bromophenylsulfonyl)piperidine-2-carbonyloxy)-3-(3,4-dimethoxyphenyl)propyl)phenoxy)acetic acid,
2-(3-((R)-1-((S)-1-(3-aminophenylsulfonyl)piperidine-2-carbonyloxy)-3-(3,4-dimethoxyphenyl)propyl)phenoxy)acetic acid.
2-(3-((R)-1-((S)-1-(4-chlorophenylsulfonyl)piperidine-2-carbonyloxy)-3-(3,4-dimethoxyphenyl)propyl) phenoxy)acetic acid,
2-(3-((R)-1-((S)-1-(4-tert-butylphenylsulfonyl)piperidine-2-carbonyloxy)-3-(3,4-dimethoxyphenyl)propyl) phenoxy)acetic acid,
2-(3-((R)-3-(3,4-dimethoxyphenyl)-1-((S)-1-(4-fluorophenylsulfonyl)piperidine-2-carbonyloxy)propyl) phenoxy)acetic acid,
2-(3-((R)-1-((S)-1-(4-chlorobenzylsulfonyl)piperidine-2-carbonyloxy)-3-(3,4-dimethoxyphenyl)propyl) phenoxy)acetic acid,
2-(3-((R)-3-(3,4-dimethoxyphenyl)-1-((S)-1-(4-(trifluoromethyl)phenylsulfonyl) piperidine-2-carbonyloxy)propyl)phenoxy)acetic acid,
2-(3-((R)-3-(3,4-dimethoxyphenyl)-1-((S)-1-(4-phenoxyphenylsulfonyl)piperidine-2-carbonyloxy)propyl) phenoxy)acetic acid,
2-(3-((R)-3-(3,4-dimethoxyphenyl)-1-((S)-1-(pyridin-3-ylsulfonyl)piperidine-2-carbonyloxy)propyl) phenoxy)acetic acid,
2-(3-((R)-3-(3,4-dimethoxyphenyl)-1-((S)-1-(6-phenoxypyridin-3-ylsulfonyl)piperidine-2-carbonyloxy) propyl)phenoxy)acetic acid,
2-(3-((R)-3-(3,4-dimethoxyphenyl)-1-((S)-1-(6-phenylpyridin-3-ylsulfonyl)piperidine-2-carbonyloxy) propyl)phenoxy)acetic acid,
2-(3-((R)-1-((S)-1-(2-chlorophenylsulfonyl)piperidine-2-carbonyloxy)-3-(3,4-dimethoxyphenyl)propyl) phenoxy)acetic acid,
2-(3-((R)-1-((S)-1-(2,6-dichlorophenylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(3-(5-methyl-1,3,4-oxadiazol-2-yl)phenylsulfonyl)piperidine-2-carbonyloxy)propyl)phenoxy)acetic acid,
2-(3-((R)-1-((S)-1-(3-chloro-4-(trifluoromethyl)phenylsulfonyl)piperidine-2-carbonyloxy)-3-(3,4-dimethoxyphenyl)propyl)phenoxy)acetic acid,
2-(3-((R)-3-(3,4-dimethoxyphenyl)-1-((S)-1-(1-methyl-1H-indol-4-ylsulfonyl)piperidine-2-carbonyloxy) propyl)phenoxy)acetic acid,
2-(3-((R)-3-(3,4-dimethoxyphenyl)-1-((S)-1-(1-methyl-1H-indol-5-ylsulfonyl)piperidine-2-carbonyloxy)propyl)phenoxy)acetic acid,
2-(3-((R)-3-(3,4-dimethoxyphenyl)-1-((S)-1-(4-methyl-3,4-dihydro-2H-benzo[b][1,4]oxazin-6-ylsulfonyl)piperidine-2-carbonyloxy)propyl)phenoxy)acetic acid,
2-(3-((R)-3-(3,4-dimethoxyphenyl)-1-((S)-1-(4-methyl-3,4-dihydro-2H-pyrido[3,2-b][1,4]oxazin-7-ylsulfonyl)piperidine-2-carbonyloxy)propyl)phenoxy)acetic acid,
2-(3-((R)-3-(3,4-dimethoxyphenyl)-1-((S)-1-(furan-3-ylsulfonyl)piperidine-2-carbonyloxy)propyl) phenoxy)acetic acid,
2-(3-((R)-1-((S)-1-(benzofuran-2-ylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(thiophen-2-ylsulfonyl)piperidine-2-carbonyloxy)propyl) phenoxy)acetic acid,
2-(3-((R)-3-(3,4-dimethoxyphenyl)-1-((S)-1-(5-phenylthiophen-2-ylsulfonyl)piperidine-2-carbonyloxy) propyl)phenoxy)acetic acid,
2-(3-((R)-1-((S)-1-(benzo[b]thiophen-3-ylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(3,5-dimethylisoxazol-4-ylsulfonyl)piperidine-2-carbonyloxy) propyl)phenoxy)acetic acid,
2-(3-((1R)-3-(3,4-dimethoxyphenyl)-1-((2S)-1-(1-methyl-4,5-dihydro-1H-pyrazol-5-ylsulfonyl)piperidine-2-carbonyloxy)propyl)phenoxy)acetic acid,
2-(3-((R)-3-(3,4-dimethoxyphenyl)-1-((S)-1-(1-methyl-1H-imidazol-4-ylsulfonyl)piperidine-2-carbonyloxy)propyl)phenoxy)acetic acid,
2-(3-((R)-3-(3,4-dimethoxyphenyl)-1-((S)-1-(1,3,5-trimethyl-1H-pyrazol-4-ylsulfonyl)piperidine-2-carbonyloxy)propyl)phenoxy)acetic acid,
2-(3-((R)-3-(3,4-dimethoxyphenyl)-1-((S)-1-(3,5-dimethyl-1-phenyl-1H-pyrazol-4-ylsulfonyl)piperidine-2-carbonyloxy)propyl)phenoxy)acetic acid,
2-(3-((R)-3-(3,4-dimethoxyphenyl)-1-((S)-1-tosylpiperidine-2-carbonyloxy)propyl)phenoxy)acetic acid,
2-(3-((R)-3-(3,4-dimethoxyphenyl)-1-((S)-1-(4-nitrophenylsulfonyl)piperidine-2-carbonyloxy) propyl)phenoxy)acetic acid,
(S)-((R)-3-(3,4-dimethoxyphenyl)-1-(3-(2-morpholinoethoxy)phenyl)propyl)1-(4-acetamido-3,5-dichlorophenylsulfonyl)piperidine-2-carboxylate.

The present invention also comprises pharmaceutically acceptable salts of the compounds according to the general formula (I), all stereoisomeric forms of the compounds according to the general formula (I) as well as solvates, especially hydrates or prodrugs thereof.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomers.

Another aspect of the present invention relates to the use of the inventive pipecolate-sulfonamide derivatives as drugs, i.e. as pharmaceutically active agents applicable in medicine.

Surprisingly it was found that the above-mentioned pipecolate-sulfonamide derivatives as well as the pharmaceutical compositions comprising said pipecolate-sulfonamide derivatives are useful for the inhibition of the FK506 binding proteins (FKBP's), especially the inhibition of FKBP51 and 52.

Therefore one aspect of the present invention is that the compounds according to the general formula (I) are suitable for use as inhibitor of FK506-binding proteins (FKBP). It is preferred if said compound is suitable for use as inhibitor of the FK506-binding protein 51 (FKBP51) and 52 (FKBP52).

FKBP inhibitors as used herein is defined as compounds that inhibit the peptidyl-prolyl isomerase activity of FKBPs (PPlase inhbitors, also referred to as rotamase inhibitors) and compounds that displace FK506 or FK506 analogs form the PPlase active site of FKBPs.

Thus, the pipecolate-sulfonamide compounds of the present invention can be used for treatment, or for the preparation of a pharmaceutical formulation for prophylaxis and treatment of psychiatric and neurodegenerative diseases, disorders and conditions, for neuroprotection or neuroregeneration, for the treatment of neurological disorders, for the treatment of diseases relating to neurodegeneration, for the treatment of cancers and especially steroid-hormone dependent cancers, for the treatment of glucocorticoid hyposensitivity syndromes and for peripheral glucocorticoid resistance, and for the treatment of infectious diseases, for the treatment of alopecia and promoting hair growth, for the treatment or prevention of multi-drug resistance, for stimulating neurite growth, for the use as wound healing agents for treating wounds resulting from injury or surgery; for the use in antiglaucomatous medications for treating abnormally elevated intraocular pressure; for the use in limiting or preventing hemorrhage or neovascularization for treating macular degeneration, and for treating oxidative damage to eye tissues, for treating a vision disorder, for improving vision, for treating memory impairment or enhancing memory performance.

Thus, the pipecolate-sulfonamide compounds of the present invention may further be used for induction of abortion and especially the inhibition of embryo implantation.

The pipecolate-sulfonamide compounds of the present invention are preferably suitable for treatment, or for the preparation of a pharmaceutical formulation for prophylaxis and treatment of psychiatric diseases. It is especially preferred if this psychiatric diseases is an affective disorder (ICD-10 classification: F30-F39) or an anxiety disorder.

Affective disorder is a mental disorder characterized by dramatic changes or extremes of mood. The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, AIDS, rheumatoid arthritis, hypertension and obesity.

Among the cancers, the attention has been given to malignant melanoma, steroid-hormone dependent cancers or prostate cancer.

Among the hundreds of infectious diseases, the attention has been given to malaria and the Legionnaires' disease.

Among the vision disorders, the attention has been given to visual impairments; orbital disorders; disorders of the lacrimal apparatus; disorders of the eyelids; disorders of the conjunctiva; disorders of the Cornea; cataract; disorders of the uveal tract; disorders of the retina; disorders of the optic nerve or visual pathways; free radical induced eye disorders and diseases; immunologically-mediated eye disorders and diseases; eye injuries; and symptoms and complications of eye disease, eye disorder, or eye injury.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions may further comprise at least one active pipecolate sulfonamide of the general formula (I).

The pharmaceutical compositions may further comprise at least one further active agent. It is preferred if this active agent is selected from the group consisting of anti-depressant and other psychotropic drugs. It is further preferred if the anti-depressant is selected from amitriptyline, amioxide clomipramine, doxepine, duloxetine, imipramine trimipramine, mirtazapine, reboxetine, citaloprame, fluoxetine, moclobemide and sertraline.

### Examples

This first FKBP domain consists of five antiparallel β-strands curved around a central α-helix, the usual FK fold A three-dimensional alignment of the binding pockets of the first FKBP domain of FKBP 51, of FKBP 52 (FKBP51 FK1 and FKBP52FK1) and FKBP12 revealed the largest structural divergences at the 80s loop (Ser¹¹⁸-Ile¹²² of FKBP51/52). The 80s loop of FKBP51 contains Leu¹¹⁹ which is replaced by Pro¹¹⁹ in FKBP52 possibly contributing for the structural difference in this region. Importantly the residue at position 119 was shown to be a major functional determinant for the effect on steroid hormone receptor. Hence an optimization of interactions with this part of the protein has a higher probability of achieving selectivity and functional relevance within the FKBP family. The X ray structure of FK506 with the FK1 domain of FKBP51 and FKBP52 revealed that the pyranose group in FK506 contacts the 80s loop. For a rapid derivatization of compounds targeting the 80s loop we envisaged a solid phase strategy for synthesis of a focused sulfonamide library.

### Generic route to pipecolate-sulfonamides

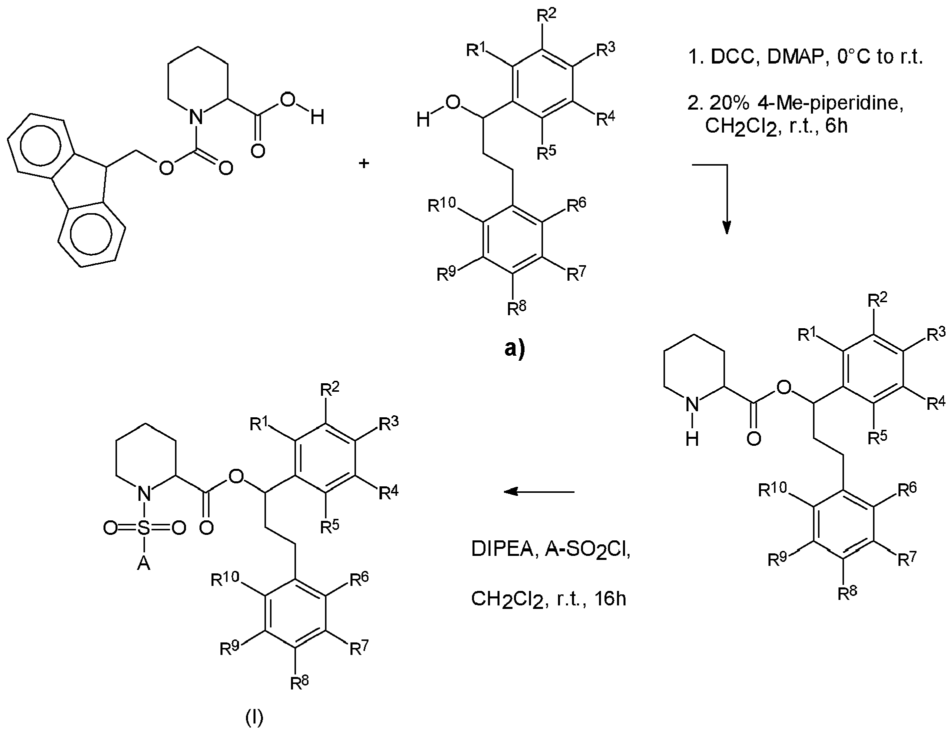

### General procedure for the synthesis of pipecolate sulfonamides

A solution of the alcohol **a**) (15 mmol), which has been synthesized as described in example 1, pipecolic carboxylic acid (1.1 equ.), and DMAP (0.1 equ.) in 50mL DCM at 0°C was treated with DCC (1.1 equ.). The mixture was allowed to warm to room temperature and was stirred for 20h after which time the organic solvent was dried and the solid was dissolved in diethyl ether (50mL) and filtered through a plug of celite. The filtrate was concentrated and then flash chromatographed to afford pipecolic ester. The Ester was immobilized on a 2-Chloro tritylresin by coupling one of the substituents R¹-R¹⁰ with the trityl-chloride moieties of the resin.

The coupled resin was weighed (210 mg, 0.08mmol) and added to syringes, swollen in DCM (4 mL) for 1h, and the Fmoc protecting group was removed using 20% 4-methyl piperidine/ DCM (4ml) for 1h. After filtration, the resin was washed with DCM (3 x 5ml) and was used for the next coupling step.

To the above resin was added i-Pr₂EtN (40mg, 0.32 mmol) in dry DCM (3 mL) and stirred for 20min. To this solution was added the sulfonyl chloride (A-SO₂-Cl, 0.75 equ.) in 500 µL of DCM and the reaction was stirred for 4h at room temperature. The coupling procedure was repeated. The resin was washed with DCM (3 x 5 ml) and dried to give the derivatized resin which was re-swollen in DCM and reacted with 1% TFA/DCM (3 ml) for 1h to cleave the sulfonamides from the resin. The combined filtrates were concentrated in vacuo to afford the crude sulfonamides, which were further purified by preparative HPLC.

By this general procedure the following pipecolate sulfonamides were synthesized:
2-(3-((R)-3-(3,4-dimethoxyphenyl)-1-((S)-1-(4-(pyrimidin-2-yl)phenylsulfonyl) pipridine -2-carbonyloxy) propyl)phenoxy)acetic acid **(compound 1),**
2-(3-((R)-1-((S)-1-(3-chlorophenylsulfonyl)piperidine-2-carbonyloxy)-3-(3,4-dimethoxy phenyl) propyl- phenoxy)acetic acid **(compound 2),**
2-(3-((R)-1-((S)-1-(benzo[d]thiazol-5-ylsulfonyl)piperidine-2-carbonyloxy)-3-(3,4-dimethoxy phenyl)propyl) phenoxy)acetic acid **(compound 3),**
2-(3-((R)-3-(3,4-dimethoxyphenyl)-1-((S)-1-(furan-3-ylsulfonyl)piperidine-2-carbonyloxy) propyl)phenoxy)acetic acid **(compound 4),**
2-(3-((R)-1-((S)-1-(benzo[b]thiophen-2-ylsulfonyl)piperidine-2-carbonyloxy)-3-(3,4-dimeth xy phenyl propyl)phenoxy)acetic acid **(compound 5),**
2-(3-((R)-1-((S)-1-(3-cyanophenylsulfonyl)piperidine-2-carbonyloxy)-3-(3,4-dimethoxyphen -yl)propyl)phenoxy)acetic acid (**compound 6**),
2-(3-((R)-3-(3,4-dimethoxyphenyl)-1-((S)-1-(3-nitrophenylsulfonyl)piperidine-2-carbonylo -xy propyl)phenoxy)acetic acid (**compound 7**),
2-(3-((R)-1-((S)-1-(3-aminophenylsulfonyl)piperidine-2-carbonyloxy)-3-(3,4-dimethoxyphenyl)propyl)phenoxy)acetic acid (**compound 8**),
2-(3-((R)-3-(3,4-dimethoxyphenyl)-1-((S)-1-(3-(2-methylpyrimidin-4-yl)phenylsulfonyl) piperidine-2-carbonyloxy)propyl)phenoxy)acetic acid (**compound 9**),
2-(3-((R)-3-(3,4-dimethoxyphenyl)-1-((S)-1-(3-(pyrimidin-4-yl)phenyl sulfonyl)piperidine-2-carbonyloxy)propyl)phenoxy)acetic acid (**compound 10**),
2-(3-((R)-3-(3,4-dimethoxyphenyl)-1-((S)-1-(3-fluorophenylsulfonyl)piperidine-2-carbonyloxy)propyl)phenoxy)acetic acid (**compound11**),
2-(3-((R)-1-((S)-1-(3-bromophenylsulfonyl)piperidine-2-carbonyloxy)-3-(3,4-dimethoxyphenyl)propyl)phenoxy)acetic acid (**compound 12**),
2-(3-((R)-1-((S)-1-(3,5-dichlorophenylsulfonyl)piperidine-2-carbonyloxy)-3-(3,4-dimethoxy -phenyl)propyl)phenoxy)acetic acid (**compound 13**),
2-(3-((R)-1-((S)-1-(2,3-dichlorophenylsulfonyl)piperidine-2-carbonyloxy)-3-(3,4-dimethoxy phenyl)propyl)phenoxy)acetic acid (**compound 14**),
2-(3-((R)-3-(3,4-dimethoxyphenyl)-1-((S)-1-(3,4-dimethoxyphenylsulfonyl) piperidine-2-carbonyloxy)propyl)phenoxy)acetic acid (**compound 15**),
2-(3-((R)-1-((S)-1-(3-chloro-4-methoxyphenylsulfonyl)piperidine-2-carbonyl oxy)-3-(3,4-dimethoxyphenyl)propyl)phenoxy)aceticacid (**compound 16**),
2-(3-((R)-1-((S)-1-(3,5-difluorophenylsulfonyl)piperidine-2-carbonyloxy)-3-(3,4-dimethoxyphenyl)propyl)phenoxy)acetic acid (**compound 17**),
2-(3-((R)-1-((S)-1-(3,5-bis(trifluoromethyl)phenylsulfonyl)piperidine-2-carbonyloxy)-3-(3,4-dimethoxyphenyl)propyl)phenoxy)acetic acid (**compound 18**),
2-(3-((R)-1-((S)-1-(3-bromo-5-(trifluoromethyl)phenylsulfonyl)piperidine-2-carbonyloxy)-3-(3,4-dimethoxyphenyl)propyl)phenoxy)acetic acid (**compound 19**),
2-(3-((R)-1-((S)-1-(3,5-bis(methoxycarbonyl)phenylsulfonyl)piperidine-2-carbonyl oxy)-3-(3,4-dimethoxyphenyl)propyl)phenoxy)acetic acid (**compound 20**),
2-(3-((R)-1-((S)-1-(3,5-dichloro-4-hydroxyphenylsulfonyl)piperidine-2-carbonyloxy)-3-(3,4-dimethoxyphenyl)propyl)phenoxy)acetic acid (**compound 21**),
2-(3-((R)-1-((S)-1-(3,5-dichloro-4-methoxyphenylsulfonyl)piperidine-2-carbonyl-oxy)-3-(3,4-dimethoxyphenyl)propyl)phenoxy)acetic acid (**compound 22**),
2-(3-((R)-1-((S)-1-(4-acetamido-3,5-dichlorophenylsulfonyl)piperidine-2-carbo nyloxy)-3-(3,4-dimethoxyphenyl)propyl)phenoxy)acetic acid (**compound 23**),
5-((S)-2-(((R)-1-(3-(carboxymethoxy)phenyl)-3-(3,4-dimethoxyphenyl)propoxy) carbonyl)piperidin-1-ylsulfonyl)-2,3-dimethoxybenzoic acid (**compound 24**),
2-(3-((R)-3-(3,4-dimethoxyphenyl)-1-((S)-1-(7-nitro-2,3-dihydrobenzofuran-5-ylsulfonyl)piperidine-2-carbonyloxy)propyl)phenoxy)acetic acid (**compound 25**),
2-(3-((R)-1-((S)-1-(7-amino-2,3-dihydrobenzofuran-5-ylsulfonyl)piperidine-2-carbonyloxy)-3-(3,4-dimethoxyphenyl)propyl)phenoxy)aceticacid (**compound 26**),
2-(3-((R)-3-(3,4-dimethoxyphenyl)-1-((S)-1-(2-methylbenzo[d]thiazol-6-ylsulf onyl)piperidine-2-carbonyloxy)propyl)phenoxy)acetic acid (**compound 27**),
2-(3-((R)-3-(3,4-dimethoxyphenyl)-1-((S)-1-(2-oxo-2,3-dihydrobenzo[d]thiazol-6-ylsulfonyl)piperidine-2-carbonyloxy)propyl)phenoxy)aceticacid (**compound 28**),
2-(3-((R)-3-(3,4-dimethoxyphenyl)-1-((S)-1-(2-oxoindolin-5-ylsulfonyl) piperidine-2-carbonyloxy)propyl)phenoxy)acetic acid (**compound 29**),
2-(3-((R)-3-(3,4-dimethoxyphenyl)-1-((S)-1-(2-oxo-2,3-dihydrobenzo[d]oxazol-6-ylsulfonyl)piperidine-2-carbonyloxy)propyl)phenoxy)aceticacid (**compound 30**),
(S)-((R)-3-(3,4-dimethoxyphenyl)-1-(3-(2-morpholinoethoxy)phenyl)propyl)1-(3,5-dichloro -phenylsulfonyl)piperidine-2-carboxylate (**compound 31**),
(S)-((R)-3-(3,4-dimethoxyphenyl)-1-(3-(2-morpholinoethoxy)phenyl)prop-yl) 1-(benzo[d]thiazol-6-ylsulfonyl)piperidine-2-carboxylate (**compound 32**),
(S)-((R)-3-(3,4-dimethoxyphenyl)-1-(3-(2-morpholinoethoxy)phenyl)propyl)1-(3,5-dichloro-4-hydroxyphenylsulfonyl)piperidine-2-carboxylate (**compound 33**),
(S)-((R)-3-(3,4-dimethoxyphenyl)-1-(3-(2-morpholinoethoxy)phenyl)propyl)1-(4-acetamido-3,5-dichlorophenylsulfonyl)piperidine-2-carboxylate (**compound 34**),
(S)-((R)-3-(3,4-dimethoxyphenyl)-1-(3-(2-morpholinoethoxy)phenyl)propyl)1-(2-oxo-2,3-dihydrobenzo[d]thiazol-6-ylsulfonyl)piperidine-2-carboxylate (**compound 35**),
2-(3-((R)-1-((S)-1-(4-chlorophenylsulfonyl)piperidine-2-carbonyloxy)-3-(3,4-dimethoxyphenyl)propyl) phenoxy)acetic acid (**compound 36**),
2-(3-((R)-1-((S)-1-(4-tert-butylphenylsulfonyl)piperidine-2-carbonyloxy)-3-(3,4-dimethoxyphenyl)propyl) phenoxy)acetic acid (**compound 37**),
2-(3-((R)-3-(3,4-dimethoxyphenyl)-1-((S)-1-(4-fluorophenylsulfonyl)piperidine-2-carbonyloxy)propyl) phenoxy)acetic acid (**compound 38**),
2-(3-((R)-1-((S)-1-(4-chlorobenzylsulfonyl)piperidine-2-carbonyloxy)-3-(3,4-dimethoxyphenyl)propyl) phenoxy)acetic acid (**compound 39**),
2-(3-((R)-3-(3,4-dimethoxyphenyl)-1-((S)-1-(4-(trifluoromethyl)phenylsulfonyl)piperidine-2-carbonyloxy)propyl)phenoxy)acetic acid (**compound 40**),
2-(3-((R)-3-(3,4-dimethoxyphenyl)-1-((S)-1-(4-phenoxyphenylsulfonyl)piperidine-2-carbonyloxy)propyl) phenoxy)acetic acid (**compound 41**),
2-(3-((R)-3-(3,4-dimethoxyphenyl)-1-((S)-1-(pyridin-3-ylsulfonyl)piperidine-2-carbonyloxy)propyl) phenoxy)acetic acid (**compound 42**),
2-(3-((R)-3-(3,4-dimethoxyphenyl)-1-((S)-1-(6-phenoxypyridin-3-ylsulfonyl)piperidine-2-carbonyloxy) propyl)phenoxy)acetic acid (**compound 43**),
2-(3-((R)-3-(3,4-dimethoxyphenyl)-1-((S)-1-(6-phenylpyridin-3-ylsulfonyl)piperidine-2-carbonyloxy) propyl)phenoxy)acetic acid (**compound 44**),
2-(3-((R)-1-((S)-1-(2-chlorophenylsulfonyl)piperidine-2-carbonyloxy)-3-(3,4-dimethoxyphenyl)propyl) phenoxy)acetic acid (**compound 45**),
2-(3-((R)-1-((S)-1-(2,6-dichlorophenylsulfonyl)piperidine-2-carbonyloxy)-3-(3,4-dimethoxyphenyl) propyl)phenoxy)acetic acid (**compound 46**),
2-(3-((R)-3-(3,4-dimethoxyphenyl)-1-((S)-1-(3-(5-methyl-1,3,4-oxadiazol-2-yl)phenylsulfonyl)piperidine-2-carbonyloxy)propyl)phenoxy)acetic acid (**compound 47**),
2-(3-((R)-1-((S)-1-(3-chloro-4-(trifluoromethyl)phenylsulfonyl)piperidine-2-carbonyloxy)-3-(3,4-dimethoxyphenyl)propyl)phenoxy)acetic acid (**compound 48**),
2-(3-((R)-3-(3,4-dimethoxyphenyl)-1-((S)-1-(1-methyl-1H-indol-4-ylsulfonyl)piperidine-2-carbonyloxy) propyl)phenoxy)acetic acid (**compound 49**),
2-(3-((R)-3-(3,4-dimethoxyphenyl)-1-((S)-1-(1-methyl-1H-indol-5-ylsulfonyl)piperidine-2-carbonyloxy)propyl)phenoxy)acetic acid (**compound 50**),
2-(3-((R)-3-(3,4-dimethoxyphenyl)-1-((S)-1-(4-methyl-3,4-dihydro-2H-benzo[b][1,4]oxazin-6-ylsulfonyl)piperidine-2-carbonyloxy)propyl)phenoxy)acetic acid (**compound 51**),
2-(3-((R)-3-(3,4-dimethoxyphenyl)-1-((S)-1-(4-methyl-3,4-dihydro-2H-pyrido[3,2-b][1,4]oxazin-7-ylsulfonyl)piperidine-2-carbonyloxy)propyl)phenoxy)acetic acid (**compound 52**),
2-(3-((R)-3-(3,4-dimethoxyphenyl)-1-((S)-1-(furan-3-ylsulfonyl)piperidine-2-carbonyloxy)propyl) phenoxy)acetic acid (**compound 53**),
2-(3-((R)-1-((S)-1-(benzofuran-2-ylsulfonyl)piperidine-2-carbonyloxy)-3-(3,4-dimethoxyphenyl) propyl)phenoxy)acetic acid (**compound 54**),
2-(3-((R)-3-(3,4-dimethoxyphenyl)-1-((S)-1-(thiophen-2-ylsulfonyl)piperidine-2-carbonyloxy)propyl) phenoxy)acetic acid (**compound 55**),
2-(3-((R)-3-(3,4-dimethoxyphenyl)-1-((S)-1-(5-phenylthiophen-2-ylsulfonyl)piperidine-2-carbonyloxy) propyl)phenoxy)acetic acid (**compound 56**),
2-(3-((R)-1-((S)-1-(benzo[b]thiophen-3-ylsulfonyl)piperidine-2-carbonyloxy)-3-(3,4-dimethoxyphenyl) propyl)phenoxy)acetic acid (**compound 57**),
2-(3-((R)-3-(3,4-dimethoxyphenyl)-1-((S)-1-(3,5-dimethylisoxazol-4-ylsulfonyl)piperidine-2-carbonyloxy) propyl)phenoxy)acetic acid (**compound 58**),
2-(3-((1R)-3-(3,4-dimethoxyphenyl)-1-((2S)-1-(1-methyl-4,5-dihydro-1H-pyrazol-5-ylsulfonyl)piperidine-2-carbonyloxy)propyl)phenoxy)acetic acid (**compound 59**),
2-(3-((R)-3-(3,4-dimethoxyphenyl)-1-((S)-1-(1-methyl-1H-imidazol-4-ylsulfonyl)piperidine-2-carbonyloxy)propyl)phenoxy)acetic acid (**compound 60**),
2-(3-((R)-3-(3,4-dimethoxyphenyl)-1-((S)-1-(1,3,5-trimethyl-1H-pyrazol-4-ylsulfonyl)piperidine-2-carbonyloxy)propyl)phenoxy)acetic acid (**compound 61**),
2-(3-((R)-3-(3,4-dimethoxyphenyl)-1-((S)-1-(3,5-dimethyl-1-phenyl-1H-pyrazol-4-ylsulfonyl)piperidine-2-carbonyloxy)propyl)phenoxy)acetic acid (**compound 62**),
2-(3-((R)-3-(3,4-dimethoxyphenyl)-1-((S)-1-tosylpiperidine-2-carbonyloxy)propyl)phenoxy)acetic acid (**compound 63**),
2-(3-((R)-3-(3,4-dimethoxyphenyl)-1-((S)-1-(4-nitrophenylsulfonyl)piperidine-2-carbonyloxy) propyl)phenoxy)acetic acid (**compound 64**),
(S)-((R)-3-(3,4-dimethoxyphenyl)-1-(3-(2-morpholinoethoxy)phenyl)propyl)1-(3,5-dichloro-4-methoxyphenylsulfonyl)piperidine-2-carboxylate (**compound 65**).

### Example 1

### Synthesis of alcohol a): (R)-tert-butyl 2-(3-(3-(3,4-dimethoxyphenyl)-1-hydroxypropyl) phenoxy) acetate

A solution of the corresponding phenol (T. Keenan et al./Bioorg. Med. Chem. 6 (1998) 1309-1335) (5g, 15.46 mmol) and K₂CO₃ (4.8g, 34.9mmol) in acetone (30mL) was treated with tert-butyl bromoacetate (3.7g, 19.21 mmol) and allowed to stir at room temperature for 20h. After this time the reaction mixture was filtered, concentrated and flash chromatographed to afford tert-butyl 2-(3-(3-(3,4-dimethoxyphenyl)propanoyl)phenoxy)acetate (6.6g, 16.5mmol, 94%).

TLC (hexane:EtOAc 8:2): RF = 0.50.

¹H NMR (400 MHz, CDCl₃) δ 1.45 (s, 9H), 2.97 (t, 2H, J = 8 Hz), 3.22 (t, 2H, J = 8 Hz), 3.82 (s, 3H), 3.83 (s, 3H), 4.52 (s, 2H), 6.73-6.78 (m, 3H), 7.09 (dd, 1H, J = 0.8, 2.4 Hz), 7.33 (t, 1H, J = 8 Hz), 7.43 (m, 1H), 7.53 (dd, 1H, J = 1.2, 7.6).

¹³C NMR (100 MHz, CDCl₃) δ 27.99, 29.77, 40.70, 55.80, 65.63, 82.55, 111.35, 111.81, 113.06, 119.99, 120.12, 121.39, 129.66, 133.77, 138.22, 147.38, 148.88, 158.12, 167.57, 198.77. MS (ESI) m/z 439.13 [M + K] ⁺.

A solution of tert-butyl 2-(3-(3-(3,4-dimethoxyphenyl)propanoyl)phenoxy)acetate (6.5g, 16.48mmol) in isopropanol was charged into the hydrogenation reactor (Highpressure laboratory autoclave Model IV from Roth) along with K₂CO₃ (2.3g, 16.48mmol). The reactor was flushed twice with argon and to the above solution was added Noyuri catalyst (ABCR). The reactor was flushed with argon to remove any traces of air and was sealed. Hydrogen gas was flushed into the reactor twice, to flush out argon. The reaction was then stirred at room temperature with hydrogen gas held at 15 bar pressure for 6 days after which time the reaction was filtered through celite pad, and washed continuously with diethyl ether. The organic solvent was dried under vacuum to yield (R)-tert-butyl 2-(3-(3-(3,4-dimethoxyphenyl)-1-hydroxypropyl)phenoxy) acetate (6.2g. 15.42mmol, 94%).

TLC (hexane:EtOAc 8:2): Rf = 0.3 ¹H NMR (300 MHz, CDCl₃) δ 1.47 (s, 9H), 2.03 (m, 2H), 2.642 (m, 2H), 3.83 (s, 3H), 3.84 (s, 3H), 4.49 (s, 2H), 4.66-4.61 (m, 1H), 6.70-6.80 (m, 4H), 6.95-6.91 (m, 2H), 7.24(t, 1H, J = 7.8 Hz).

¹³C NMR (75 MHz, CDCl₃) δ 28.01, 31.56, 40.62, 55.79, 55.90, 65.62, 73.55, 82.32, 111.31, 111.80, 112.18, 113.53, 119.08, 120.19, 129.48, 134.40, 146.56, 147.16, 148.82, 158.07, 168.01.

MS (ESI) m/z 425.20 [M + Na] ⁺, 441.17 [M + K] ⁺.

### Synthesis of 2-(3-((R)-3-(3,4-dimethoxyphenyl)-1-((S)-1-(4-(pyrimidin-2-yl)-phenylsulfonyl) pipridine -2-carbonyloxy) propyl)phenoxy)acetic acid (compound 1)

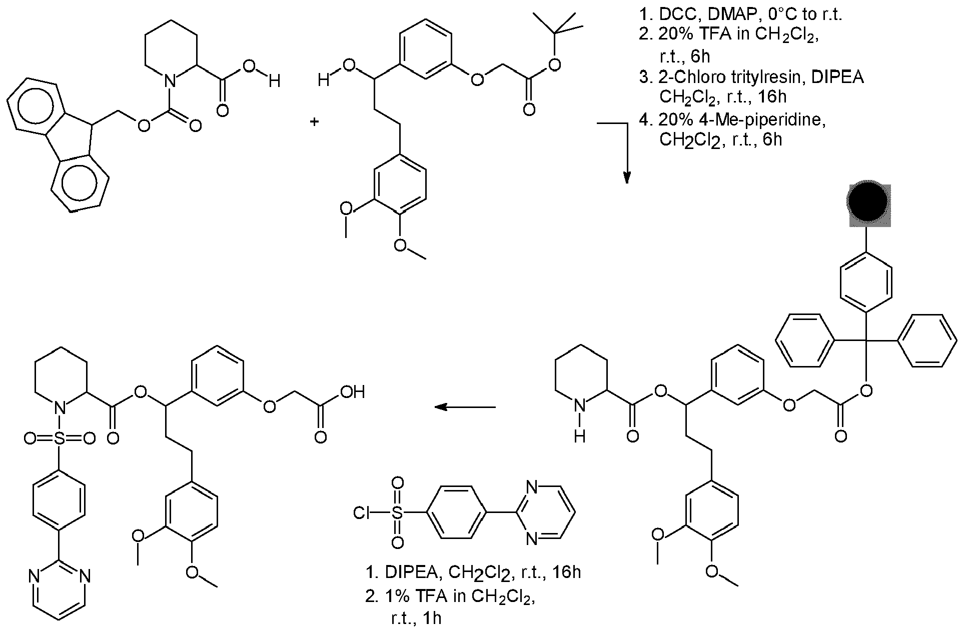

A solution of alcohol (6.2g, 15.44mmol), pipecolic carboxylic acid (6.2g, 17.64 mmol), and DMAP (0.2 g, 1.76mmol) in 50mL DCM at 0°C was treated with DCC (3.6g, 17.6mmol). The mixture was allowed to warm to room temperature and was stirred for 20h after which time the organic solvent was dried and the solid was dissolved in diethyl ether (50mL) and filtered through a plug of celite. The filtrate was concentrated and then flash chromatographed using hexane: EtOAc 2:1 to afford pipecolic ester (9.8g, 13.3 mmol, 84%). TLC (hexane: EtOAc 2:1): Rf = 0.4.

To the pipecolic ester_ (3g, 4mmol) was added a solution of 20% TFA in DCM (20mL) at 0°C to remove the t-Bu protective group. The mixture was allowed to warm to room temperature and stirred for 6h after which time it was diluted with DCM and evaporated under vacuo to remove of TFA. The crude material was then subjected to column chromatography using hexane: EtOAc: TFA 7.2: 2.8: 0.2 to afford the product (2-(3-((1R)-1-(1-(((9H-fluoren-9-yl)methoxy) carbonyl) piperidine-2-carbonyloxy)-3-(3,4- dimethoxyphenyl)propyl)phenoxy) acetic acid) (2.7g, 4mmol, 100%). TLC (hexanehexane:EtOAc: TFA 7:2.8:0.2): Rf = 0.375.

2-Chloro tritylresin (6.1 g, 7.9mmol, Novabiochem) was swollen in DCM for 1h, and added to a mixture of 2-(3-((1R)-1-(1-(((9H-fluoren-9-yl)methoxy)carbonyl) piperidine-2-carbonyloxy)-3-(3,4-dimethoxyphenyl)propyl)phenoxy) acetic acid (2.7 g, 3.97mmol ) and i-Pr₂EtN (2g, 15.89mmol) in 20 ml dry DCM. The reaction was monitored by removing aliquots of the reactant mixture (10 µL) over a period of 1- 24h filtered and dried. The analyte was then re-dissolved in buffer A and subjected to HPLC analysis for the disappearance of the educts. The slurry was agitated for 16h. The slurry was then filtered, and the resin was washed with DCM(3 x 50ml); DCM: MeOH: i-Pr₂EtN ::17:2:1 (3 x 50ml); DCM (3 x 20ml); DMF (3 x 25ml); MeOH (3 x 50ml); CHCl₃ (3 x 50ml) and diethyl ether (3 x 50ml) The resin was dried under high vacuum overnight to yield a free flowing resin immobilized with (2-(3-((1 R)-1-(1-(((9H-fluoren-9-yl)methoxy) carbonyl) piperidine-2-carbonyloxy)-3-(3,4-dimethoxyphenyl)propyl)phenoxy) acetic acid). The loading of the free acid was calculated using Fmoc deprotection strategy, and measuring the Fmoc absorbance.

The coupled resin was weighed (210 mg, 0.08mmol) and added to syringes, swollen in DCM (4 mL) for 1h, and the Fmoc protecting group was removed using 20% 4-methyl piperidine/DCM (4ml) for 1 h. After filtration, the resin was washed with DCM (3 x 5ml) and was used for the next coupling step.

To the above resin was added *i*-Pr₂EtN (40mg, 0.317mmol) in dry DCM (3 mL) and stirred for 20min. To this solution was added the sulfonyl chloride (0.237mmol) in 500 µL of DCM and the reaction was stirred for 4h at rt. After the first coupling strategy the resin was filtered washed with DCM (3 x 10ml) and then subjected to second coupling with i-Pr₂EtN (30mg, 0.237mmol), sulfonyl chloride (0.158 mmol) in DCM (3 mL) and stirred for 16h at rt. The resin was washed with DCM (3 x 5ml) and dried to give the derivatized resin which was re-swollen in DCM and reacted with 1% TFA/DCM (3ml) for 1 h and then washed with 1 % TFA/DCM (3 x 3ml), DCM (3 x 5ml). The combined filtrates were concentrated in vacuo to yield the crude product. The crude compound was further purified by preparative HPLC using the gradient: MEOH: H₂O : CF₃COOH (0.1 %) gradient of 40-70% B in 15 min at 25ml/min. The purified peaks were further dried by lyophilization and then given for NMR characterization in CDCl₃.

TLC (hexane:EtOAc: TFA 6:3.8:0.2): Rf = 0.18, Yield- 13.5mg, % Yield- 30%. HPLC (MeCN: H₂O CF₃COOH (0.1 %) gradient of 0 - 100% in 45 min at 1ml/min) retention time- 25.1-25.3 min

¹H NMR (600 MHz, CDCl₃) δ 1.29 (t, 1H, *J* = 7.2 Hz), 1.55 (dd, 1H, *J* = 3.6, 9 Hz), 1.67 (d, 1H, *J* = 12.6), 1.72 (d, 1H, *J* = 13.8 Hz), 1.85-1.91 (m, 1H), 1.93-1.99 (m, 1H), 2.13- 2.19 (m, 1H), 2.24 (d, 1H, *J* = 13.2 Hz), 2.45- 2.50 (m, 2H), 2.55- 2.59 (m, 1H), 3.11-3.16 (m,2H), 3.75 (d, 1H, *J* = 9.6 Hz), 3.82 (s, 3H), 3.83 (s, 3H), 4.91 (d, 1H, *J* = 4.8 Hz), 5.53 (q, 1H, *J* = 3, 4.8 Hz), 6.61 (s, 1H), 6.63 (d, 1H, *J* = 7.8 Hz), 6.74 (d, 1H, *J* = 7.8 Hz), 6.80 (s, 1H), 6.92 (d, 1H, *J* = 7.2 Hz), 7.01(d, 1H, *J* = 7.8 Hz), 7.32- 7.35 (m,2H), 7.59 (d. 2H, *J* = 8.4 Hz), 8.25 (d, 2H, *J* = 8.4 Hz), 8.91 (d, 2H, *J* = 4.8 Hz).

¹³C NMR (150 MHz, CDCl₃) δ 20.02, 24.74, 28.17, 31.42, 38.30, 42.45, 45.66, 55.33, 55.82, 65.24, 76.43, 111.28, 111.60, 112.94, 114.37, 118.85, 120.02, 120.09, 127.61, 128.51, 130.01, 133.25, 140.05, 141.31, 142.19, 147.34, 148.85, 157.41, 157.77, 162.90, 169.73

MS (ESI) m/z: found RT 11.74 min. (Method LCMS), 676.12 [M + H]⁺, 698.16 [M + Na]⁺, HRMS 676.2305 [M + H]⁺, calculated 676.2251.

### Example 2

### 2-(3-((R)-1-((S)-1-(3-chlorophenylsulfonyl)piperidine-2-carbo-nyloxy)-3-(3,4-dimethoxy phenyl) propyl- phenoxy)acetic acid (compound 2)

TLC (hexane:EtOAc: TFA 6:3.8:0.2): Rf = 0.50, Yield- 25.4 mg, % Yield- 57%. HPLC (MeCN: H₂O CF₃COOH (0.1 %) gradient of 0 - 100% in 45 min at 1ml/min) retention time- 26.1-26.5 min

¹H NMR (400 MHz, DMSO) δ 1.12-1.18 (m,4H), 1.59 (t, 2H, *J* = 13.6 Hz), 1.98-2.13 (m, 2H), 2.40 (t, 2H, *J* = 6.8 Hz), 3.07 (t. 1H, *J* = 12 Hz), 3.61 (d, 1H, *J* = 12 Hz), 3.69 (s,3H), 3.70 (s,3H), 4.55 (s,2H), 4.68 (d, 1H, *J* = 4Hz), 5.50 (t, 1H, *J* = 4.8Hz), 6.56 (d,1H, *J* = 7.6 Hz), 6.60 (s,1H), 6.72 (d,1H, *J* = 8 Hz), 6.76-6.80 (m, 3H), 7.19- 7.26 (m, 2H), 7.46 (d, 2H, *J* = 7.2 Hz), 7.61 (s, 1H).

¹³C NMR (100 MHz, DMSO) δ 20.05, 24.51, 27.74, 31.05, 37.92, 42.79, 55.32, 55.76, 64.99, 76.13, 111.95, 112.23, 112.85, 114.11, 119.22, 120.33, 125.36, 126.62, 129.77, 130.88, 132.66, 133.48, 134.48, 141.75, 141.95, 147.34, 148.92, 158.18, 169.61, 172.41.

MS (ESI) m/z: found RT 12.34 min. (Method LCMS), 654.17[M + Na]⁺, 656.16 [M + 2+ Na]⁺, HRMS 632.2212 [M + H] ⁺, 634.2205[M + 2+ H] ⁺, calculated 632.1643.

### Example 3

### 2-(3-((R)-1-((S)-1-(benzo[d]thiazol-5-ylsulfonyl)piperidine-2-carbonyloxy)-3-(3,4-dimethoxy phenyl)propyl) phenoxy)acetic acid (compound 3)

TLC (hexane:EtOAc: TFA 6:3.8:0.2): Rf = 0.20, Yield- 25.4mg, % Yield- 59%. HPLC (MeCN: H₂O CF₃COOH (0.1 %) gradient of 0 - 100% in 45 min at 1ml/min) retention time- 24.6-25.0 min

¹H NMR (600 MHz, CDCl₃) δ 1.08-1.15 (m,1H), 1.38-1.45 (m,1H), 1.60 (t, 2H, *J* = 12 Hz), 1.70-1.76 (m,1H), 1.94-2.00 (m,1H),, 2.11- 2.18 (m,1H), 2.43- 2.54 (m,1H), 3.23 (dt, 1H, *J* = 3, 6 Hz), 3.76-3.78 (m,1H), 3.85(s, 6H), 4.65 (s, 2H), 4.84 (d, 1H, *J* = 4.2 Hz), 5.55 (t, 1H, *J* = 7.2 Hz)), 6.63 (s, 1H), 6.64 (d, 1H, *J* = 1.8 Hz)), 6.78 (d, 2H, *J* = 5.4 Hz), 6.81 (dd, 1H, *J* = 2.4, 6 Hz), 6.85 (d, 2H, *J* = 8.5 Hz), 7.22 (t, 1H, *J* = 7.8 Hz), 7.8 (dd, 1H, *J* = 1.8, 8.4 Hz), 8.13 (d, 1H, *J* = 8.4 Hz), 8.46 (d, 1H, *J* = 1.8 Hz), 9.18 (s, 1H).

¹³C NMR (150 MHz, CDCl₃) δ 19.91, 24.61, 27.69, 31.22, 37.87, 42.81, 55.27, 55.91, 55.92, 65.00, 76.54, 111.34, 111.73, 112.91, 114.16, 119.88, 120.14, 121.92, 123.95, 124.83, 129.78, 133.20, 133.83, 137.56, 141.50, 147.39, 148.87, 154.92, 157.56, 158.03, 169.95, 171.84

MS (ESI) m/z: found RT 11.34 min. (Method LCMS), 655.06 [M + 1] ⁺, 677.16 [M + Na]⁺, HRMS 655.2286 [M + H]⁺, calculated 655.1706.

### Example 4

### 2-(3-((R)-3-(3,4-dimethoxyphenyl)-1-((S)-1-(furan-3-ylsulfonyl)piperidine-2-carbonyloxy) propyl)phenoxy)acetic acid (compound 4)

TLC (hexane:EtOAc: TFA 6:3.8:0.2): Rf = 0.375, Yield- 25mg, , % Yield- 56%. HPLC (MeCN: H₂O CF₃COOH (0.1 %) gradient of 0 - 100% in 45 min at 1ml/min) retention time- 24.4-24.8 min

¹HNMR (300 MHz, CDCl₃) δ 1.10-1.18 (m,1H), 1.43-1.52 (m,1H), 1.61-1.81(m,2H), 2.00-2.12(m,1H), 2.19-2.30 (m, 2H), 2.37 (s,2H), 2.52-2.65 (m,2H), 3.14-3.28 (m,2H), 3.87(s,3H), 3.88 (s,3H), 4.68 (s, 2H), 4.82 (d, 1H, *J* = 4.5 Hz), 5.70 (dd. 1H, *J* = 2.1,5.7 Hz), 6.58 (q,1H, *J* = 0.9, 1.2Hz), 6.70 (dd, 2H, *J* = 2.1, 4.5 Hz), 6.79-6.87 (m,2H), 6.91-6.96 (m, 2H), 7.25 (d, 1H, *J* = 2.1 Hz), 7.44 (t, 1H, *J* = 2.4 Hz), 7.91 (q, 1H, *J* = 0.9 Hz).

¹³C NMR (75 MHz, CDCl₃) δ 21.45, 24.62, 27.72, 31.25, 37.92, 42.57, 55.18, 55.93, 55.94, 64.92, 76.40, 108.52, 111.40, 111.80, 112.88, 114.30, 119.97, 120.20, 127.37, 128.21, 133.29, 141.86, 144.42, 145.50, 147.42, 148.92, 157.66, 170.10 MS (ESI) m/z: found RT 11.29 min. (Method LCMS), 610.16 [M + Na]⁺, HRMS 588.2354 [M + H]⁺, calculated 588.1825.

### Example 5

### 2-(3-((R)-1-((S)-1-(benzo[b]thiophen-2-ylsulfonyl)piperidine-2-carbonyloxy)-3-(3,4-dimethoxy phenyl propyl)phenoxy)acetic acid (compound 5)

TLC (hexane:EtOAc: TFA 6:3.8:0.2): Rf = 0.625, Yield- 23.8 mg, % Yield- 55%. HPLC (MeCN: H₂O CF₃COOH (0.1 %) gradient of 0 - 100% in 45 min at 1ml/min) retention time- 21.8-22.2 min

¹HNMR (300 MHz, CDCl₃) δ 1.12- 1.21 (m,1H), 1.44-1.52(m,1H), 1.60-1.64 (m,2H), 1.70-1.81 (m,1H), 1.96-2.07 (m,1H), 2.14-2.26(m,2H). 2.47-2.65(m,2H), 3.36 (dt, 1H, *J* = 3 Hz), 3.86 (s,3H), 3.87 (s, 3H), 4.66 (s,2H), 4.87 (d, 1H, *J* = 4.2 Hz), 5.65 (dd, 1H, *J* = 2.4, 6.6 Hz), 6.66-6.69 (m,2H), 6.77-6.85 (m,2H), 6.91 (d, 2H, *J* = 7.2), 7.24 (t, 1H, *J* = 8.1 Hz), 7.40-7.49(m, 2H), 7.79-7.84 (m,3H).

¹³C NMR (75 MHz, CDCl₃) δ 19.87, 24.47, 27.45, 31.28, 37.98, 43.04, 55.47, 55.93, 55.94, 64.90, 76.44, 111.39, 111.84, 112.73, 114.36, 119.95, 120.22, 122.66, 125.36, 125.58, 127.08, 128.91, 129.76, 133.36, 137.63, 141.35, 141.61, 141.82, 147.39, 148.90, 157.59, 169.84.

MS (ESI) m/z: found RT 12.28 min. (Method LCMS), 676.95 [M + Na]⁺, HRMS 732.2501 [M + DMSO]⁺, calculated 654.1753.

### Example 6

### 2-(3-((R)-1-((S)-1-(3-cyanophenylsulfonyl)piperidine-2-carbonyloxy)-3-(3,4-dimethoxyphen -yl )propyl)phenoxy)acetic acid (compound 6)

Yield- 29.5mg, % Yield- 61 %.

¹HNMR (600 MHz, CDCl₃) δ 1.02- 1.10 (m,1H), 1.40-1.47 (m,1H), 1.60-1.64 (m,2H), 1.72-1.78 (m,1H), 1.99-2.06 (m,1H), 2.16-2.22 (m,2H), 2.49-2.59 (m,2H), 3.15(dt,1H, *J* = 3 Hz, 13.2 Hz),3.7 (d, 1H, *J* = 9.6 Hz), 3.86 (s,3H), 3.87 (s, 3H), 4.67 (s,2H), 4.81 (d, 1H, *J* = 4.8 Hz), 5.60 (dd, 1H, *J* = 6 Hz), 6.67-6.69 (m,2H), 6.81 (d, 1H, *J* = 7.8 Hz), 6.83-6.84 (m, 1 H), 6.90 (d,1H, *J* = 7.8 Hz), 7.26 (dd, 1H, *J* = 7.8 Hz), 7.56 (t, 1H, *J* = 7.8 Hz), 7.78 (td, 1H, *J* = 1.2 Hz, 7.8 Hz), 7.99-7.99 (m, 1H), 8.07 (t, 1H, *J* = 1.8Hz).

¹³C NMR (150 MHz, CDCl₃) δ 19.83, 24.63, 27.80, 31.25, 37.82, 42.88, 55.39, 55.93, 55.97, 64.78, 76.70, 111.37, 111.82, 112.95, 113.38, 114.23, 117.30, 120.00, 120.16, 129.87, 130.70, 131.05, 133.16, 135.56, 141.58, 141.77, 147.43, 148.88, 157.53, 169.67, 171.85.

MS (ESI) m/z: found RT 13.12min. (Method LCMS), 645.40 [M + Na]⁺, HRMS 701.2628 [M + DMSO]⁺, calculated 623.1985.

### Example 7

### 2-(3-((R)-3-(3,4-dimethoxyphenyl)-1-((S)-1-(3-nitrophenylsulfonyl)piperidine-2-carbonylo -xy propyl)phenoxy)acetic acid (compound 7)

TLC (hexane:EtOAc: TFA 5.5:4.5:0.2): Rf = 0.44, Yield- 24.0 mg, % Yield- 48%. HPLC (MeCN: H2O CF3COOH (0.1 %) gradient of 0 - 100% in 45 min at 1ml/min) retention time- 24.4-24.8 min

¹HNMR (600 MHz, CDCl₃) δ 1.00- 1.01 (m,1H), 1.43-1.49 (m,1H), 1.60-1.63 (m,2H), 1.74-1.80 (m,1H), 1.98-2.04 (m,1H), 2.15-2.23 (m,2H), 2.46-2.57 (m,2H), 3.15 (dt,1H, J = 2.4 Hz, 12.6 Hz), 3.74 (td, 1H, J = 2.4, 9.6 Hz), 3.85 (s,3H), 3.86 (s, 3H), 4.65 (s,2H), 4.85 (d, 1H, J = 4.2 Hz), 5.57 (dd, 1H, J = 6.6 Hz), 6.65-6.67 (m,2H), 6.79-6.82 (m, 3H), 6.87 (d, 1H, J = 7.8 Hz), 7.24 (dd, 1H, J = 7.8 Hz), 7.62 (t, 1H, J = 7.8 Hz), 8.06-8.08 (m, 1H), 8.34-8.36 (m, 1H), 8.59(t, 1H, J = 1.8 Hz).

¹³C NMR (150 MHz, CDCl₃) δ 19.81, 24.65, 27.86, 31.21, 37.76, 42.88, 55.47, 55.92, 55.93, 64.76, 76.73, 111.35, 111.77, 112.98, 114.14, 120.12, 122.34, 126.91, 129.85, 130.13, 132.62, 133.17, 141.53, 142.09, 147.40, 148.07, 148.87, 157.55, 169.65, 172.65

MS (ESI) m/z: found RT 13.40 min. (Method LCMS), 665.25 [M + Na]⁺, HRMS 721.2027 [M + DMSO]⁺, calculated 643.1883.

### Example 8

### 2-(3-((R)-1-((S)-1-(3-aminophenylsulfonyl)piperidine-2-carbonyloxy)-3-(3,4-dimethoxyphenyl)propyl)phenoxy)acetic acid (compound 8),

TLC (hexane:EtOAc: TFA 6:3.8:0.2): Rf = 0.28, Yield- 35 mg, % Yield-66%. HPLC (gradient A) retention time- 21.32-21.52 min

¹HNMR (600 MHz, CDCl₃) δ 1.21-1.29 (m, 1H), 1.46-1.52 (m, 1H), 1.60-1.66 (m, 1H), 1.69-1.74 (m, 1H), 1.79- 1.86 (m, 1H), 1.89-1.96 (m, 1H), 2.07-2.13 (m, 1H), 2.42-2.56 (m, 2H), 3.10-3.25 (m, 1H), 3.74-3.79 (m, 1H), 3.84 (s, 6H), 4.66 (s, 2H), 4.70-4.74 (m, 1H), 5.53 (s, 1H), 6.60-6.68 (m, 3H), 6.74-6.80 (m, 2H), 6.82-6.86 (m, 2H), 7.13-7.20 (m, 2H), 7.36-7.54 (m, 2H).

¹³C NMR (150 MHz, CDCl₃) δ 20.06, 24.80, 28.03, 31.36, 37.99, 42.76, 55.20, 55.89, 55.92, 65.02, 76.38, 111.33, 111.68, 112.54, 114.44, 118.69, 119.84, 120.12, 123.19, 124.25, 129.94, 130.21, 133.19, 139.69, 140.92, 141.93, 147.39, 148.87, 157.64, 169.72, 172.07

MS (ESI) m/z: found RT 10.87 min. (Method LCMS), 613.12 [M + 1]⁺, 635.17 [M + Na]⁺, HRMS 613.2704 [M + 1]⁺, calculated 613.2142.

### Example 9

### 2-(3-((R)-3-(3,4-dimethoxyphenyl)-1-((S)-1-(3-(2-methylpyrimidin-4-yl)phenyl sulfonyl) piperidine-2-carbonyloxy)propyl)phenoxy)acetic acid (compound 9)

TLC (hexane:EtOAc: TFA 6:3.8:0.2): Rf = 0.22, Yield- 48.3 mg, % Yield- 90%. HPLC (MeCN: H2O CF3COOH (0.1 %) gradient of 0 - 100% in 45 min at 1ml/min) retention time- 23.1-23.5 min

¹HNMR (600 MHz, CDCl₃) δ 1.14- 1.21 (m,1H), 1.51-1.58(m,1H), 1.61-1.63 (m,1H), 1.71-1.75 (m,1H), 1.83-1.90 (m,1H), 1.96-2.02 (m,1H). 2.12-2.18 (m,1H), 2.32 (d, 1H, J = 13.8 Hz), 2.45- 2.49 (m,1H), 2.52-2.57(m,1H),3.66 (d, 1H, J = 12 Hz), 3.79 (s,3H), 3.80 (s, 3H), 4.59 (d,1H, J = 16.8 Hz), 4.67 (d, 2H, J = 16.8 Hz), 4.98 (d, 1H, J = 5.4 Hz), 5.51 (dd, 1H, J = 5.4 Hz), 6.66-6.64 (m,2H), 6.75 (m, 2H, J= 8.4 Hz), 6.81-6.86 (m, 2H), 7.26 (m, 1H), 7.66 (t, 1H, J = 7.8 Hz), 8.01 (d, 1H, J = 6 Hz), 8.01- 8.08 (m, 1H), 8.41-8.43(m,1H), 8.73 (t, 1H, J = 1.8 Hz), 9.07 (d, 1H, J = 6.6 Hz).

¹³C NMR (150 MHz, CDCl₃) δ 20.19, 22.94, 24.75, 28.08, 31.23, 37.79, 43.03, 55.21, 55.87, 55.88, 65.36, 77.06, 111.41, 111.61, 112.78, 115.21, 115.33, 118.94, 120.22, 127.02, 129.86, 130.19, 131.83, 132.08, 133.02, 134.97, 141.62, 141.84, 147.39, 148.85, 151.43, 157.99, 164.28, 167.69, 170.58, 171.54 MS (ESI) m/z: found RT 12.59 min. (Method LCMS), 690.37 [M + 1]⁺, 712.17 [M + Na]⁺, HRMS 690.2936 [M + 1]⁺, calculated 690.2407.

### Example 10

### 2-(3-((R)-3-(3,4-dimethoxyphenyl)-1-((S)-1-(3-(pyrimidin-4-yl)phenyl sulfonyl)piperidine-2-carbonyloxy)propyl)phenoxy)acetic acid (compound 10)

TLC (hexane:EtOAc: TFA 5:4.8:0.2): Rf = 0.43, Yield- 41.9 mg, % Yield- 80%. HPLC (MeCN: H2O CF3COOH (0.1 %) gradient of 0 - 100% in 45 min at 1ml/min) retention time- 24.2-24.6 min

¹HNMR (600 MHz, CDCl₃) δ 1.10- 1.17 (m,1H), 1.37-1.44 (m,1H), 1.58-1.61 (m,2H), 1.66-1.72 (m,1H), 1.94-2.00 (m,1H), 2.13-2.19 (m,2H). 2.42-2.47 (m,1H), 2.50- 2.55 (m,1H), 3.25(dt,1H, J= 3 Hz, 12.6 Hz),3.83 (d, 1 H, J = 6 Hz), 3.85 (s,3H), 3.85 (s, 3H), 4.67 (d, 2H, J =6 Hz), 4.83 (d, 1H, J = 4.2 Hz), 5.60 (dd, 1H, J =5.4 Hz),6.62-6.64 (m,2H), 6.77 (d, 1H, J =8.4 Hz), 6.80-6.82 (m, 1H), 6.86-6.88 (m.2H), 7.22 (t, 1H, J =8.4 Hz), 7.28 (t, 1H, J = 4.8 Hz), 7.59 (t, 1H, J = 7.8 Hz), 7.92-7.94 (m, 1H), 8.55 (dd, 1H, J = 1.2 Hz, 7.8Hz), 8.85 (d, 3H, J = 4.8 Hz).

¹³C NMR (150 MHz, CDCl₃) δ 19.99, 24.53, 27.41, 31.28, 38.02, 42.93, 55.35, 55.88, 55.92, 64.85, 111.31, 111.74, 112.30, 114.60, 119.88, 119.95, 120.17, 127.01, 129.17, 129.45, 129.71, 132.08, 133.42, 137.71, 141.12, 141.77, 147.27, 148.79, 157.41, 157.63, 162.78, 170.08, 172.04.

MS (ESI) m/z: found RT 13.30 min. (Method LCMS), 676.20 [M + 1]⁺, 698.33 [M + Na]⁺, HRMS 676.2783 [M + 1]⁺, calculated 676.2251.

### Example 11

### 2-(3-((R)-3-(3,4-dimethoxyphenyl)-1-((S)-1-(3-fluorophenylsulfonyl)piperidine-2-carbonyloxy)propyl)phenoxy)acetic acid (compound11),

TLC (hexane: EtOAc: TFA 5.5:4.5:0.2): Rf = 0.44, Yield- 23.5 mg, % Yield- 49%. HPLC (gradient A) retention time- 24.70-24.85 min

¹HNMR (300 MHz, CDCl₃) δ 1.24-1.33 (m,1H), 1.41-1.53(m,1H), 1.63-1.85 (m, 3H), 1.94-2.06 (m,1H), 2.12-2.27 (m, 2H), 2.45-2.61 (m, 2H), 3.18 (t, 1H, J= 12.7 Hz), 3.74 (d, 1H, J= 12.6 Hz), 3.85 (s, 6H), 4.71 (s, 2H), 4.80 (d, 1H, J= 4.8Hz), 5.63 (t, 1H, J= 7.8 Hz), 6.65-6.67 (m, 2H), 6.75 (d, 1H, J= 8.7 Hz), 6.89- 6.93 (m, 3H), 7.13- 7.34 (m, 3H), 7.39-7.41 (m, 2H).

¹³C NMR (75 MHz, CDCl₃) δ 20.05, 24.62, 27.89, 31.24, 37.95, 42.77, 55.37, 55.88, 55.93, 65.32, 76.34, 111.38, 111.78, 112.56, 114.18, 114.50, 119.43, 119.71, 120.18, 122.83, 129.86, 130.49, 133.29, 141.77, 147.39, 148.89, 157.75, 160.53, 163.86, 165.31, 169.73.

MS (ESI) m/z: found RT 13.19 min. (Method LCMS), 638.19 [M + Na]⁺, HRMS 616.2488 [M + 1]⁺ calculated 616.1938.

### Example 12

### 2-(3-((R)-1-((S)-1-(3-bromophenylsulfonyl)piperidine-2-carbonyloxy)-3-(3,4-dimethoxyphenyl)propyl)phenoxy)acetic acid (compound 12),

TLC (hexane:EtOAc: TFA 5.5:4.5:0.2): Rf = 0.36, Yield- 29.87mg, % Yield- 57%. HPLC (gradient A) retention time- 25.89-26.02 min

¹HNMR (300 MHz, CDCl₃) δ 1.24-1.32 (m, 1H), 1.39-1.52 (m,1H), 1.62-1.85 (m, 3H), 1.94- 2.05(m, 1H), 2.11-2.27(m, 2H), 2.44-2.62 (m, 2H), 3.16 (dt, 1H, J= 2.7; 12.6 Hz), 3.71 (d, 1H, J= 9.9 Hz), 4.70 (s, 2H), 4.80 (d, 1H, J= 4.5 Hz), 5.65 (t, 1h, J= 8.1 Hz), 6.65-6.68 (m, 2H), 6.79 (d, 1H, J= 8.7 Hz), 6.88-6.93 (m, 3H), 7.11 (t, 1H, J= 8.1 Hz), 7.31 (t, 1H, J= 8.25 Hz), 7.52 (dd, 1H, J= 0.9; 7.8 Hz), 7.59 (dd, 1H, J= 0.9, 7.9 Hz), 7.87 (t, 1H, J= 1.8Hz).

¹³C NMR (75 MHz, CDCl₃) δ 20.06, 24.59, 27.87, 31.23, 37.96, 42.79, 55.48, 55.89, 55.94, 64.86, 76.43, 111.41, 111.80, 112.60, 114.53, 120.09, 120.21, 122.73, 125.64, 129.89, 130.33, 133.32, 135.46, 141.60, 141.75, 147.37, 148.86, 157.75, 169.81, 172.68.

MS (ESI) m/z: found RT 13.33 min. (Method LCMS), 698.31 [M + Na]⁺, 700.12 [M + 2 + Na]⁺, HRMS 676.1716 [M + 1]⁺, 678.1715 [M + 3]⁺, calculated 676.1138.

### Example 13

### 2-(3-((R)-1-((S)-1-(3,5-dichlorophenylsulfonyl)piperidine-2-carbonyloxy)-3-(3,4-dimethoxy-phenyl)propyl)phenoxy)acetic acid (compound 13)

TLC (hexane:EtOAc: TFA 5.5:4.5:0.2): Rf = 0.58, Yield- 22.6mg, % Yield- 44%. HPLC (MeCN: H2O CF3COOH (0.1 %) gradient of 0 - 100% in 45 min at 1ml/min) retention time- 26.8-27.3 min

¹HNMR (600 MHz, CDCl₃) δ 1.03- 1.10 (m,1H), 1.40-1.47 (m,1H), 1.60-1.63 (m,2H), 1.71-1.77 (m,1H), 1.99-2.06 (m,1H), 2.16-2.23 (m,2H), 2.49-2.59 (m,2H), 3.20 (dt,1H, J = 2.4 Hz, 12.6 Hz), 3.71 (td, 1H, J = 1.2, 12.6 Hz), 3.85 (s,3H), 3.86 (s, 3H), 4.66 (s,2H), 4.78 (d, 1H, J = 4.8 Hz), 5.63 (dd, 1H, J = 6 Hz), 6.66-6.68 (m,2H), 6.79 (d, 1H, J = 7.8 Hz), 6.83-6.84 (m, 1H), 6.85 (t,1H, J = 2.4 Hz), 6.90 (d, 1H, J = 7.8 Hz), 7.26 (dd, 1H, J = 7.8 Hz), 7.49 (t, 1H, J = 1.8 Hz), 7.66 (d, 1H, J = 1.8 Hz).

¹³C NMR (150 MHz, CDCl₃) δ 19. 74, 24.61, 27.74, 31.28, 37.84, 42.89, 55.32, 55.92, 64.78, 76.65, 111.33, 111.75, 112.95, 114.17, 120.00, 120.17, 125.45, 129.87, 132.42, 133.21, 135.73, 141.63, 142.89, 147.40, 148.86, 157.53, 169.57, 172.53. MS (ESI) m/z: found RT 14.33 min. (Method LCMS), 688.19 [M + Na]⁺, 690.19 [M + 2+ Na]⁺, HRMS 666.1753 [M + 1]⁺, 668.1713 [M + 3]⁺, calculated 666.1253.

### Example 14

### 2-(3-((R)-1-((S)-1-(2,3-dichlorophenylsulfonyl)piperidine-2-carbonyloxy)-3-(3,4-dimethoxy phenyl)propyl)phenoxy)acetic acid (compound 14)

TLC (hexane:EtOAc: TFA 6:3.8:0.2): Rf = 0.50, Yield- 18.7 mg

HPLC (MeCN: H2O CF3COOH (0.1 %) gradient of 0 - 100% in 45 min at 1ml/min) retention time- 27.1-27.5 min

MS (ESI) m/z: found RT 12.79 min. (Method LCMS), 688.14 [M + Na]⁺, 690.11 [M + 2+ Na]⁺, HRMS 688.1090 [M + Na]⁺, 690.1061 [M + 2 + Na]⁺, calculated 666.1253.

### Example 15

### 2-(3-((R)-3-(3,4-dimethoxyphenyl)-1-((S)-1-(3,4-dimethoxyphenylsulfonyl) piperidine-2-carbonyloxy)propyl)phenoxy)acetic acid (compound 15)

Yield- 32.89 mg, % Yield- 62%.

HPLC (MeCN: H2O CF3COOH (0.1 %) gradient of 0 - 100% in 45 min at 1ml/min) retention time- 23.1-23.5 min

¹HNMR (600 MHz, CDCl₃) δ 1.08- 1.15 (m,1H), 1.32-1.39(m,1H), 1.53-1.60(m,2H), 1.64-1.70(m,1H), 1.99-2.06 (m,1H), 2.14-2.23(m,2H). 2.48-2.59(m,2H), 3.19 (dt, 1H, J = 3, 10.2 Hz), 3.63 (d,1H, J = 11.4Hz), 3.83 (s,3H), 3.84 (s,3H), 3.87 (s,3H), 3.87 (s, 3H), 4.65 (s,2H), 4.78 (d, 1H, J = 4.8 Hz), 5.64 (dd, 1H, J = 1.2, 6.0 Hz), 6.66 (d,2H, J = 7.2 Hz), 6.73 (s, 1H, J = 8.4Hz), 6.81- 6.83(m, 2H), 6.88-6.91 (m, 2Hz), 7.22-7.24(m,2H), 7.41 (dd,1H, J = 0.6, 8.4Hz).

¹³C NMR (150 MHz, CDCl₃) δ 20.04, 24.43, 27.53, 31.25, 37,98,42.63, 55.16, 55.88,55.89, 56.06, 56.17, 64.89, 76.48, 109.76, 110.41, 111.32, 111.74, 112.74, 114.40, 119.85, 120.16, 121.03, 129.75, 131.88, 133.30, 141.79, 147.34, 148.84, 148.93, 152.41, 157.58

MS (ESI) m/z: found RT 12.83 min. (Method LCMS), 680.21 [M + Na]⁺, HRMS 680.2753 [M + Na]⁺, calculated 658.2244.

### Example 16

### 2-(3-((R)-1-((S)-1-(3-chloro-4-methoxyphenylsulfonyl)piperidine-2-carbonyl oxy)-3-(3,4-dimethoxyphenyl)propyl)phenoxy)aceticacid (compound 16),

TLC (hexane:EtOAc: TFA 6:3.8:0.2): Rf = 0.45, Yield- 11.7mg, % Yield- 25%.

HPLC (gradient A) retention time- 24.98-25.12 min

¹HNMR (300 MHz, CDCl₃) δ 1.25-1.34 (m, 1H), 1.41-1.54 (m, 1H), 1.63-1.85 (m, 3H), 1.95-2.06 (m, 1H), 2.13-2.27 (m, 2H), 2.46-2.64 (m, 2H), 3.15 (dt, 1H, J= 2.7, 12.6 Hz), 3.69 (d, 1H, J= 12.1 Hz), 3.86 (s, 6H), 3.89 (s, 3H), 4.72 (s, 2H), 4.81 (d, 1H, J= 4.5 Hz), 5.66 (t, 1H, J= 6.6 Hz), 6.65-6.72 (m, 3H), 6.78 (d, 1H, J= 8.7 Hz), 6.91- 6.96 (m,3H), 7.32 (t, 1H, J= 8.3 Hz), 7.47 (dd, 1H, J= 2.4, 9.3 Hz), 7.72 (d, 1H, J= 2.4 Hz) ¹³C NMR (75 MHz, CDCl₃) δ 20.09, 24.60, 27.93, 31.25, 38.11, 42.63, 55.31, 55.90, 55.94, 56.37, 64.87, 76.21, 111.33, 111.38, 111.79, 112.22, 114.68, 120.14, 120.19, 122.95, 127.55, 129.16, 129.85, 132.20, 133.30, 142.00, 147.40, 148.89, 157.74, 158.07, 169.87, 172.04.

MS (ESI) m/z: found RT 13.48 min. (Method LCMS), 684.27 [M + Na]⁺, HRMS 662.2304 [M + 1]⁺, 664.2288 [M + 3]⁺, calculated 662.1748.

### Example 17

### 2-(3-((R)-1-((S)-1-(3,5-difluorophenylsulfonyl)piperidine-2-carbonyloxy)-3-(3,4-dimethoxyphenyl)propyl)phenoxy)acetic acid (compound 17),

TLC (hexane:EtOAc: TFA 6:3.8:0.2): Rf = 0.38, Yield- 23.5mg, % Yield- 57%.

HPLC (gradient A) retention time- 25.22-25.39 min

¹HNMR (300 MHz, CDCl₃) δ 1.21-1.35 (m, 1H), 1.42-1.55 (m, 1H), 1.65-1.84 (m, 3H), 1.96-2.08 (m, 1H), 2.14-2.30 (m, 2H), 2.45-2.63 (m, 2H), 3.17 (t, 1H, J= 12 Hz), 3.73 ( d, 1H, J= 11.9 Hz), 3.85 (s, 6H), 4.78 (s, 2H), 4.72 (s, 1H), 5.66 (t, 1H, J= 6 Hz), 6.65-6.68 (m, 2H), 6.79 (d, 1H, J = 4.4 Hz), 6.87-6.93 ( m, 4H), 7.20 (d, 2H, J= 1.8 Hz), 7.31 (t, 1H, J= 7.5 Hz).

¹³C NMR (75 MHz, CDCl₃) δ 20.09, 24.66, 27.88, 31.23, 37.83, 42.99, 53.42, 55.59, 55.87, 55.93, 77.23, 107.66, 107.98, 110.43, 110.79, 111.39, 111.76, 112.74, 114.49, 120.09, 120.18, 129.93, 133.26, 141.51, 143.17, 147.40, 148.88, 157.69, 160.92, 164.13, 169.63.

MS (ESI) m/z: found RT 13.46min. (Method LCMS), 656.19 [M + Na]⁺, HRMS 634.7102 [M + 1]⁺, calculated 634.6568.

### Example 18

### 2-(3-((R)-1-((S)-1-(3,5-bis(trifluoromethyl)phenylsulfonyl)piperidine-2-car-bonyloxy)-3-(3,4-dimethoxyphenyl)propyl)phenoxy)acetic acid (compound 18)

TLC (hexane:EtOAc: TFA 6:3.8:0.2): Rf = 0.47, Yield- 30.1 mg, % Yield- 51 %.

HPLC (MeCN: H2O CF3COOH (0.1 %) gradient of 0 - 100% in 45 min at 1ml/min) retention time- 27.5-27.8 min

¹HNMR (600 MHz, CDCl₃) δ 0.99- 1.01 (m,1H), 1.40-1.47(m,1H), 1.59-1.61 (m,2H), 1.73-1.79 (m,1H), 1.99-2.05 (m,1H), 2.14-2.23(m,2H). 2.46-2.56(m,2H), 3.09 (dt, 1H, J = 2.4 Hz),3.69 (dd, 1H, J = 1.8 Hz), 3.84 (s,3H), 3.85 (s, 3H), 4.64 (s,2H), 4.83 (d, 1H, J = 4.2 Hz), 5.56 (t, 1H, J = 6.6 Hz), 6.63-6.66 (m,2H), 6.78(d,1H, J = 7.8 Hz), 6.80-6.82 (m,2H), 6.88(d, 2H, J= 7.8), 7.24 (t, 1H, J =7. 8 Hz), 8.031 (s, 1H), 8.20(s, 1H).

¹³C NMR (150 MHz, CDCl₃) δ 19.74, 24.58, 27.88, 31.21, 37.71, 42.83, 55.59, 55.87, 55.88, 64.73, 76.92, 111.31, 111.71, 113.02, 114.13, 119.98, 120.09, 121.62, 123.44, 125.27, 127.29, 127.51, 129.87, 133.12, 141.57, 142.63, 147.39, 148.83, 157.48, 169.47, 172.22 MS (ESI) m/z: found RT 14.65 min. (Method LCMS), 756.20 [M + Na]⁺, HRMS 812.2680 [M + DMSO]⁺, calculated 734.1780.

### Example 19

### 2-(3-((R)-1-((S)-1-(3-bromo-5-(trifluoromethyl)phenylsulfonyl)piperidine-2-carbo-nyloxy)-3-(3,4-dimethoxyphenyl)propyl)phenoxy)acetic acid (compound 19)

TLC (hexane:EtOAc: TFA 6:3.8:0.2): Rf = 0.50, Yield- 28.5mg, % Yield- 47%.

HPLC (MeCN: H2O CF3COOH (0.1 %) gradient of 0 - 100% in 45 min at 1ml/min) retention time- 27.5-27.8 min

¹HNMR (600 MHz, CDCl₃) δ 0.97-1.04 (m,1H), 1.40-1.46(m,1H), 1.59-1.62(m,2H), 1.71-1.77(m,1H), 1.99-2.05(m,1H), 2.15-2.22(m,2H). 2.47-2.57(m,2H), 3.15 (dt, 1H, J 2.4, 12.6 Hz, ), 3.68 (dd, 1H, J = 2.6Hz), 3.84 (s,3H), 3.85 (s, 3H), 4.65 (s,2H), 4.80 (d, 1H, J = 4.8 Hz), 5.59 (t, 1H, J = 7.2 Hz), 6.64-6.67 (m,2H), 6.78 (d, 1H, J = 8.4Hz), 6.81-6.84(m, 2H), 6.89(d,1H, J = 7.2Hz), 7.25 (t, 1H, J = 7.8Hz), 7.90(s, 1H), 7.95 (s,1H), 8.08(s,1H)

¹³C NMR (150 MHz, CDCl₃) δ 19.74, 24.59, 27.80, 31.24, 37.77, 42.86, 55.45, 55.90, 64.75, 76.77, 111.32, 111.74, 112.98, 114.15, 119,99, 120.14, 122.80, 123.32, 129.87, 132.12, 132.15, 133.17, 133.25, 141.60, 142.89, 147.39, 148.84, 157.49, 169.49, 172.39.

MS (ESI) m/z: found RT 14.60 min. (Method LCMS), 766.16 [M + Na]⁺, 768.08 [M + 2+ Na]⁺, HRMS 766.1568 [M + Na] ⁺, 768.1556 [M + 2+ Na] ⁺, calculated 744.1011.

### Example 20

### 2-(3-((R)-1-((S)-1-(3,5-bis(methoxycarbonyl)phenylsulfonyl)piperidine-2-carbonyl oxy)-3-(3,4-dimethoxyphenyl)propyl)phenoxy)acetic acid (compound 20)

TLC (hexane:EtOAc: TFA 6:3.8:0.2): Rf = 0.52, Yield- 22mg, % Yield- 41 %.

HPLC (gradient A) retention time- 24.82-25.02 min

¹HNMR (400 MHz, CDCl₃) δ 1.21-1.31 (m, 1H), 1.37-1.47 (m, 1H), 1.61-1.80 (m, 3H), 1.91-2.00 (m, 1H), 2.08-2.17 (m, 1H), 2.28 (d, 1H, J= 6.8 Hz), 2.40-2.55 (m, 2H), 3.15 (dt, 1H, J= 2.8, 12.8 Hz), 3.78 (d, 1H, J= 12.6 Hz), 3.82 (s, 3H), 3.83 (s, 3H), 3.93 (s, 6H), 4.70 (s, 2H), 4.81 (s, 1H, J= 2.4 Hz), 5.63 (t, 1H, J= 6.8 Hz), 6.61-6.63 (m, 2H), 6.75 (d, 1H, J= 4.2 Hz), 6.84-6.88 (m, 3H), 7.23-7.27 (m, 1H), 8.54 (s, 1H), 8.55 (s, 1H), 8.76 (t, 1H, J= 1.6 Hz).

¹³C NMR (100 MHz, CDCl₃) δ 20.21, 24.65, 27.77, 31.13, 37.76, 43.04, 52.85, 55.61, 55.84, 55.89, 64.91, 76.47, 111.27, 111.68, 112.70, 114.57, 120.08, 120.10, 129.79, 131.53, 131.95, 133.25, 134.01, 141.30, 141.73, 147.31, 148.80, 157.58, 164.86, 169.61, 172.02. MS (ESI) m/z: found RT 11.80 min. (Method LCMS), 736.16 [M + Na]⁺, HRMS 714.2720 [M + 1]⁺, calculated 714.2142.

### Example 21

### 2-(3-((R)-1-((S)-1-(3,5-dichloro-4-hydroxyphenylsulfonyl)piperidine-2-carbo nyloxy)-3-(3,4-dimethoxyphenyl)propyl)phenoxy)acetic acid (compound 21)

TLC (hexane:EtOAc: TFA 4.8 :5:0.2): Rf = 0.50, Yield- 12.3mg, % Yield- 23%. HPLC (MeCN: H2O CF3COOH (0.1 %) gradient of 0 - 100% in 45 min at 1 ml/min) retention time- 26.8-27.1 min

¹HNMR (300 MHz, CDCl₃) δ 1.00-1.10 (m, 1H), 1.38-1.53 (m, 1H), 1.60-1.68 (m, 2H), 1.74-1.87 (m, 1H), 2.01-2.12 (m, 1H), 2.16-2.26 (m, 2H), 2.53-2.62 (m, 2H), 3.21 (t, 1H, J= 12Hz), 3.72 (d, 1H, J= 12Hz), 3.87 (s, 6H), 4.65 (s, 2H), 4.77 (d, J= 2.7Hz), 5.63 (t, J= 6Hz), 6.69-6.71 (m, 2H), 6.80-6.93(m, 4H), 7.28 (s, 1H), 7.78 (s, 1H), 7.99 (s, 1H).

¹³C NMR (75 MHz, CDCl₃) δ 19.66, 24.66, 27.85, 31.34, 37.88, 43.01, 55.4, 55.95, 64.82, 77.21, 111.44, 111.83, 113.02, 114.23, 119.84, 120.21, 122.20, 127.86, 128.97, 129.89, 139.94, 141.75, 145.83, 147.22, 148.89, 153.52, 157.56, 169.51, 172.35

MS (ESI) m/z: found Rt 13.66 min. (Method LCMS), 704.68 [M + Na]⁺.

HRMS 682.1962, 684.1949 [M + H]⁺, calculated 682.1902 [M + H]⁺.

### Example 22

### 2-(3-((R)-1-((S)-1-(3,5-dichloro-4-methoxyphenylsulfonyl)piperidine-2-carbo nyloxy)-3-(3,4-dimethoxyphenyl)propyl)phenoxy)acetic acid (compound 22)

TLC (hexane: EtOAc: TFA 6:3.8:0.2): Rf = 0.41, Yield- 18.3mg, % Yield- 39%.

HPLC (gradient B) retention time- 11.6-11.9 min

¹HNMR (600 MHz, CDCl₃) δ 1.02-1.09 (m,1H), 1.39-1.46(m,1H), 1.59-1.62(m,2H), 1.70-1.76(m,1H), 1.99-2.05(m,1H), 2.15-2.23(m,2H). 2.49-2.59(m,2H), 3.18 (dt, 1H, J 2.4, 12.6 Hz, ), 3.68 (dd, 1H, J = 3, 12.6 Hz), 3.84 (s,3H), 3.85 (s, 3H), 3.92 (s, 3H), 4.66 (s,2H), 4.76 (d, 1H, J = 4.8 Hz), 5.61 (t, 1H, J = 6.6 Hz), 6.64-6.67 (m,2H), 6.78 (d, 1H, J = 8.4Hz), 6.81-6.84 (m, 2H), 6.89 (d,1H, J = 7.8Hz), 7.25 (t, 1H, J = 7.8Hz), 7.71 (s, 2H)

¹³C NMR (150 MHz, CDCl₃) δ 19.78, 24.58, 27.70, 31.28, 37.83, 42.84, 55.31, 55.90, 60.95, 64.85, 76.64, 111.34, 111.76, 112.90, 114.23, 120.01, 120.17, 127.73, 129.85, 130.06, 133.19, 136.95, 141.66, 147.37, 148.84, 155.65, 157.48, 169.67 MS (ESI) m/z: found RT 11.84 min. (Method LCMS), 718.37 [M + Na]⁺, 720.26 [M + Na + 2]⁺, HRMS 696.1423 [M + 1]⁺, 698.2819 [M + 3]⁺, calculated 696.1359.

### Example 23

### 2-(3-((R)-1-((S)-1-(4-acetamido-3,5-dichlorophenylsulfonyl)piperidine-2-carbo nyloxy)-3-(3,4-dimethoxyphenyl)propyl)phenoxy)acetic acid (compound 23),

TLC (hexane: EtOAc: TFA 6:3.8:0.2): Rf = 0.48, Yield- 10.98 mg, % Yield- 21 %. HPLC (gradient B) retention time- 22.45-22.67min

¹HNMR (300 MHz, CDCl₃) δ 1.27 (s, 3H), 1.63-1.81 (m, 1H), 1.95-2.05 (m, 2H), 2.12-2.33 (m, 5H), 2.51 (t, 2H, J= 6.9 Hz), 3.06 (t, 1H, J= 10.8 Hz), 3.74 (d, 1H, J= 10.2 Hz), 3.85 (s, 3H), 3.86 (s, 3H), 4.67 (s, 3H), 4.80 (d, 1H, J= 4.8 Hz), 5.63 (t, 1H, J= 6 Hz), 6.65-6.68 (m, 2H), 6.74(s, 1H), 6.80 (d, 1H, J= 8.4 Hz), 6.85-6.92 (m, 2H), 7.29 (t, 1H, J= 7.9Hz), 7.68 (s, 2H).

¹³C NMR (75 MHz, CDCl₃) δ 20.14, 24.80, 28.02, 29.69, 31.24, 37.65, 43.11, 55.61, 55.86, 55.96, 64.97, 76.72, 111.41, 111.77, 112.99, 114.45, 120.25, 120.30, 127.00, 130.00, 133.20, 134.24, 135.52, 140.49, 141.28, 147.38, 148.84, 157.66, 169.49, 169.60, 171.46.

MS (ESI) m/z: found RT 12.25 min. (Method LCMS), 723.02 [M + 1]⁺, 725.02 [M + 3]⁺, HRMS 723.2017 [M + 1]⁺, 725.2081 [M + 3]⁺, calculated 723.1468.

### Example 24

### 5-((S)-2-(((R)-1-(3-(carboxymethoxy)phenyl)-3-(3,4-dimethoxyphenyl)propoxy) carbonyl)piperidin-1-ylsulfonyl)-2,3-dimethoxybenzoic acid (compound 24),

TLC (DCM : MeOH 9.7:0.3): Rf = 0.38, Yield- 7.23mg, % yield 45%.

HPLC (gradient A) retention time- 22.79-22.95 min

¹HNMR (400 MHz, DMSO) δ= 1.58-1.73 (m, 3H), 1.94-2.05 (m, 2H), 2.15-2.30 (m, 4H), 2.71-2.97 (m, 3H), 3.67-3.71 (m, 9H), 3.80 (s, 3H), 4.22 (t, 1H, J= 7.6 Hz), 4.58 (s, 2H), 5.35 (s, 1H), 6.68-6.72 (m, 1H), 6.75 (s, 1H), 6.81-6.89 (m, 4H), 6.95-6.99 (m, 1H), 7.06-7.11 (m, 1H), 7.16-7.20 (m, 1H).

¹³C NMR (100 MHz, DMSO) δ= 21.20, 25.10, 31.08, 31.61, 36.50, 51.04, 52.19, 55.33, 55.92, 56.03, 56.06, 61.24, 64.84, 108.62, 111.06, 112.33, 112.40, 112.62, 114.33, 120.46, 120.71, 129.88, 133.63, 135.79, 137.97, 145.07, 147.45, 147.74, 148.33, 148.59, 149.09, 151.85, 158.28, 170.61.

MS (ESI) m/z: found RT 10.48 min. (Method LCMS), 702.10 [M + 1]⁺, HRMS 702.2761 [M + 1]⁺, calculated 702.2142.

### Example 25

### 2-(3-((R)-3-(3,4-dimethoxyphenyl)-1-((S)-1-(7-nitro-2,3-dihydrobenzofuran-5-ylsulfonyl)piperidine-2-carbonyloxy)propyl)phenoxy)acetic acid (compound 25)

TLC (DCM : MeOH 9.7:0.3): Rf = 0.43, Yield- 22mg, % yield 42%.

HPLC (gradient A) retention time- 24.08-24.24 min

¹HNMR (400 MHz, CDCl₃) δ 1.24-1.32 (m, 1H), 1.47-1.50 (m, 1H), 1.67-1.77 (m, 2H), 1.82-1.91 (m, 1H), 1.92-2.01 (m, 1H), 2.09-2.19 (m, 1H), 2.28 (d, 1H, J= 12.4Hz), 2.41-2.60 (m, 2H), 2.92-3.04 (m, 1H), 3.11-3.20 (m, 2H), 3.74 (d, 1H, J= 12Hz), 3.83 (s, 3H), 3.84 (s, 3H), 4.70 (s, 2H), 4.78-4.87 (m, 3H), 4.78-4.87 (m, 3H), 5.55-5.59 (m, 1H), 6.61-6.64 (m, 2H), 6.76 (d, 1H, J= 8Hz), 6.80-6.88 (m, 3H), 7.27 (t, 1H, J= 8Hz), 7.52 (d, 1H, J= 2Hz), 8.24 (d, 1H, J= 2Hz).

¹³C NMR (100 MHz, CDCl₃) δ 20.17, 24.75, 28.16, 28.29, 31.28, 38.25, 42.17, 55.52, 55.85, 55.90, 64.78, 74.88, 76.31, 111.30, 111.62, 111.97, 114.46, 119.62, 120.12, 124.34, 128.80, 129.83, 132.14, 133.13, 133.79, 141.85, 147.37, 148.85, 157.74, 157.90, 169.71, 171.79

MS (ESI) m/z: found RT 11.54 min. (Method LCMS), 707.14 [M + Na]⁺, HRMS 685.2601 [M + 1]⁺, calculated 685.1989.

### Example 26

### 2-(3-((R)-1-((S)-1-(7-amino-2,3-dihydrobenzofuran-5-ylsulfonyl)piperidine-2-car-bonyloxy)-3-(3,4-dimethoxyphenyl)propyl)phenoxy)aceticacid (compound 26)

TLC (DCM : MeOH 9.7:0.3): Rf = 0.35, Yield- 6.7mg, % yield 14%.

HPLC (gradient A) retention time- 21.68-21.89 min

¹HNMR (600 MHz, CDCl₃) δ = 1.24-1.32 (m, 1H), 1.47-1.50 (m, 1H), 1.67-1.77 (m, 2H), 1.82-1.91 (m, 1H), 1.92-2.01 (m, 1H), 2.09-2.19 (m, 1H), 2.28 (d, 1H, J= 12.4Hz), 2.41-2.60 (m, 2H), 2.92-3.04 (m, 1H), 3.11-3.20 (m, 2H), 3.74 (d, 1H, J= 12Hz), 3.83 (s, 3H), 3.84 (s, 3H), 4.70 (s, 2H), 4.78-4.87 (m, 3H), 4.78-4.87 (m, 3H), 5.55-5.59 (m, 1H), 6.61-6.64 (m, 2H), 6.76 (d, 1H, J= 8Hz), 6.84 (s, 1H), 6.90-6.98 (m, 3H), 7.27 (t, 1H, J= 8Hz), 8.24 (d, 1H, J= 2Hz).

¹³C NMR (150 MHz, CDCl₃) δ = 20.17, 24.75, 28.16, 28.29, 31.28, 38.25, 42.17, 55.52, 55.85, 55.90, 64.78, 74.88, 76.31, 111.30, 111.62, 111.97, 114.46, 119.62, 120.12, 124.34, 128.80, 129.83, 131.69, 132.14, 133.13, 137.16, 141.85, 147.37, 148.85, 157.74, 157.90, 169.71, 171.79

MS (ESI) m/z: found RT 12.22 min. (Method LCMS), 655.08 [M + 1]⁺, 677.25 [M + Na]⁺, HRMS 655.2291 [M + 1]⁺, calculated 655.2247.

### Example 27

### 2-(3-((R)-3-(3,4-dimethoxyphenyl)-1-((S)-1-(2-methylbenzo[d]thiazol-6-ylsulf onyl)piperidine-2-carbonyloxy)propyl)phenoxy)acetic acid (compound 27),

TLC (DCM : MeOH 9.7:0.3): Rf = 0.26, Yield- 22mg, % yield 43%.

HPLC (gradient A) retention time- 24.22-24.38 min

¹HNMR (400 MHz, CDCl₃) δ= 1.24-1.32 (m, 1H), 1.50-1.61 (m, 1H), 1.65-1.73 (m, 2H), 1.85-1.95 (m, 2H), 2.03-2.13 (m, 1H), 2.18 (d, 1H, J=13.6 Hz), 2.38-2.54 (d, 2H), 2.89 (s, 3H), 3.17-3.25 (m, 1H), 3.81 (s, 3H), 3.82 (s, 3H), 4.72 (d, 1H, J= 8.4 Hz), 4.82 (d, 1H, J= 4.4 Hz), 5.44-5.47 (m, 1H), 6.58-6.61 (m, 2H), 6.68 (s, 1H), 6.74 (d, 1H, J= 8 Hz), 6.98-7.02 (m, 1H).

¹³C NMR (100 MHz, CDCl₃) δ= 19.76, 19.96, 24.74, 28.21, 31.33, 38.54, 42.44, 55.07, 55.82, 55.88, 65.29, 75.86, 111.25, 111.55, 112.34, 114.90, 119.22, 120.06, 120.93, 121.81, 125.10, 130.15, 133.14, 134.98, 135.77, 142.02, 147.34, 148.83, 154.03, 157.70, 169.43, 171.12, 172.52.

MS (ESI) m/z: found RT 11.66 min. (Method LCMS), 668.16 [M + 1]⁺, 691.11 [M + Na]⁺, HRMS 669.2459 [M + 1]⁺, calculated 669.1862.

### Example 28

### 2-(3-((R)-3-(3,4-dimethoxyphenyl)-1-((S)-1-(2-oxo-2,3-dihydrobenzo[d]thiazol-6-ylsulfonyl)piperidine-2-carbonyloxy)propyl)phenoxy)aceticacid (compound 28),

TLC (DCM : MeOH 9.7:0.3): Rf = 0.37, Yield- 24.20mg, % yield 46.8%.

HPLC (gradient A) retention time- 22.74-22.94 min

¹HNMR (600 MHz, CDCl₃) δ 1.38-1.45 (m, 1H), 1.59-1.66 (m, 1H), 1.73-1.75 (m, 2H), 1.89-1.99 (m, 2H), 2.09-2.16 (m, 2H), 2.41-2.46 (m, 1H), 2.52-2.57 (m, 1H), 3.23 (t, 1H, J= 10.2 Hz), 3.78 (d, 1H, J= 7.8Hz), 3.82 (s, 3H), 3.83 (s, 3H), 4.72-4.82 (m, 3H), 5.41-5.43 (m, 1H), 6.18 (d, 1H, J= 8.4Hz), 6.58-6.65 (m, 3H), 6.76 (d, 1H, j= 8.4 Hz), 6.92 (d, 1H, J= 6.6Hz), 7.01 (d, 1H, J= 8.4Hz), 7.09 (d, 1H, J= 8.4Hz), 7.41 (t, 1H, J= 7.8Hz), 7.66 (d, 1H, J= 1.8Hz).

¹³C NMR (150 MHz, CDCl₃) δ 19.52, 24.83, 28.47, 31.44, 38.63, 42.16, 54.77, 55.85, 55.92, 64.28, 75.54, 111.34, 111.60, 111.69, 113.56, 118.85, 120.12, 121.10, 124,32, 125.93, 130.27, 133.04, 133.23, 138.48, 142.51, 147.43, 148.89, 157.44, 169.46, 172.87, 173.00.

MS (ESI) m/z: found RT 11.08 min. (Method LCMS), 693.26 [M + Na]⁺, HRMS 671.2268 [M + 1]⁺, calculated 671.1655.

### Example 29

### 2-(3-((R)-3-(3,4-dimethoxyphenyl)-1-((S)-1-(2-oxoindolin-5-ylsulfonyl)piperidine-e-2-carbonyloxy) propyl)phenoxy)acetic acid (compound 29)

TLC (hexane: EtOAc: TFA 6:4:0.1): Rf = 0.25, yield= 6.0mg (12.5%).

HPLC (gradient A) retention time= 21.21-21.40min

¹HNMR (600 MHz, CDCl₃) δ= 1.55-1.64 (m, 1H), 1.69-1.76 (m, 2H), 1.91-2.00 (m, 2H), 2.10-2.16 (m, 2H), 2.43-2.48 (m, 1H), 2.53-2.58 (m, 1H), 3.16-3.21 (m, 1H), 3.39 (d, 1H, J= 22.8 Hz), 3.52 (d, 1H, J= 22.8 Hz), 3.73 (s, 1H), 3.82 (s, 3H), 3.83 (s, 3H), 3.87 (s, 1H), 4.71-4.81 (m, 3H), 5.43-5.45 (m, 1H), 6.25 (d, 1H, J= 8.4 Hz), 6.60-6.62 (m, 2H), 6.72-6.77 (m, 2H), 6.84-6.89 (m, 1H), 7.04-7.10 (m, 2H), 7.38 (t, 1H, J= 8.4 Hz), 7.45 (s, 1H).

¹³C NMR (150 MHz, CDCl₃) δ= 19.63, 24.79, 28.43, 31. 39, 36.16, 38.61, 42.13, 54.81, 55.83, 55.90, 63.93, 75.75, 110.47, 111.35, 111.61, 113.16, 113.50, 118.85, 120.14, 123.25, 125.28, 128.33, 130.10, 133.09, 142.60, 145.55, 147.37, 148.85, 157.49, 169.65, 171.81, 179.46.

MS (ESI) m/z: found Rt 11.03 min. (Method LCMS), 675.18 [M + Na]⁺ HRMS 653.2603 [M + H]⁺, calculated 653.2591 [M + H]⁺.

### Example 30

### 2-(3-((R)-3-(3,4-dimethoxyphenyl)-1-((S)-1-(2-oxo-2,3-dihydrobenzo[d]oxazol-6-yl-sulfonyl)piperidine-2-carbonyloxy)propyl)phenoxy)acetic acid (compound 30)

TLC (hexane: EtOAc: TFA 6:4:0.1): Rf = 0.42, yield= 6.4mg (24.6%).

HPLC (gradient A) retention time= 21.68-21.85min

¹HNMR (600 MHz, CDCl₃) δ= 1.57-1.64 (m, 1H), 1.701.75 (m, 2H), 1.91-1.99 (m, 2H), 2.10-2.16 (m, 2H), 2.43-2.48 (m, 1H), 2.53-2.59 (m, 1H), 3.19-3.24 (m, 1H), 3.36 (d, 1H, J= 22.2 Hz), 3.48 (d, 1H, J= 22.2 Hz), 3.74 (s, 2H), 3.83 (s, 3H), 3.84 (s, 3H), 4.75 (d, 2H, J= 19.0 Hz), 4.80-4.81 (m, 1H), 5.44-5.46 (m, 1H), 6.20 (d, 1H, J= 8.4 Hz), 6.59-6.63 (m, 2H), 6.72-6.78 (m, 2H), 6.89 (d, 1H, J= 8.4 Hz), 7.05 (d, 1H, J= 7.8Hz), 7.10 (d, 1H, J= 7.8 Hz), 7.39 (t, 1H, J= 8.4 Hz), 7.44 (s, 1H).

MS (ESI) m/z: found Rt 14.66 min. (Method LCMS), 677.66 [M + Na]⁺, calculated 677.54 [M + Na]⁺.

### Example 31

### (S)-((R)-3-(3,4-dimethoxyphenyl)-1-(3-(2-morpholinoethoxy)phenyl)propyl)1-(3,5-dichloro -phenylsulfonyl)piperidine-2-carboxylate (compound 31)

TLC (DCM : MeOH 9.7:0.3): Rf = 0.48, Yield- 16.42 mg, % yield 62%

HPLC (MeCN: H2O CF3COOH (0.1 %) gradient of 0 - 100% in 45 min at 1ml/min) retention time- 20.8-21.2 min

¹HNMR (600 MHz, CDCl₃) δ 1.28-1.31 (m, 1H), 1.38-1.47 (m, 1H), 1.64-1.79 (m, 1H), 1.98-2.04 (m, 1H), 2.15-2.21 (m, 1H), 2.27 (d, 1H, J= 13.8 Hz), 2.48-2.53 (m, 1H), 2.56-2.60 (m, 1H), 3.09-3.18 (m, 3H), 3.56 (t, 2H, J= 4.8 Hz), 3.66 (dd, 1H, J= 2.4, 13.2Hz), 3.76 (d, 2H, J= 12Hz), 3.84 (s, 3H), 3.85 (s, 3H), 3.95 (t, 2H, J= 12.6 Hz), 4.02 (d, 1H, J= 2.4Hz), 4.04 (d, 1H, J= 2.4 Hz), 4.39 (t, 2H, J= 4.6 Hz), 4.79 (d, 1H, J= 4.8Hz), 6.65-6.67 (m, 2H), 6.77 (d, 1H, J= 7.8 Hz), 6.80-6.82 (m, 1H), 6.85-6.86 (m, 1H), 6.91 (d, 1H, J= 7.8 Hz), 7.29 (t, 1H, J=8.1 Hz), 7.46 (t, 1H, J=1.8Hz), 7.55 (s, 1H), 7.55(s, 1H).

¹³C NMR (150 MHz, CDCl₃) δ 20.15, 24.71, 27.93, 31.28, 37.86, 43.01, 53.93, 55.64, 55.86, 55.95, 57.55, 65.55, 66.45, 77.21, 111.34, 111.73, 112.87, 114.24, 119.07, 120.13, 125.53, 129.82, 132.35, 133.33, 135.64, 141.19, 142.98, 147.42, 148.92, 158.7, 169.61 MS (ESI) m/z: found RT 8.76 min. (Method LCMS), 721.65 [M + 1]⁺, 723.37 [M + 3]⁺, HRMS 721.2797 [M + 1]⁺, 723.2766 [M + 3]⁺, calculated 721.2039.

### Example 32

### (S)-((R)-3-(3,4-dimethoxyphenyl)-1-(3-(2-morpholinoethoxy)phenyl)prop-yl)1-(benzo[d]thi -azol -6-ylsulfonyl)piperidine-2-carboxylate (compound 32)

TLC (DCM : MeOH 9.7:0.3): Rf = 0.27, Yield- 31.72mg, % yield 91 %

HPLC (MeCN: H2O CF3COOH (0.1 %) gradient of 0 - 100% in 45 min at 1ml/min) retention time- 20.78-20.98 min

¹HNMR (300 MHz, CDCl₃) δ= 1.61-1.74 (m, 3H), 1.77-1.87 (m, 3H), 1.94-2.06 (m, 1H), 2.13-2.31 (m, 1H), 2.47-2.66 (m, 2H), 3.05-3.23 (m, 3H), 3.55-3.57 (m, 2H), 3.66-3.81 (m, 3H), 3.85 (s, 6H), 4.00 (s, 4H), 4.46 (s, 2H), 2.90 (d, 1H, J= 4.5 Hz), 5.58-5.63 (m, 1H), 6.65-6.68 (m, 2H), 6.88-6.96 (m, 3H), 7.24-7.29 (m, 1H), 7.32-7.37 (m, 1H), 7.64-7.68 (m, 1H), 7.89-7.96 (m, 1H), 8.35 (s, 1H), 9.20 (s, 1H).

¹³C NMR (75 MHz, CDCl₃) δ 24.56, 27.80, 31.30, 33.33, 42.74, 46.50, 52.74, 55.42, 55.85, 55.93, 56.47, 62.47, 63.91, 76.23, 111.37, 111.73, 112.28, 114.31, 119.97, 120.16, 121.93, 123.74, 124.85, 129.02, 130.08, 133.25, 137.21, 142.29, 147.42, 148.93, 155.00, 157.39, 157.99, 169.98.

MS (ESI) m/z: found RT 8.45 min. (Method LCMS), 710.51 [M + 1]⁺, HRMS 710.2517 [M + 1]⁺, calculated 710.2492.

### Example 33

### (S)-((R)-3-(3,4-dimethoxyphenyl)-1-(3-(2-morpholinoethoxy)phenyl)propyl)1-(3,5-dichloro-4-hydroxyphenylsulfonyl)piperidine-2-carboxylate (compound 33)

TLC (DCM : MeOH 9.7:0.3): Rf = 0.45, Yield- 15.96mg, % yield 58%.

HPLC (MeCN: H2O CF3COOH (0.1 %) gradient of 0 - 100% in 45 min at 1ml/min) retention time- 19.28-19.61 min

¹HNMR (300 MHz, CDCl₃) δ 1.54-2.06 (m, 6H), 2.14-2.30 (m, 2H), 2.44-2.64 (m, 2H), 2.99-3.27 (m, 3H), 3.51 (s, 2H), 3.74-3.80 (m, 3H), 3.86 (s, 3H), 3.87 (s, 3H), 4.06 (s, 4H), 4.43 (s, 2H), 4.72 (d, 1H, J= 4.2Hz), 5.53-5.59 (m , 1H), 6.65-6.69 (m, 3H), 6.78-6.85 (m, 2H), 6.92 (d, 1H, J=7.8 Hz), 7.29-7.33 (m, 1H), 7.49 (s, 2H).

¹³C NMR (75 MHz, CDCl₃) δ 20.15, 24.94, 28.07, 31.45, 37.87, 42.86, 53.05, 55.25, 55.87, 55.95, 56.81, 62.50, 63.96, 76.25, 111.39, 111.71, 112.71, 114.02, 119.57, 120.15, 121.53, 127.30, 130.22, 132.31, 133.18, 141.75, 147.47, 148.95, 151.71, 157.01, 169.52.

MS (ESI) m/z: found RT 8.76 min. (Method LCMS), 737.04 [M + 1]⁺, 739.13 [M + 3]⁺, HRMS 737.2532 [M + 1]⁺, 739.2506 [M + 3]⁺, calculated 737.1988.

### Example 34

### (S)-((R)-3-(3,4-dimethoxyphenyl)-1-(3-(2-morpholinoethoxy)phenyl)propyl)1-(4-acetamido-3,5-dichlorophenylsulfonyl)piperidine-2-carboxylate (compound 34)

TLC (DCM: MeOH 9.7:0.3): Rf = 0.12, yield= 15.4mg (70.8%).

HPLC (gradient A) retention time= 19.64-19.81 min

¹HNMR (600 MHz, CDCl₃) δ= 1.27-1.29 (m, 1H), 1.49-1.51 (m, 1H), 1.67 (d, 1H, J=12.6 Hz), 1.73 (d, 1H, J-13.2 Hz), 1.78-1.84 (m, 1H), 1.95-2.00 (m, 1H), 2.13-2.19 (m, 1H), 2.26 (s, 4H), 2.48-2.55 (m, 2H), 3.08-3.14 (m, 3H), 3.54 (s, 2H), 3.65-3.74 (m, 3H), 3.81 (s, 3H), 3.84 (s, 3H), 3.92-3.98 (m, 2H), 4.00-4.04 (m, 2H), 4.36 (m, 2H), 4.78 (d, 1H, J= 4.8Hz), 5,58 (t, 1H, J= 7.2 Hz), 6.62-6.64 (m, 2H), 6.76-6.81 (m, 3H), 6.90 (d, 1H, J= 7.8 Hz), 7.28 (t, 1H, J= 7.8 Hz), 7.61 (s, 2H).

¹³C NMR (150 MHz, CDCl₃) δ= 19.95, 24.60, 27.88, 29.66, 31.16, 37.54, 42.98, 53.07, 55.76, 55.93, 56.80, 61.99, 63.80, 63.82, 76.65, 111.38, 111.68, 114.17, 116.07, 120.06, 120.30, 126.96, 126.97, 130.13, 133.21, 135.30, 141.68, 147.29, 148.76, 157.19, 159.79, 160.05, 160.32, 160.58, 169.76

MS (ESI) m/z: found Rt 10.30 min. (Method LCMS), 778.25, 780.31 [M + H]⁺, 800.23, 802.20 [M + Na]⁺

HRMS 778.3114, 780.3122 [M + H]⁺, calculated 778.3154, 780.3162 [M + H]⁺.

### Example 35

### (S)-((R)-3-(3,4-dimethoxyphenyl)-1-(3-(2-morpholinoethoxy)phenyl)propyl)1-(2-oxo-2,3-dihydrobenzo[d]thiazol-6-ylsulfonyl)piperidine-2-carboxylate (compound 35).

TLC (DCM : MeOH 9.7:0.3): Rf = 0.39, Yield- 6.4mg, % yield 25%.

HPLC (gradient A) retention time- 18.92-19.16min

¹HNMR (300 MHz, CDCl₃) δ 1.71-1.81 (m, 2H), 1.84-2.01 (m, 2H), 2.06-2.22 (m, 2H), 2.39-2.62 (m, 2H), 3.01-3.12 (m, 2H), 3.26-3.37 (m, 2H), 3.54 (s, 2H), 3.80-3.91 (m, 12H), 4.06 (s, 4H), 4.26-4.50 (m, 2H), 4.74 (d, 1H, J= 4.8Hz), 5.36-5.41 (m, 1H), 6.55-6.64 (m, 4H), 6.77 (d, 1H, J= 7.5Hz), 6.89 (d, 1H, J= 6.9 Hz), 6.99 (d, 1H, J= 7.5 Hz), 7.19 (d, 1H, J= 8.4 Hz), 7.38 (t, !h, J= 7.6 Hz), 7.62 (d, 1H, J= 1.8Hz).

¹³C NMR (75 MHz, CDCl₃) δ 24.98, 28.29, 31.43, 38.45, 42.48, 53.47, 54.79, 55.87, 55.94, 57.28, 62.02, 63.93, 75.83, 111.03, 111.37, 111.69, 112.73, 113.79, 119.18, 120.17, 121.54, 124.28, 125.73, 130.46, 133.10, 133.39, 138.88, 142.22, 147.47, 148.93, 156.99, 169.62.

MS (ESI) m/z: found RT 9.47 min. (Method LCMS), 726.29 [M + 1]⁺, HRMS 726.3111 [M + 1]⁺, calculated 726.2441.

### Example 36-64

Compounds 36 to 64 are characterized using MS and HPLC for purity (table 1 in example 67).

### Example 65

### (S)-((R)-3-(3,4-dimethoxyphenyl)-1-(3-(2-morpholinoethoxy)phenyl)propyl)1-(3,5-dichloro-4-methoxyphenylsulfonyl)piperidine-2-carboxylate (compound 65)

TLC (DCM : MeOH 9.7:0.3): Rf = 0.42, Yield- 21 mg, % yield 75%.

HPLC (gradient A) retention time- 23.31-23.63min

¹HNMR (600 MHz, CDCl₃) δ 1.27-1.31 (m, 1H), 1.41-1.46 (m, 1H), 1.65 (d, 1H, J= 13.8Hz), 1.71-1.80 (m, 2H), 1.99-2.05 (m, 1H), 2.17-2.23 (m, 1H), 2.28 (d, 1H, J= 14.4 Hz), 2.49-2.54 (m, 1H), 2.56-2.61 (m, 1H), 3.09 (t, 2H, J= 11.4Hz), 3.15(dt, 1H, J= 3, 12.6Hz), 3.54 (t, 2H, J= 5.2Hz), 3.66 (d, 1H, J= 10.8Hz), 3.71-3.74 (m, 2H), 3.84 (s, 6H), 3.91 (s, 3H), 3.96-4.03 (m, 4H), 4.40 (d, 2H, J =5.0Hz), 4.79 (d, 1H, J= 4.8Hz), 5.68 (q, 1H, J=3, 5.4 Hz), 6.65-6.67 (m, 2H), 6.77 (d, 1H, J= 7.8Hz), 6.81-6.83 (m, 1H), 6.85(t, 1H, J= 1.8Hz), 6.90(d, 1H, J= 7.2Hz), 7.29 (t, 1H, J= 7.8 Hz), 7.61 (s, 2H).

¹³C NMR (150 MHz, CDCl₃) δ 20.05, 24.56, 27.73, 31.26, 37.95, 42.93, 52.87, 55.57, 55.82, 55.91, 56.58, 60.94, 62.32, 63.90, 76.44, 111.32, 111.70, 112.33, 141.12, 120.03, 120.15, 127.73, 129.98, 130.07, 133.24, 137.01, 141.83, 147.38, 148.88, 155.61, 157.27, 169.80.

MS (ESI) m/z: found RT 9.08 min. (Method LCMS), 751.53 [M + 1]⁺, 753.32 [M + 3]⁺, HRMS 751.2705 [M + 1]⁺, 753.2674 [M + 3]⁺, calculated 751.2145.

### Example 66 Synthesis of reference sulfonamides:

The following reference structures were synthesized according to literature procedures (Juli et al., J.Med. Chem, 2011, 277; Choi et al., Bio. Med. Chem. Let., 2002, 1421):

Synthesis of Ethyl 1-(benzylsulfonyl)piperidine-2-carboxylate (Ref .1)

Ethyl piperidine-2-carboxylate (100mg, 0.63 mmol) was dissolved in 5mL of anhydrous DMF, and triethylamine (191mg, 1.89 mmol) was added drop wise followed by phenylmethanesulfonyl chloride (119.7mg, 0.63 mmol). The reaction was stirred for 6h at RT and subsequently the solvent was removed in vacuo. The crude mixture was purified using column chromatography (hexane : EtOAc 8:2) to yield Ethyl 1-(benzylsulfonyl)piperidine-2-carboxylate_(170mg, 0.55 mmol, 86%).

TLC (hexane: EtOAc 8:2): Rf = 0.20.

HPLC (gradient A) retention time= 23.4-23.8 min

¹HNMR (300 MHz, CDCl₃) δ= 1.14-1.25 (m, 1H), 1.28 (t, 3H, J= 7.2 Hz), 1.34-1.49 (m, 1H), 1.54-1.68 (m, 3H), 2.14 (d, 1H, J= 13.5 Hz), 3.14 (dt, 1H, J= 3.3, 12.6 Hz), 3.44 (d, 1H, J= 12.6 Hz), 4.18-4.24 (m, 2H), 4.26 (s, 2H), 4.54 (d, 1H, J= 4.8 Hz), 7.31-7.38 (m, 3H), 7.43-7.48 (m, 2H).

¹³C NMR (75 MHz, CDCl₃) δ= 14.22, 20.31, 25.04, 27.78, 43.39, 55.95, 58.71, 61.36, 128.40, 128.48, 129.35, 130.96, 171.38.

MS (ESI) m/z: found Rt 14.28 min. (Method LCMS), 312.12 [M + H]⁺, 334.22 [M + Na]⁺.

calculated 312.12 [M + H]⁺, 334.11 [M + Na]⁺.

Synthesis of Ethyl 1-(3-nitrophenylsulfonyl)piperidine-2-carboxylate (Ref .2)

Ethyl piperidine-2-carboxylate (100mg, 0.63 mmol) was dissolved in 5mL of anhydrous DMF, and triethylamine (191mg, 1.89 mmol) was added drop wise followed by 3-nitrobenzene-1-sulfonyl chloride (139.2mg, 0.63 mmol). The reaction was stirred for 6h at RT and subsequently the solvent was removed in vacuo. The crude mixture was purified using column chromatography (hexane: EtOAc 8:2) to yield Ethyl 1-(3-nitrophenylsulfonyl)piperidine-2-carboxylate (195mg, 0.57 mmol, 89%).

TLC (hexane: EtOAc 7:3): Rf = 0.45.

HPLC (gradient A) retention time= 23.6-24.1 min

¹HNMR (300 MHz, CDCl₃) δ= 1.12 (t, 3H, J= 7.2 Hz), 1.45-1.84 (m, 5H), 2.18 (d, 1H, J=13.8 Hz), 3.15 (dt, 1H, J=3 , 12.6 Hz), 3.79 (d, 1H, J= 12.6 Hz), 4.39-4.04 (m, 2H), 4.76 (d, 1H, J= 5.4 Hz), 7.68 (t, 1H, J= 7.8 Hz), 8.06-8.10 (m, 1H), 8.35-8.39 (m, 1H), 8.57 (t, 1H, J=1.8 Hz).

¹³C NMR (75 MHz, CDCl₃) δ= 14.02, 19.95, 24.80, 27.94, 42.89, 55.44, 61.33, 122.39, 126.77, 130.05, 132.74, 142.03, 148.06, 170.17.

MS (ESI) m/z: found Rt 14.27 min. (Method LCMS), 343.06 [M + H]⁺, calculated 343.09 [M + H]⁺.

Synthesis of 1-(benzylsulfonyl)piperidine-2-carboxylic acid

To Ethyl 1-(benzylsulfonyl)piperidine-2-carboxylate (150mg, 0.48 mmol) was added 1 M LiOH in MeOH: H₂O (1:1) and the reaction stirred for 6h at room temperature. The reaction was acidified to pH=2 by addition of 1 M HCl. The aqueous layer was extracted with dichloromethane (3x 20ml). The combined organic phases were washed with brine (30ml) and dried over MgSO₄. The solution was concentrated under reduced pressure to furnish the free acid 1-(Benzylsulfonyl)piperidine-2-carboxylic acid as a white solid (128mg, 0.45 mmol, 94%).

TLC (hexane: EtOAc: TFA 6:4:0.1): Rf = 0.20.

HPLC (gradient A) retention time= 18.1-18.6 min

¹HNMR (300 MHz, CDCl₃) δ= 1.25-1.45 (m, 2H), 1.57-1.72 (m, 3H), 2.16 (d, 1H, J=14.1Hz), 3.09-3.19 (dt, 1H, J= 2.7, 12.6 Hz), 3.47 (d, 1H, J= 13.2 Hz), 4.27 (s, 2H), 4.55 (d, 1H, J= 4.5 Hz), 7.34-7.37 (m, 3H), 7.40-7.45 (m, 2H).

¹³C NMR (75 MHz, CDCl₃) δ= 20.27, 24.85, 27.51, 43.47, 55.71, 58.91, 128.57, 128.61, 129.09, 130.91, 177.43.

MS (ESI) m/z: found Rt 12.32 min. (Method LCMS), 306.26 [M + Na]⁺, calculated 306.08 [M + Na]⁺.

Synthesis of 1-(3-nitrophenylsulfonyl)piperidine-2-carboxylic acid

To Ethyl 1-(3-nitrophenylsulfonyl)piperidine-2-carboxylate (175mg, 0.51 mmol) was added 1M LiOH in MeOH: H₂O (1:1) and the reaction stirred for 6h at room temperature. The reaction was acidified to pH=2 by addition of 1 M HCl. The aqueous layer was extracted with dichloromethane (3x 20ml). The combined organic phases were washed with brine (30ml) and dried over MgSO₄. The solution was concentrated under reduced pressure to furnish the free acid 1-(3-nitrophenylsulfonyl)piperidine-2-carboxylic acid as a white solid (152mg, 0.48 mmol, 95%).

TLC (hexane: EtOAc: TFA 6:4:0.1): Rf = 0.27.

HPLC (gradient A) retention time= 18.7-19.1 min

¹HNMR (300 MHz, CDCl₃) δ= 1.20-1.36 (m, 1H), 1.45-1.61 (m, 1H), 1.69-1.87 (m, 3H), 2.22 (m, 1H, J= 14.4 Hz), 3.09 (dt, 1H, J= 3, 12.9 Hz), 3.78 (d, 1H, J= 4.8 Hz), 7.70 (t, 1H, J= 8.1 Hz), 8.09-8.13 (m, 1H), 8.36-8.40 (m, 1H), 8.59 ( t, 1H, J= 2.1 Hz). ¹³C NMR (75 MHz, CDCl₃) δ= 20.03, 24.62, 27.72, 42.90, 55.23, 122.38, 126.88, 130.16, 132.75, 141.89, 148.07, 176.47.

MS (ESI) m/z: found Rt 14.10 min. (Method LCMS), 337.10 [M + Na]⁺, calculated 337.05 [M + Na]⁺.

Synthesis of 3-(3,4-dimethoxyphenyl)propyl 1-(benzylsulfonyl)piperidine-2-carboxylate (Ref. 3)

A solution of alcohol 3-(3,4-dimethoxyphenyl)propan-1-ol (37.8mg, 0.193 mmol), acid 1-(Benzylsulfonyl)piperidine-2-carboxylic acid (50mg, 0.176 mmol) and DMAP (6.4 mg, 0.05 mmol) in DCM at room temperature was treated with DCC (54.47 mg, 0.264 mmol). After stirring for 12 h the mixture was diluted with EtOAc and filtered through a plug of celite. The filtrate was concentrated and the crude material flash chromatographed (hexane: EtOAc 7:3) to afford compound 3-(3,4-dimethoxyphenyl)propyl 1-(benzylsulfonyl)piperidine-2-carboxylate (65 mg, 0.14 mmol, 80%).

TLC (hexane: EtOAc: 7:3): Rf = 0.40.

HPLC (gradient A) retention time= 26.3-26.6 min

¹HNMR (300 MHz, CDC₃) δ= 1.35-1.71 (m, 5H), 1.92-2.01 (m, 2H), 2.14 (d, 1H, J= 12 Hz), 2.65 (t, 2H, J= 8.1 Hz), 3.16 (dt, 1H, J= 3, 12.6 Hz), 3.44 (d, 1H, J= 17.7 Hz), 3.85 (s, 3H), 3.86 (s, 3H), 4.13-4.22 (m, 2H), 4.26 (s, 2H), 4.54 (d, 1H, J= 4.5 Hz), 6.69-6.73 (m, 2H), 6.78-6.80 (m, 1H), 7.34-7.37 (m, 3H), 7.43-7.47 (m, 2H).

¹³C NMR (75 MHz, CDCl₃) δ= 20.36, 24.99, 27.79, 30.41, 31.63, 43.44, 55.84, 55.93, 56.04, 58.80, 64.63, 111.34, 111.78, 120.22, 128.46, 128.51, 129.31, 130.94, 133.48, 147.38, 148.94, 171.46.

MS (ESI) m/z: found Rt 14.98 min. (Method LCMS), 484.38 [M + Na]⁺, calculated 484.18 [M + Na]⁺.

Synthesis of 3-(pyridin-3-yl)propyl 1-(3-nitrophenylsulfonyl)piperidine-2-carboxylate

### (Ref. 4)

A solution of alcohol 3-(pyridin-3-yl)propan-1-ol (24mg, 0.175 mmol), acid 1-(3-nitrophenylsulfonyl)piperidine-2-carboxylic acid (50mg, 0.159 mmol) and DMAP (5.8 mg, 0.047 mmol) in DCM at room temperature was treated with DCC (49.2 mg, 0.238 mmol). After stirring for 12 h the mixture was diluted with EtOAc and filtered through a plug of celite. The filtrate was concentrated and the crude material flash chromatographed (DCM: MeOH 9.7: 0.3) to afford compound 3-(pyridin-3-yl)propyl 1-(3-nitrophenylsulfonyl)piperidine-2-carboxylate (60 mg, 0.138 mmol, 87%).

TLC (DCM : MeOH 9.7:0.3): Rf = 0.50.

HPLC (gradient A) retention time= 17.0-17.4 min

¹HNMR (300 MHz, CDCl₃) δ= 1.55-1.86 (m, 7H), 2.19 (d, 1H, J=13.5 Hz), 2.67 (t, 2H, J= 8.1 Hz), 3.17 (dt, 1H, J= 3, 12.6 Hz), 3.78 (d, 1H, J=7.9 Hz), 4.02 (dt, 2H, J= 1.2, 6.6 Hz), 4.80 (d, 1H, J= 5.1 Hz), 7.26-7.31 (m, 1H), 7.55 (d, 1H, J= 7.8 Hz), 7.68 (t, 1H, J= 8.1 Hz), 8.10 (d, 1H, J= 7.8 Hz), 8.36-8.40 (m, 1H), 8.45-8.48 (m, 2H), 8.59 (s, 1H).

¹³C NMR (75 MHz, CDCl₃) δ=20.04, 24.91, 27.93, 29.19, 29.71, 33.92, 42.95, 55.49, 64.28, 122.39, 123.76, 126.85, 130.10, 132.69, 136.52, 136.67, 142.07, 146.86, 148.11, 148.88, 170.25.

MS (ESI) m/z: found Rt 10.62 min. (Method LCMS), 434.26 [M + H]⁺, calculated 434.13 [M + H]⁺.

### Example 66 Medium throughput small scale synthesis:

Pre-weighed samples of Fmoc protected immobilized pipecolate solid support were distributed to each of 36 wells of a 96 well parallel synthesis reactor platform obtained from FlexChem® peptide synthesis system. The Fmoc deprotection was carried out individually in each of the wells followed by coupling with sulfonyl chlorides obtained commercially from Maybridge. The unreacted excess sulfonyl chlorides were washed followed by the cleavage of the pipecolate sulfonamides from the resin under mild acidic condition.

Out of the 36 compounds 35 compounds were purified by preparative. Analytical HPLC showed the compounds had at least > 90 % purity with most compounds > 95% purity. The correct identity was confirmed by mass spectroscopy, as summarized in Table 1.

### Library Testing:

The purified and chemically validated compounds were tested for their binding to the FK1 domains of FKBP51 and FKBP52 in a fluorescence polarization assay (Kozany, et al., ChemBioChem 10, 8, 2009, 1402-1410). The binding of the compounds to the proteins was analyzed by calculating the % inhibition at a concentration of 5 µM (Table 1).

**Table 1: Chemical compounds according to formula (I) used in Library Testing:**

| Following compounds: **R1-3= -H, R5-7= -H, R8-9= -OCH₃,R10 = -H, R4= -OCH₂-COOH** | | | | | | |
|---|---|---|---|---|---|---|
| **Cpd No.** | **A** | **Mol Wt calc.** | **Mol Wt Found** | **% purity HPLC** | **FKBP 51 FK1 % inhibition** | **FKBP52 FK1 % inhibition** |
| **36** | 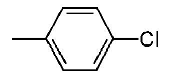 | 631 16 | 654 13 [M + Na]⁺ | 98 | 11.7 | 15.2 |
| **37** | 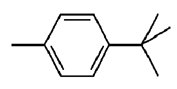 | 645 19 | 663 34 [M + NH₄]⁺ | 96 | 4.3 | 0 |
| **38** | 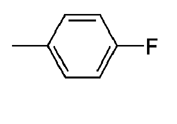 | 61519 | 633 00 [M + NH₄]⁺, 638 33 [M + Na]⁺. | 96 | 4.3 | 6.6 |
| **39** | 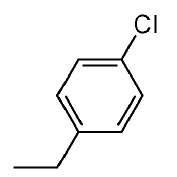 | 653 27 | 652 27 [M -1] (a) | 98 | 8.3 | 14.2 |
| **40** | 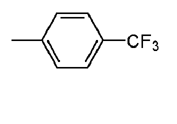 | 665 19 | 688 13 [M + Na]⁺, 702 13 [M + K]⁺ | 99 | 8.9 | 12.3 |
| **41** | 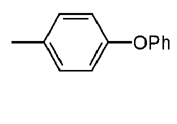 | 689 23 | 712 33 [M + Na]⁺, 726 27 [M + K]⁺ | 97 | 7.2 | 15.2 |
| **1** | 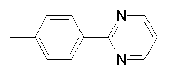 | 675 23 | 676 13 [M + 1 ]⁺, 698 27 [M + Na]⁺ | 99 | 16.3 | 15.2 |
| **42** | 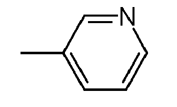 | 598 20 | 599 33 [M + 1]⁺, 621 20 [M + Na]⁺ | 97 | 6.6 | 9.5 |
| **43** | 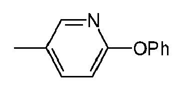 | 690 22 | 689 27 [M -1 ] (a) | 99 | 3.7 | 11.4 |
| **44** | 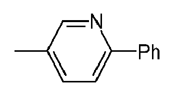 | 674 23 | 675 33 [M + 1 ]⁺ | 99 | 2.6 | 8.5 |
| **45** | 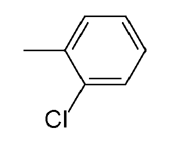 | 631 16 | 654 20 [M + Na]⁺, 668 27 [M + K]⁺ | 99 | 1.4 | 9.5 |
| **46** | 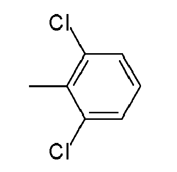 | 665 13 | 688 27 [M + Na]⁺, 706 20 [M + K]⁺ | 99 | 3.2 | 9.5 |
| **2** | 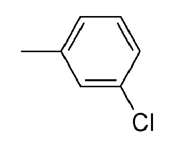 | 631 16 | 649 27 [M + NH₄]⁺, 654 27 [M + Na]⁺, 670 27 [M + K]⁺ | 99 | 17.5 | 18.0 |
| **47** | 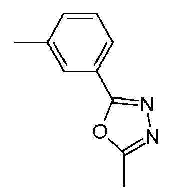 | 679 22 | 679 93 [M + 1]⁺, 702 33 [M + Na]⁺, 716 33 [M + K]⁺ | 95 | 4.9 | 3.8 |
| **48** | 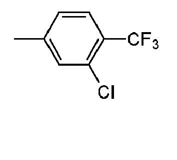 | 699 15 | 716 93 [M + NH₄]⁺, 722 20 [M + Na]⁺, 736 27 [M + K]⁺. | 91 | -0.2 | 11.4 |
| **49** | 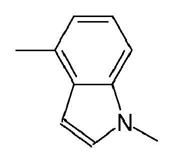 | 650 23 | 673 20 [M + Na]⁺, 689 27 [M + K]⁺ | 95 | -0.8 | 6.6 |
| **3** | 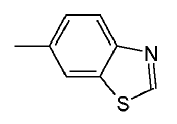 | 654 17 | 655 07 [M + 1]⁺, 677 20 [M + Na]⁺ | 93 | 25.5 | 16.1 |
| **50** | 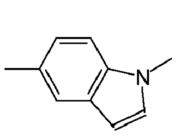 | 650 23 | 668 20 [M + NH₄]⁺, 673 13 [M + Na]⁺, 689 20 [M + K]⁺ | 86 (b) | 10.0 | 10.4 |
| **51** | 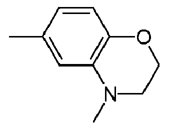 | 668 24 | 669 40 [M + 1 ]⁺ | 93 | 9.5 | 6.6 |
| **52** | 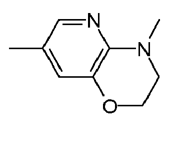 | 669 24 | 670 27 [M + 1]⁺, 692 20 [M + Na]⁺, 708 33 [M + K]⁺ | 99 | 3.2 | 1.9 |
| **4** | 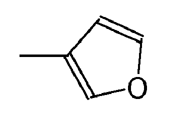 | 587 18 | 610 24 [M + Na]⁺, 626 18 [M + K]⁺ | 97 | 15.2 | 16.2 |
| **53** | 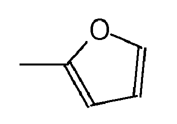 | 587 18 | 610 27 [M + Na]⁺, 624 20 [M + K]⁺ | 97 | 2.0 | 5.7 |
| **54** | 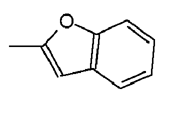 | 637 20 | 660 13 [M + Na]⁺, 674 33 [M + K]⁺ | 99 | 5.5 | 6.6 |
| **55** | 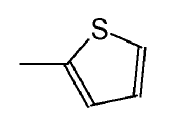 | 603 16 | 626 27 [M + Na]⁺, 642 13 [M + K]⁺ | 95 | 3.2 | 8.5 |
| **56** | 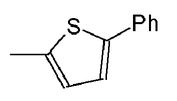 | 679 19 | 702 27 [M + Na]⁺ | 99 | 3.2 | 9.5 |
| **5** | 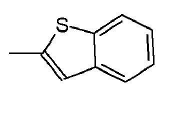 | 653 18 | 676 27 [M + Na]⁺, 691 93 [M + K]⁺ | 98 | 12.4 | 15.2 |
| **57** | 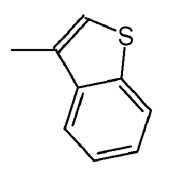 | 653 18 | 676 07 [M + Na]⁺ | 99 | 3.2 | 4.7 |
| **58** | 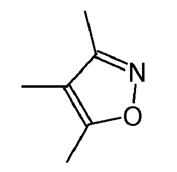 | 616 21 | 639 20 [M + Na]⁺, 655 27 [M + K]⁺ | 89 | -3.0 | 3.8 |
| **59** | 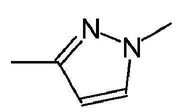 | 601 21 | 602 27 [M + 1]⁻, 624 20 [M + Na]⁺, 640 13 [M + K]⁺ | 97 | 2.1 | 4.7 |
| **60** | 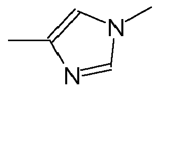 | 601.21 | 602.20 [M + 1]⁺, 617.07 [M + NH₄]⁺, 624.40 [M + Na]⁺, 638.40 [M + K]⁺. | 96 | -2.5 | 3.8 |
| **61** | 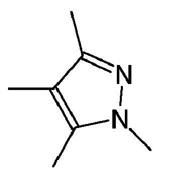 | 629.24 | 630.27 [M + 1]⁺, 652.27 [M + Na]⁺, 668.20 [M + K]⁺. | 97 | 5.5 | 1.9 |
| **62** | 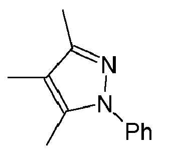 | 691.26 | 692.33 [M + 1]⁺, 714.20 [M + Na]⁺, 728.47 [M +K]⁺. | 94 | 0.9 | 4.7 |
| **63** | 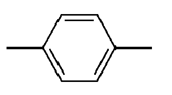 | 611.22 | 634.27 [M + Na]⁺. | 95 | 3.2 | 3.8 |
| **64** | 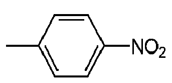 | 642.19 | 665.07 [M + Na]⁺. | 95 | 0.3 | 0 |

### Example 66

### Generation of biological data: Fluorescence polarization assay

IC50 values for human FKBP51 FK1 and human FKBP52FK2 were determined as described (Kozany, et al., ChemBioChem 10, 8, 2009, 1402-1410).

**Table 2: Chemical compounds according to formula (I) and their binding affinity (IC₅₀-value) determined in a fluorescence polarization assay.**

| | | | | |
|---|---|---|---|---|
| 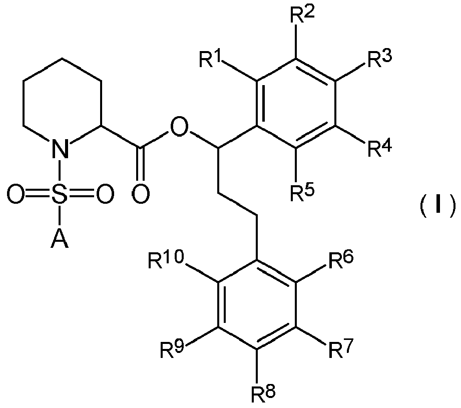 | | | | |

| Following compounds: **R1-3= -H, R5-7= -H, R8-9= -OCH₃, R10 = -H, R4= -OCH₂-COOH** | | | | |
|---|---|---|---|---|
| **Compd. No.** | **A** | **Purity (%)** | **FKBP51 FK1 IC₅₀(µM)** | **FKBP52FK1 IC₅₀(µM)** |
| **Ref. 1** (①) | | >99 | >100 | >100 |
| **Ref. 2** (①) | | >99 | >100 | >100 |
| **Ref. 3** (①) | | >99 | >100 | >100 |
| **Ref. 4** (①) | | >98 | >100 | >100 |
| **1** | 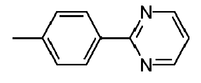 | >99 | 62.8 ± 10.7 | > 100 |
| **2** | 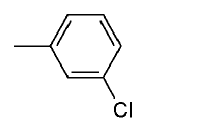 | >98 | 30.7 ± 15.7 | 32.8 ±14.5 |
| **3** | 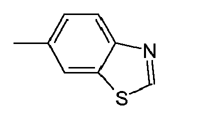 | >99 | 11.6 ± 1.1 | 32.5 ±3.5 |
| **5** | 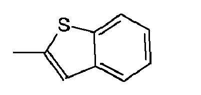 | >99 | 67.1 ± 12.1 | > 100 |
| **6** | 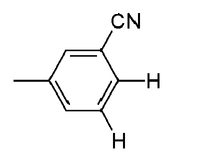 | >98 | 28.5 ± 9.6 | 69.9 ± 65.4 |
| **7** | 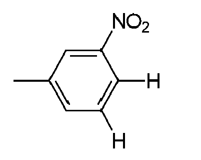 | >98 | 47.2 ± 7.1 | > 100 |
| **8** | 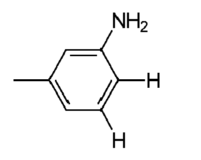 | >99 | 45.4 ± 13.1 | > 100 |
| **11** | 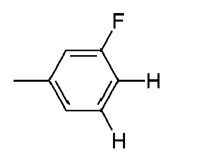 | >99 | 18.25 ± 8.8 | 37.3 ± 13.6 |
| **12** | 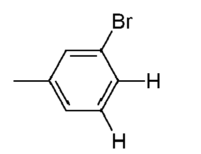 | >99 | 3.06 ± 0.25 | 17.09 ± 11.0 |
| **13** | 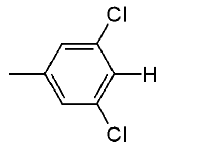 | >99 | 22.6 ± 8.2 | 14.3 ± 1.8 |
| **16** | 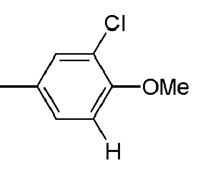 | >99 | 18.8 ± 2.4 | 20.2 ± 3.5 |
| **17** | 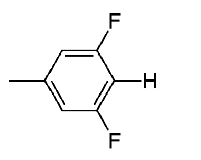 | >98 | 17.2 ± 1.7 | 84.7 ± 26.3 |
| **21** | 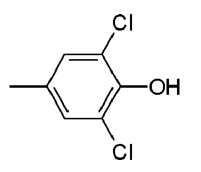 | >99 | 15.1 ± 1.4 | 56.6 ± 12.2 |
| **22** | 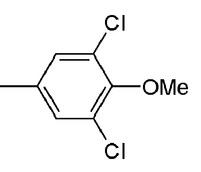 | >99 | 16.4 ± 1.7 | 17.7 ± 1.6 |
| **23** | 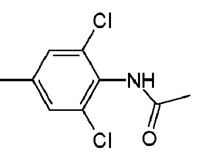 | >98 | 8.9 ± 0.9 | 15.6 ± 2.2 |
| **25** | 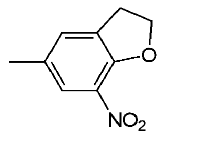 | >98 | 27.2 ± 3.1 | 43.9 ± 10.1 |
| **26** | 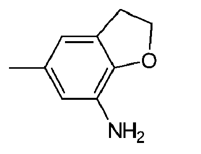 | >98 | 43.6 ± 5.5 | > 100 |
| **27** | 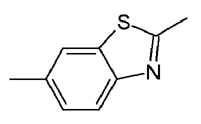 | >98 | 18.4 ± 1.4 | 26.5 ± 4.9 |
| **28** | 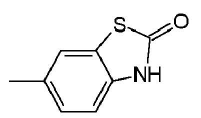 | >99 | 14.7 ± 1.1 | 66.5 ± 27.1 |

| Following compounds: **R1-3= -H, R5-7= -H, R8-9= -OCH₃,R10 = -H, R4**= ② | | | | |
|---|---|---|---|---|
| **33** | 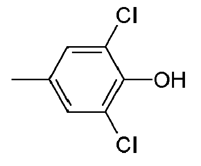 | >99 | 0.456 ± 0.05 | 0.71 ± 0.10 |
| **34** | 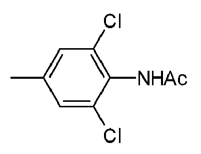 | >99 | 12.3 ± 18.9 | 8.3 ± 6.8 |
| **35** | 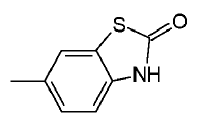 | >99 | 1.3±0.13 | 2.5 ± 0.38 |

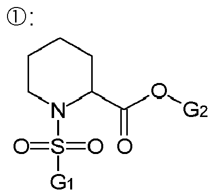

reference compounds from :
Juli et al., J.Med. Chem, 2011, 277;
Choi et al., Bio. Med. Chem. Let., 2002, 1421

| Compd. No. | G1 | G2 |
|---|---|---|
| **Ref. 1** | 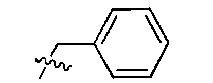 | Et |
| **Ref. 2** | 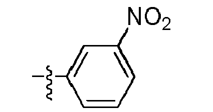 | Et |
| **Ref. 3** | 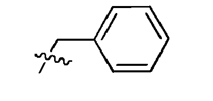 | 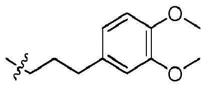 |
| **Ref. 4** | 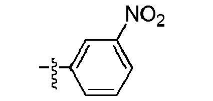 | 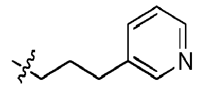 |

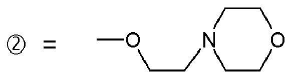

## Claims

1. Compound of the general formula (I): 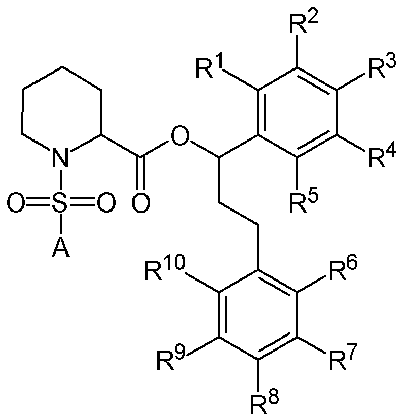 wherein
**R¹-R¹⁷** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NiO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃O₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-N H-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, _{-C2H5}, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH2₋C(CH3)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 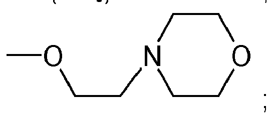 R^{N} represents the following -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COCN, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂C₃H₇, -SO₂-cyclo-C₃H₅, -SO₂CH(CH₃)₂, -SO₂C(CH₃)₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂Hs)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
and A represents the following 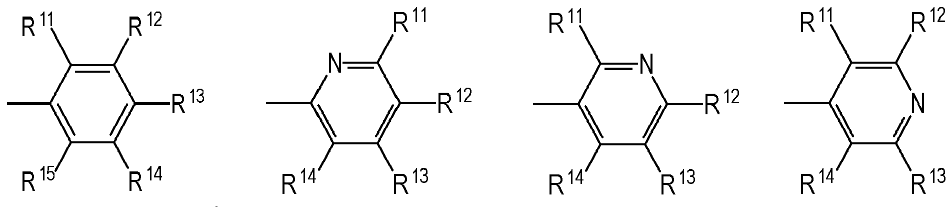 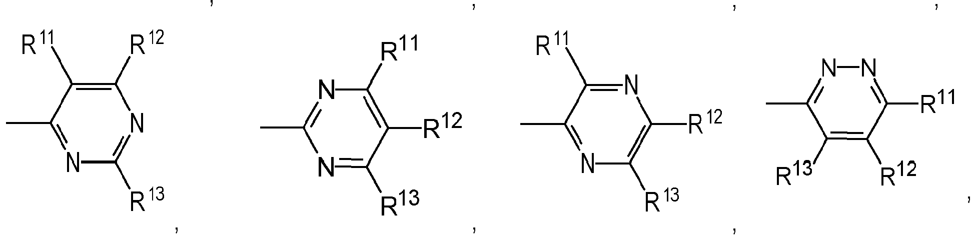 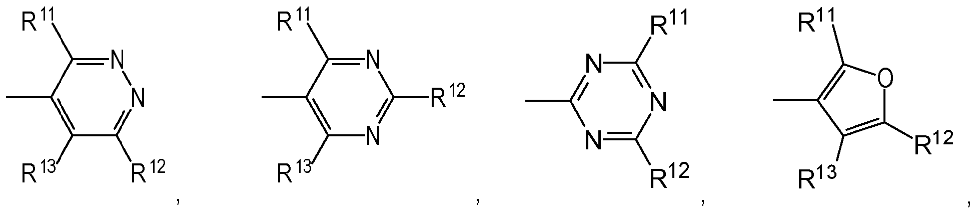 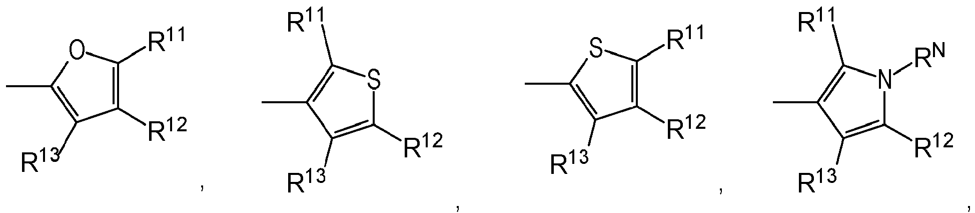 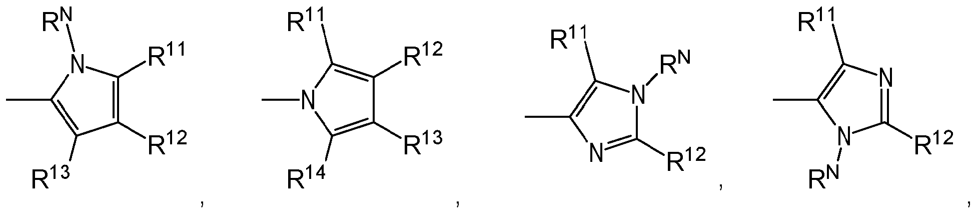 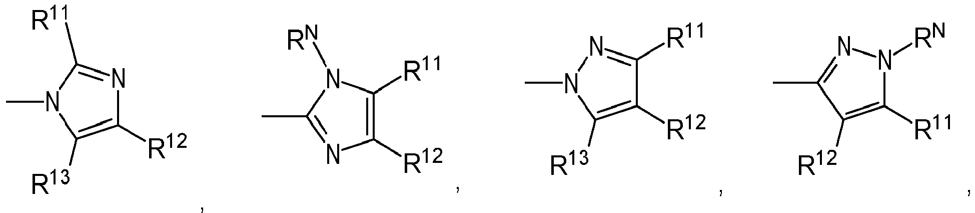 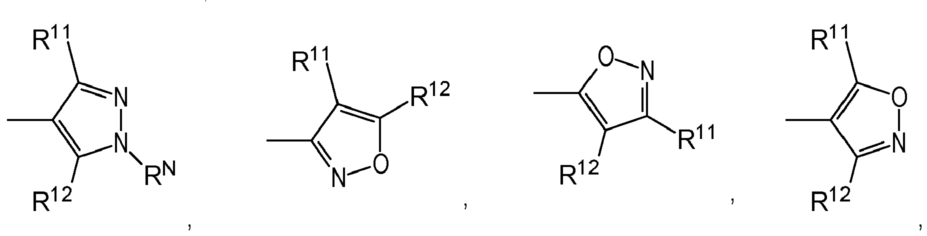 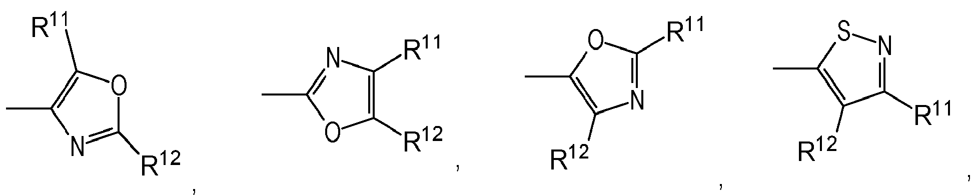 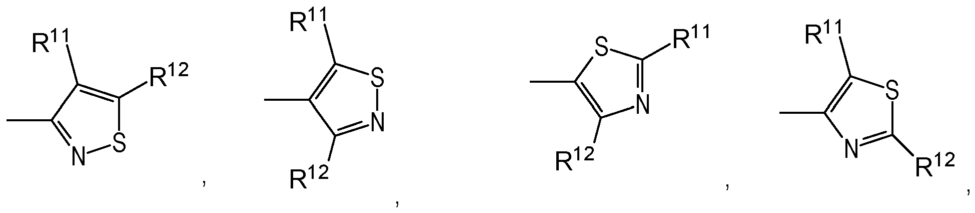 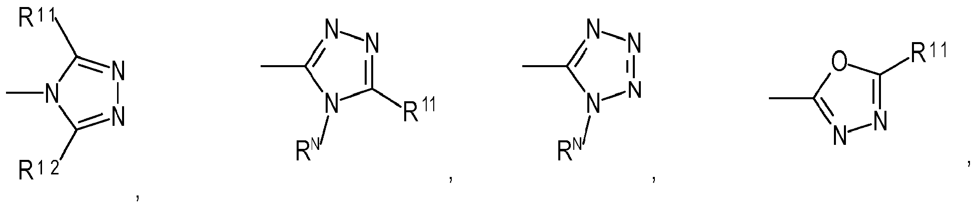 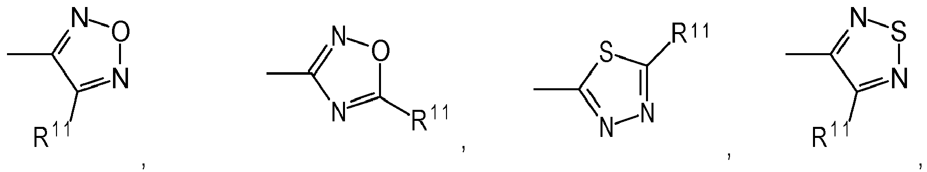 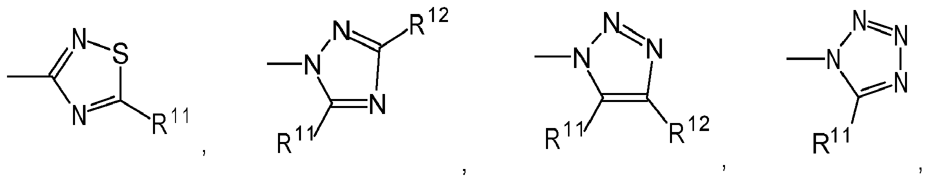 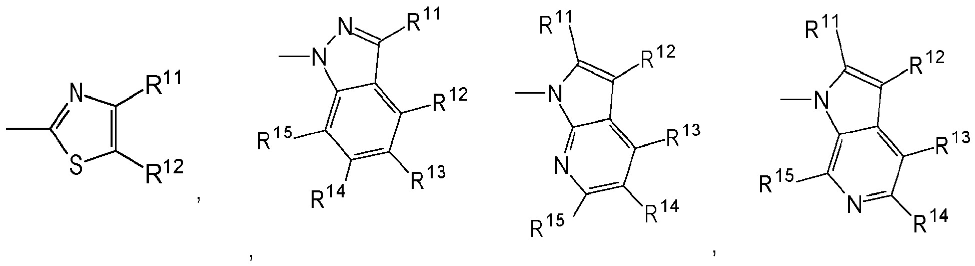 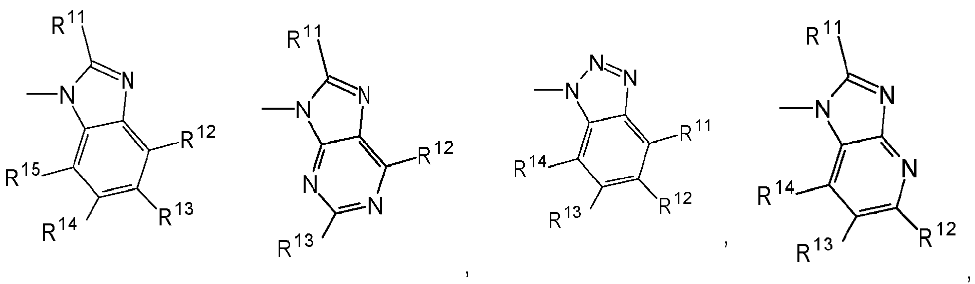 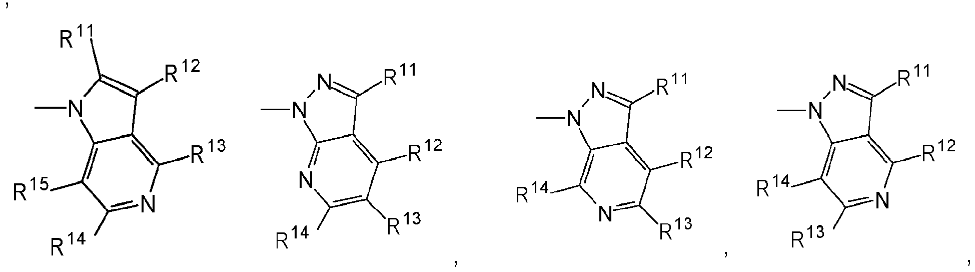 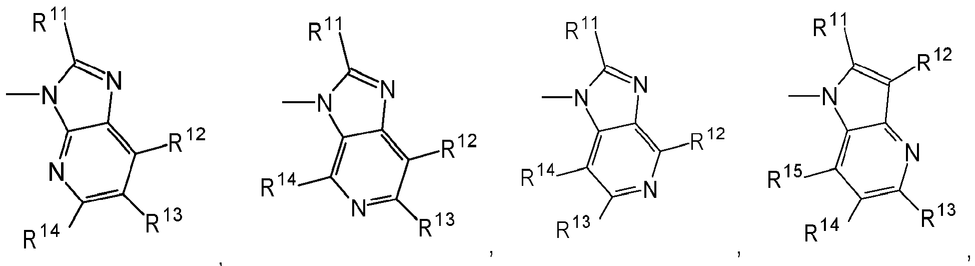 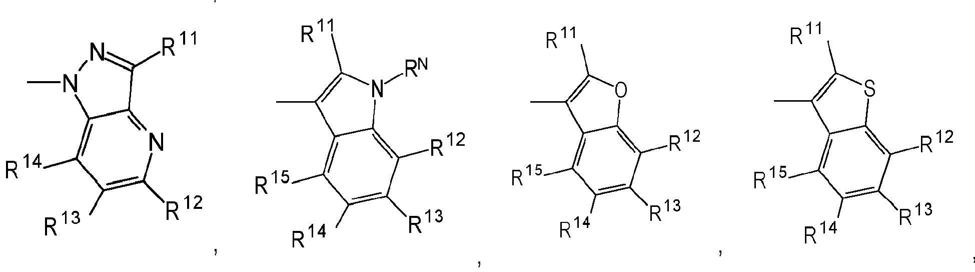 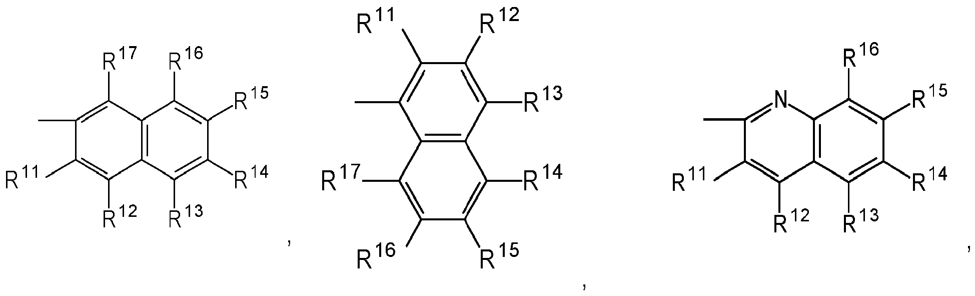 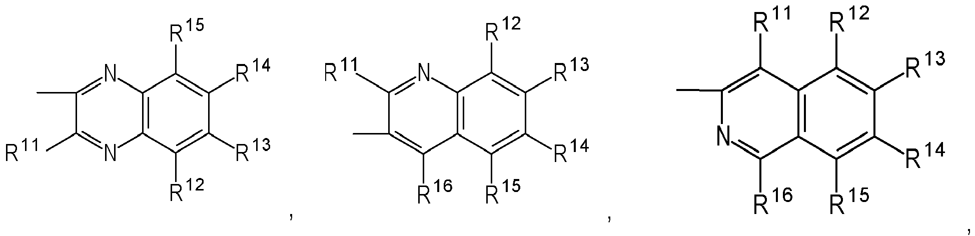 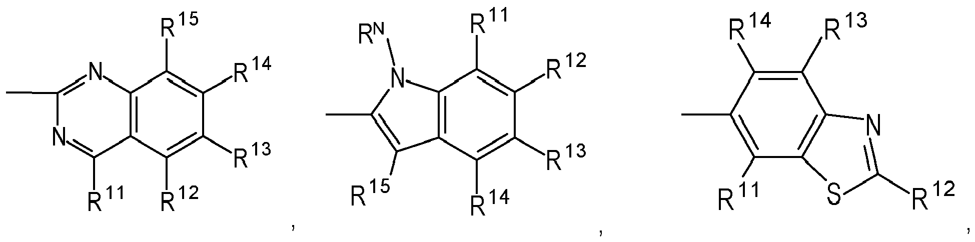 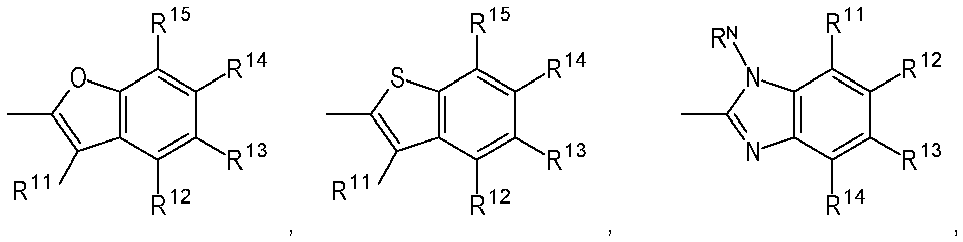 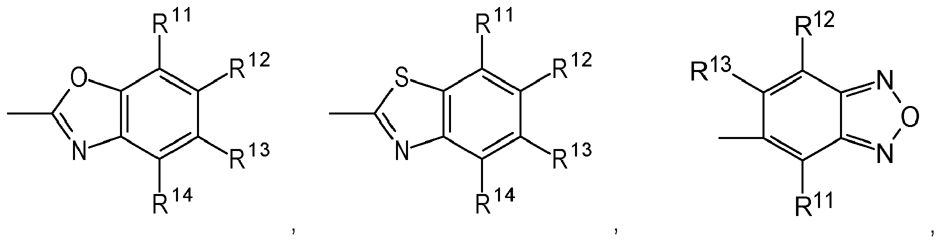 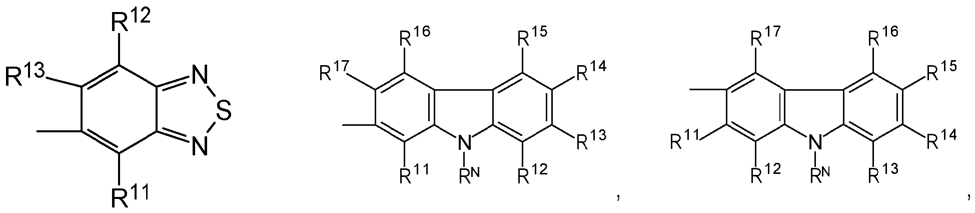 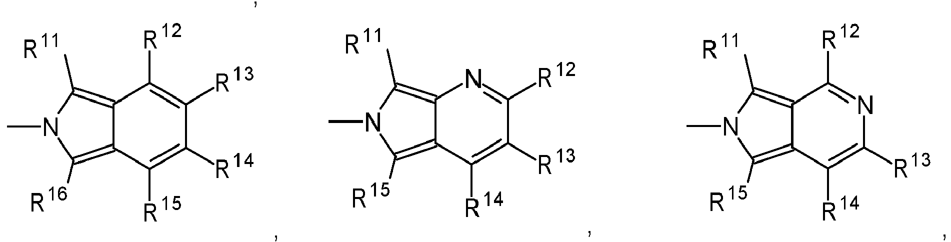 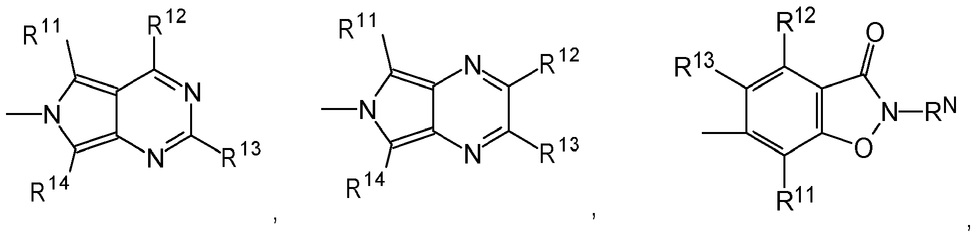 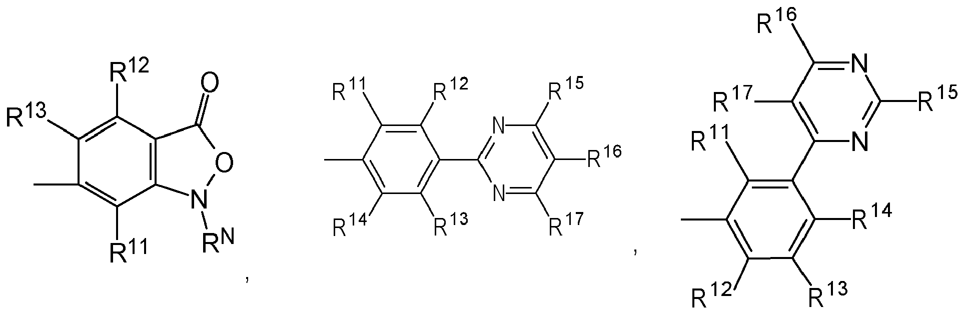 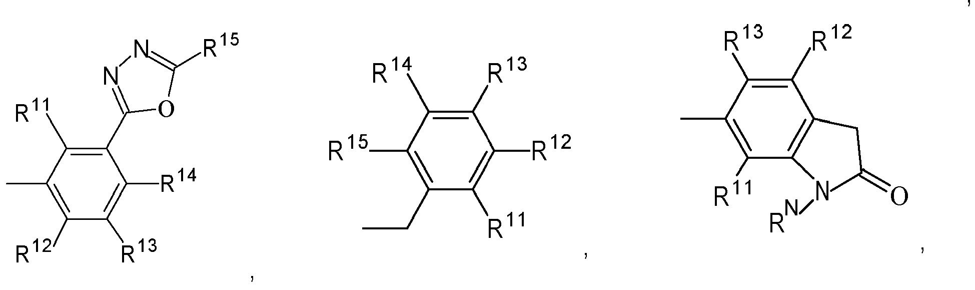 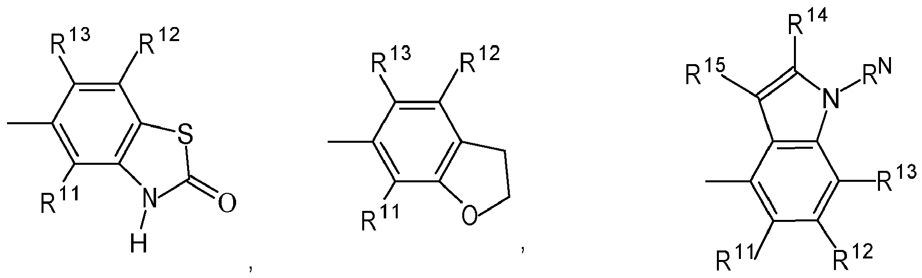 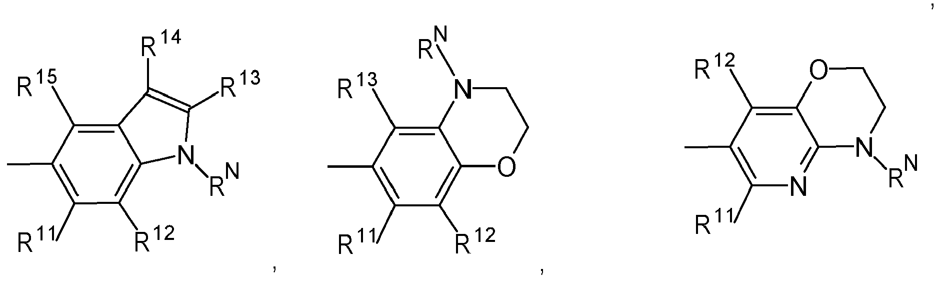 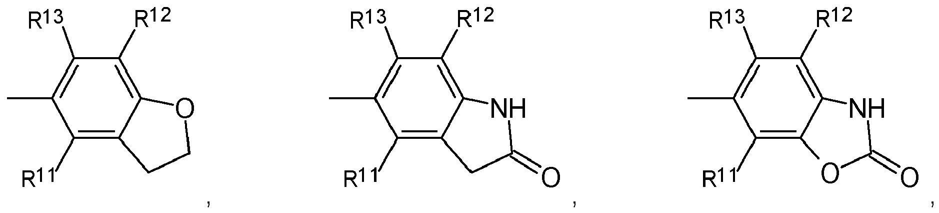 and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

2. Compound according to claim 1 for use as pharmaceutically active agent in medicine.

3. Compound according to claim 1 for use as inhibitor of FK506-binding proteins (FKBP).

4. Compound according to claim 1 for use as inhibitor of the FK506-binding protein 51 (FKBP51) and/or 52 (FKBP52).

5. Compound according to claim 1 selected from the group consisting of:
2-(3-((R)-3-(3,4-dimethoxyphenyl)-1-((S)-1-(4-(pyrimidin-2-yl)phenylsulfonyl)pipridine -2-carbonyloxy) propyl)phenoxy)acetic acid,
2-(3-((R)-1-((S)-1-(3-chlorophenylsulfonyl)piperidine-2-carbonyloxy)-3-(3,4-dimethoxy phenyl) propyl- phenoxy)acetic acid,
2-(3-((R)-1-((S)-1-(benzo[d]thiazol-5-ylsulfonyl)piperidine-2-carbonyloxy)-3-(3,4-dimethoxy phenyl)propyl) phenoxy)acetic acid,
2-(3-((R)-3-(3,4-dimethoxyphenyl)-1-((S)-1-(furan-3-ylsulfonyl)piperidine-2-carbonyloxy) propyl)phenoxy)acetic acid,
2-(3-((R)-1-((S)-1-(benzo[b]thiophen-2-ylsulfonyl)piperidine-2-carbonyloxy)-3-(3,4-dimeth xy phenyl propyl)phenoxy)acetic acid,
2-(3-((R)-1-((S)-1-(3-cyanophenylsulfonyl)piperidine-2-carbonyloxy)-3-(3,4-dimethoxyphenyl)propyl)phenoxy)acetic acid,
2-(3-((R)-3-(3,4-dimethoxyphenyl)-1-((S)-1-(3-nitrophenylsulfonyl)piperidine-2-carbonylo -xy propyl)phenoxy)acetic acid,
2-(3-((R)-3-(3,4-dimethoxyphenyl)-1-((S)-1-(3-(2-methylpyrim idin-4-yl)phenylsulfonyl) piperidine-2-carbonyloxy)propyl)phenoxy)acetic acid,
2-(3-((R)-3-(3,4-dimethoxyphenyl)-1-((S)-1-(3-(pyrim idin-4-yl)phenyl sulfonyl)piperidine-2-carbonyloxy)propyl)phenoxy)acetic acid,
2-(3-((R)-1-((S)-1-(3,5-dichlorophenylsulfonyl)piperidine-2-carbonyloxy)-3-(3,4-dimethoxy -phenyl)propyl)phenoxy)acetic acid,
2-(3-((R)-1-((S)-1-(2,3-dichlorophenylsulfonyl)piperidine-2-carbonyloxy)-3-(3,4-dimethoxy phenyl)propyl)phenoxy)acetic acid,
2-(3-((R)-3-(3,4-dimethoxyphenyl)-1-((S)-1-(3,4-dimethoxyphenylsulfonyl) piperidine-2-carbonyloxy)propyl)phenoxy)acetic acid,
2-(3-((R)-1-((S)-1-(3,5-bis(trifluoromethyl)phenylsulfonyl)piperidine-2-carbonyloxy)-3-(3,4-dimethoxyphenyl)propyl)phenoxy)acetic acid,
2-(3-((R)-1-((S)-1-(3-bromo-5-(trifluoromethyl)phenylsulfonyl)piperidine-2-carbonyloxy)-3-(3,4-dimethoxyphenyl)propyl)phenoxy)acetic acid,
2-(3-((R)-1-((S)-1-(3,5-dichloro-4-hydroxyphenylsulfonyl)piperidine-2-carbonyloxy)-3-(3,4-dimethoxyphenyl)propyl)phenoxy)acetic acid,
2-(3-((R)-1-((S)-1-(3,5-dichloro-4-methoxyphenylsulfonyl)piperidine-2-carbonyloxy)-3-(3,4-dimethoxyphenyl)propyl)phenoxy)acetic acid,
(S)-((R)-3-(3,4-dimethoxyphenyl)-1-(3-(2-morpholinoethoxy)phenyl)propyl)1-(3,5-dichloro -phenylsulfonyl)piperidine-2-carboxylate,
(S)-((R)-3-(3,4-dimethoxyphenyl)-1-(3-(2-morpholinoethoxy)phenyl)propyl)1-(benzo[d]thi -azol -6-ylsulfonyl)piperidine-2-carboxylate,
(S)-((R)-3-(3,4-dimethoxyphenyl)-1-(3-(2-morpholinoethoxy)phenyl)propyl)1-(3,5-dichloro-4-hydroxyphenylsulfonyl)piperidine-2-carboxylate
(S)-((R)-3-(3,4-dimethoxyphenyl)-1-(3-(2-morpholinoethoxy)phenyl)propyl)1-(3,5-dichloro-4-methoxyphenylsulfonyl)piperidine-2-carboxylate,
(S)-((R)-3-(3,4-dimethoxyphenyl)-1-(3-(2-morpholinoethoxy)phenyl)propyl)1-(2-oxo-2,3-dihydrobenzo[d]thiazol-6-ylsulfonyl)piperidine-2-carboxylate,
2-(3-((R)-3-(3,4-dimethoxyphenyl)-1-((S)-1-(2-oxoindolin-5-ylsulfonyl) piperidine-2-carbonyloxy)propyl)phenoxy)acetic acid,
2-(3-((R)-3-(3,4-dimethoxyphenyl)-1-((S)-1-(2-oxo-2,3-dihydrobenzo[d]oxazol-6-ylsulfonyl)piperidine-2-carbonyloxy)propyl)phenoxy)aceticacid,
2-(3-((R)-1-((S)-1-(4-acetamido-3,5-dichlorophenylsulfonyl)piperidine-2-carbo nyloxy)-3-(3,4-dimethoxyphenyl)propyl)phenoxy)acetic acid,
5-((S)-2-(((R)-1-(3-(carboxymethoxy)phenyl)-3-(3,4-dimethoxyphenyl)propoxy) carbonyl)piperidin-1-ylsulfonyl)-2,3-dimethoxybenzoic acid,
2-(3-((R)-3-(3,4-dimethoxyphenyl)-1-((S)-1-(7-nitro-2,3-dihydrobenzofuran-5-ylsulfonyl)piperidine-2-carbonyloxy)propyl)phenoxy)acetic acid,
2-(3-((R)-1-((S)-1-(7-amino-2,3-dihydrobenzofuran-5-ylsulfonyl)piperidine-2-carbonyloxy)-3-(3,4-dimethoxyphenyl)propyl)phenoxy)aceticacid,
2-(3-((R)-3-(3,4-dimethoxyphenyl)-1-((S)-1-(2-methylbenzo[d]thiazol-6-ylsulf onyl)piperidine-2-carbonyloxy)propyl)phenoxy)acetic acid,
2-(3-((R)-3-(3,4-dimethoxyphenyl)-1-((S)-1-(2-oxo-2,3-dihydrobenzo[d]thiazol-6-ylsulfonyl)piperidine-2-carbonyloxy)propyl)phenoxy)aceticacid,
2-(3-((R)-1-((S)-1-(3,5-bis(methoxycarbonyl)phenylsulfonyl)piperidine-2-carbonyl oxy)-3-(3,4-dimethoxyphenyl)propyl)phenoxy)acetic acid,
2-(3-((R)-1-((S)-1-(3,5-difluorophenylsulfonyl)piperidine-2-carbonyloxy)-3-(3,4-dimethoxyphenyl)propyl)phenoxy)acetic acid,
2-(3-((R)-1-((S)-1-(3-chloro-4-methoxyphenylsulfonyl)piperidine-2-carbonyl oxy)-3-(3,4-dimethoxyphenyl)propyl)phenoxy)aceticacid,
2-(3-((R)-3-(3,4-dimethoxyphenyl)-1-((S)-1-(3-fluorophenylsulfonyl)piperidine-2-carbonyloxy)propyl)phenoxy)acetic acid,
2-(3-((R)-1-((S)-1-(3-bromophenylsulfonyl)piperidine-2-carbonyloxy)-3-(3,4-dimethoxyphenyl)propyl)phenoxy)acetic acid,
2-(3-((R)-1-((S)-1-(3-aminophenylsulfonyl)piperidine-2-carbonyloxy)-3-(3,4-dimethoxyphenyl)propyl)phenoxy)acetic acid,
2-(3-((R)-1-((S)-1-(4-chlorophenylsulfonyl)piperidine-2-carbonyloxy)-3-(3,4-dimethoxyphenyl)propyl) phenoxy)acetic acid,
2-(3-((R)-1-((S)-1-(4-tert-butylphenylsulfonyl)piperidine-2-carbonyloxy)-3-(3,4-dimethoxyphenyl)propyl) phenoxy)acetic acid,
2-(3-((R)-3-(3,4-dimethoxyphenyl)-1-((S)-1-(4-fluorophenylsulfonyl)piperidine-2-carbonyloxy)propyl) phenoxy)acetic acid,
2-(3-((R)-1-((S)-1-(4-chlorobenzylsulfonyl)piperidine-2-carbonyloxy)-3-(3,4-dimethoxyphenyl)propyl) phenoxy)acetic acid,
2-(3-((R)-3-(3,4-dimethoxyphenyl)-1-((S)-1-(4-(trifluoromethyl)phenylsulfonyl) piperidine-2-carbonyloxy)propyl)phenoxy)acetic acid,
2-(3-((R)-3-(3,4-dimethoxyphenyl)-1-((S)-1-(4-phenoxyphenylsulfonyl)piperidine-2-carbonyloxy)propyl) phenoxy)acetic acid,
2-(3-((R)-3-(3,4-dimethoxyphenyl)-1-((S)-1-(pyridin-3-ylsulfonyl)piperidine-2-carbonyloxy)propyl) phenoxy)acetic acid,
2-(3-((R)-3-(3,4-dimethoxyphenyl)-1-((S)-1-(6-phenoxypyridin-3-ylsulfonyl)piperidine-2-carbonyloxy) propyl)phenoxy)acetic acid,
2-(3-((R)-3-(3,4-dimethoxyphenyl)-1-((S)-1-(6-phenylpyridin-3-ylsulfonyl)piperidine-2-carbonyloxy) propyl)phenoxy)acetic acid,
2-(3-((R)-1-((S)-1-(2-chlorophenylsulfonyl)piperidine-2-carbonyloxy)-3-(3,4-dimethoxyphenyl)propyl) phenoxy)acetic acid,
2-(3-((R)-1-((S)-1-(2,6-dichlorophenylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(3-(5-methyl-1,3,4-oxadiazol-2-yl)phenylsulfonyl)piperidine-2-carbonyloxy)propyl)phenoxy)acetic acid,
2-(3-((R)-1-((S)-1-(3-chloro-4-(trifluoromethyl)phenylsulfonyl)piperidine-2-carbonyloxy)-3-(3,4-dimethoxyphenyl)propyl)phenoxy)acetic acid,
2-(3-((R)-3-(3,4-dimethoxyphenyl)-1-((S)-1-(1-methyl-1H-indol-4-ylsulfonyl)piperidine-2-carbonyloxy) propyl)phenoxy)acetic acid,
2-(3-((R)-3-(3,4-dimethoxyphenyl)-1-((S)-1-(1-methyl-1H-indol-5-ylsulfonyl)piperidine-2-carbonyloxy)propyl)phenoxy)acetic acid,
2-(3-((R)-3-(3,4-dimethoxyphenyl)-1-((S)-1-(4-methyl-3,4-dihydro-2H-benzo[b][1,4]oxazin-6-ylsulfonyl)piperidine-2-carbonyloxy)propyl)phenoxy)acetic acid,
2-(3-((R)-3-(3,4-dimethoxyphenyl)-1-((S)-1-(4-methyl-3,4-dihydro-2H-pyrido[3,2-b][1,4]oxazin-7-ylsulfonyl)piperidine-2-carbonyloxy)propyl)phenoxy)acetic acid,
2-(3-((R)-3-(3,4-dimethoxyphenyl)-1-((S)-1-(furan-3-ylsulfonyl)piperidine-2-carbonyloxy)propyl) phenoxy)acetic acid,
2-(3-((R)-1-((S)-1-(benzofuran-2-ylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(thiophen-2-ylsulfonyl)piperidine-2-carbonyloxy)propyl) phenoxy)acetic acid,
2-(3-((R)-3-(3,4-dimethoxyphenyl)-1-((S)-1-(5-phenylthiophen-2-ylsulfonyl)piperidine-2-carbonyloxy) propyl)phenoxy)acetic acid,
2-(3-((R)-1-((S)-1-(benzo[b]thiophen-3-ylsulfonyl)piperidine-2-carbonyloxy)-3-(3,4-dimethoxyphenyl) propyl)phenoxy)acetic acid,
2-(3-((R)-3-(3,4-dimethoxyphenyl)-1-((S)-1-(3,5-dimethylisoxazol-4-ylsulfonyl)piperidine-2-carbonyloxy) propyl)phenoxy)acetic acid,
2-(3-((1 R)-3-(3,4-dimethoxyphenyl)-1-((2S)-1-(1-methyl-4,5-dihydro-1H-pyrazol-5-ylsulfonyl)piperidine-2-carbonyloxy)propyl)phenoxy)acetic acid,
2-(3-((R)-3-(3,4-dimethoxyphenyl)-1-((S)-1-(1-methyl-1H-imidazol-4-ylsulfonyl)piperidine-2-carbonyloxy)propyl)phenoxy)acetic acid,
2-(3-((R)-3-(3,4-dimethoxyphenyl)-1-((S)-1-(1,3,5-trimethyl-1H-pyrazol-4-ylsulfonyl)piperidine-2-carbonyloxy)propyl)phenoxy)acetic acid,
2-(3-((R)-3-(3,4-dimethoxyphenyl)-1-((S)-1-(3,5-dimethyl-1-phenyl-1H-pyrazol-4-ylsulfonyl)piperidine-2-carbonyloxy)propyl)phenoxy)acetic acid,
2-(3-((R)-3-(3,4-dimethoxyphenyl)-1-((S)-1-tosylpiperidine-2-carbonyloxy)propyl)phenoxy)acetic acid,
2-(3-((R)-3-(3,4-dimethoxyphenyl)-1-((S)-1-(4-nitrophenylsulfonyl)piperidine-2-carbonyloxy) propyl)phenoxy)acetic acid,
(S)-((R)-3-(3,4-dimethoxyphenyl)-1-(3-(2-morpholinoethoxy)phenyl)propyl)1-(4-acetamido-3,5-dichlorophenylsulfonyl)piperidine-2-carboxylate.

6. Compound according to any one of claims 1 - 5 for use in treatment or prophylaxis of psychiatric disorders, neurological disorders, cancers, glucocorticoid hyposensitivity syndromes, peripheral glucocorticoid resistance, infectious diseases, alopecia, abnormally elevated intraocular pressure, macular degeneration, oxidative damage to eye tissues, vision disorder, memory impairment and for neuroprotection, neuroregeneration, promoting hair growth, stimulating neurite growth, wound healing, antiglaucomatous medications, improving vision, enhancing memory performance, for the use in treatment or prevention of multi-drug resistance, for the use in induced abortion and the inhibition of embryo implantation, for the use in limiting or preventing hemorrhage or neovascularization and for the use in treatment of diseases relating to neurodegeneration.

7. Compound according to claim 6, wherein the psychiatric diseases is an affective disorder or an anxiety disorder.

8. Compound according to claim 7, wherein the affective disorder is selected from the group consisting of: depression, bipolar disorder, mania, substance induced mood disorder and seasonal affective disorder (SAD).

9. Compound according to claim 7, wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

10. Pharmaceutical composition comprising at least one compound according to claim 1 or 5 together with at least one pharmaceutically acceptable carrier, solvent or excipient.

11. Pharmaceutical composition according to claim 10 further comprising at least one active agent selected from the group consisting of an anti-depressant and other psychotropic drugs..

12. Pharmaceutical composition according to claim 11, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.

13. Compound according to claim 6, wherein the infectious disease is selected from the group consisting of: malaria and Legionnaires' disease
